(19) Europäisches Patentamt
European Patent Office
Office européen des brevets

(11) Veröffentlichungsnummer: **0 244 728 B1**

# (12) EUROPÄISCHE PATENTSCHRIFT

(45) Veröffentlichungstag der Patentschrift: **11.11.92**

(51) Int. Cl.$^5$: **C07D 213/73**, C07D 213/61, C07D 213/82, C07D 213/80, C07D 213/78, A23K 1/16

(21) Anmeldenummer: **87106083.6**

(22) Anmeldetag: **27.04.87**

Die Akte enthält technische Angaben, die nach dem Eingang der Anmeldung eingereicht wurden und die nicht in dieser Patentschrift enthalten sind.

## (54) Verwendung von Heteroarylethylaminen zur Leistungsförderung bei Tieren, Heteroarylethylamine und Verfahren zu ihrer Herstellung.

(30) Priorität: **06.05.86 DE 3615293**

(43) Veröffentlichungstag der Anmeldung:
**11.11.87 Patentblatt 87/46**

(45) Bekanntmachung des Hinweises auf die Patenterteilung:
**11.11.92 Patentblatt 92/46**

(84) Benannte Vertragsstaaten:
**AT BE CH DE ES FR GB GR IT LI NL SE**

(56) Entgegenhaltungen:
**EP-A- 0 026 298**
**EP-A- 0 103 830**
**EP-A- 0 105 053**
**EP-A- 0 120 770**
**EP-A- 0 166 025**
**EP-A- 0 170 538**
**DE-A- 1 811 833**
**DE-A- 2 603 600**

**B.J.R. Nicolaus in Decision Making in Drug Research (Ed. F. Gross), Raven Press, New York, 1983, S. 173 - 188**

**Agricultural and Biological Chemistry, Band 48, Nr. 11, November 1984, Seiten 2883-2888; G.Asato et al.**

(73) Patentinhaber: **BAYER AG**

**W-5090 Leverkusen 1 Bayerwerk(DE)**

(72) Erfinder: **Lindel, Hans, Dr.**
**Carl-Duisberg-Strasse 321**
**W-5090 Leverkusen 1(DE)**
Erfinder: **Hallenbach, Werner, Dr.**
**Kleiststrasse 10**
**W-4018 Langenfeld(DE)**
Erfinder: **Bonse, Gerhard, Dr.**
**Wolfskaul 3**
**W-5000 Koeln 80(DE)**
Erfinder: **Böshagen, Horst, Dr.**
**Wiesenstrasse 4**
**W-5657 Haan 2(DE)**
Erfinder: **Stoltefuss, Jürgen, D. I.**
**Parkstrasse 20**
**W-5657 Haan 2(DE)**

**EP 0 244 728 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Berschauer, Friedrich, Dr.**
**Claudiusweg 9**
**W-5600 Wuppertal 1(DE)**
Erfinder: **De Jong, Anno, Dr.**
**Stockmannsmühle 46**
**W-5600 Wuppertal 1(DE)**
Erfinder: **Scheer, Martin, Dr.**
**Herberts-Katernberg 7**
**W-5600 Wuppertal 1(DE)**

## Beschreibung

Die vorliegende Erfindung betrifft die Verwendung von Heteroarylethylaminen als Leistungsförderer bei Tieren, neue Heteroarylethylamine und Verfahren zu ihrer Herstellung.

Die Verwendung von Futtermittelzusätzen zur Erzielung höherer Gewichtszuwächse und verbesserter Futterausnutzung wird in der Tierernährung insbesondere bei der Mast von Schweinen, Rindern und Geflügel bereits weitgehend praktiziert.

Phenylethylamine und ihre Verwendung bei Tierernährung und Leistungsförderung sind bereits bekannt geworden (EP-OS 26 298,103 830, 166 025).

Heteroarylethylamine sind bereits bekannt geworden. Die Verbindungen besitzen beta-sympathomimetische Wirkungen (DE-OS 2 603 600, EP-OS 120 770, US-PS 4 358 455).
Es ist jedoch nichts über ihre Eignung als Leistungsförderer bei Tieren bekannt. Aus EP-OS 170 538 ist die Verwendung von zwei einzelnen Pyridylethanolaminen als Leistungsförderer für Tiere bekannt. Eine breite Verwendung dieser Verbindungsklasse für diesen Zweck ist aus EP-OS 170 538 nicht bekannt.

Die vorliegende Erfindung betrifft:

1. Verwendung von Heteroarylethylaminen der Formel (I)

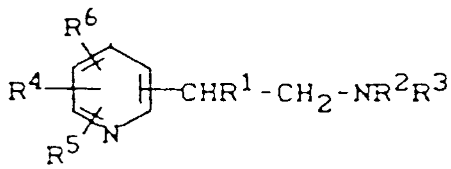

(I)

in welcher

| | |
|---|---|
| $R^1$ | für OH, Acyloxy oder Alkoxy steht |
| $R^2$ | für Wasserstoff oder Alkyl steht, |
| $R^3$ | für Wasserstoff, Alkyl, Cycloalkyl, substituiertes Alkyl, gegebenenfalls substituiertes Aralkyl, Aryl oder Heterocyclyl steht, |
| $R^2$ und $R^3$ | können gemeinsam mit dem Stickstoffatom, an das sie gebunden sind, für einen gegebenenfalls substituierten heterocyclischen Rest stehen, |
| $R^4$ | für Wasserstoff, Alkyl, Halogen, Halogenalkyl, Hydroxyl, Cyano, Alkoxycarbonyl, Aminocarbonyl, Mono- und Dialkylaminocarbonyl, Alkoxy, Halogenalkoxy, Halogenalkylthio, $NHSO_2$-Alkyl steht, |
| $R^5$ | für Wasserstoff, Hydroxyl, Alkoxy oder den Rest $-NR^7R^8$ steht, |
| $R^6$ | für die bei $R^4$ angegebenen Reste steht, |
| $R^7$ | für Wasserstoff oder Alkyl steht, |
| $R^8$ | für Wasserstoff, Alkyl, Halogenalkyl, Acyl steht, |

ausgenommen die Verbindung 6-Amino-α-(((1-methyl-3-phenylpropyl)amino)methyl)-3-pyridinmethanol,
(Die Verbindungen der Formel I können dabei in Form ihrer Tautomeren sowie in Form ihrer Racemate oder Enantiomeren vorliegen) zur Leistungsförderung bei Tieren.

2. Heteroarylethylamine der Formel I,

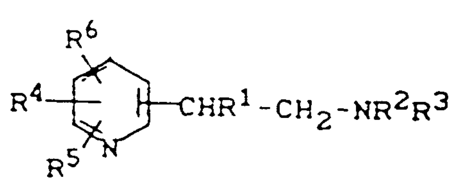

(I)

in welcher

| | |
|---|---|
| $R^1$ | für OH, $C_{1-6}$-Alkoxy oder für Oxycarbonyl-$C_{1-6}$-alkyl, gegebenenfalls substituiertes Oxycarbonylphenyl,Oxysulfonyl-$C_{1-6}$-alkyl, gegebenenfalls substituiertes Oxysulfonylphenyl steht, |
| $R^2$ | für Wasserstoff oder $C_{1-6}$-Alkyl steht, |
| $R^3$ | für Wasserstoff, gegebenenfalls substituiertes $C_{1-6}$-Alkyl, $C_{3-6}$-Cycloalkyl, substituiertes $C_{1-6}$-Alkylphenyl oder Phenyl steht, |

$R^4$ für Wasserstoff, $C_{1-4}$-Alkyl, Fluor, Chlor, Brom, $C_{1-4}$-Halogenalkyl, Cyano, $C_{1-4}$-Alkoxycarbonyl, Mono- oder -di-$C_{1-4}$-alkylaminocarbonyl, $C_{1-6}$-Alkoxy, $C_{1-6}$-Halogenalkoxy, $C_{1-6}$-Halogenalkylthio, -$NHSO_2$-$C_{1-6}$-Alkyl steht,

$R^5$ für Wasserstoff, $C_{1-6}$-Alkoxy oder den Rest -$NR^7R^8$ steht,

$R^6$ für Wasserstoff, $C_{1-4}$-Alkyl, Fluor, Chlor, Brom, $C_{1-4}$-Halogenalkyl, Cyano, $C_{1-4}$-Alkoxycarbonyl, $C_{1-6}$-Alkoxy, $C_{1-6}$-Halogenalkoxy, $C_{1-6}$-Halogenalkylthio, $NHSO_2$-Alkyl steht,

unter der Voraussetzung, daß $R^4$, $R^5$, $R^6$ nicht gleichzeitig für Wasserstoff stehen,

$R^7$ für Wasserstoff oder $C_{1-6}$-Alkyl steht,

$R^8$ für Wasserstoff, $C_{1-6}$-Alkyl, $C_{1-6}$-Halogenalkyl oder Acyl steht.

Wobei als Substituenten der gegebenenfalls substituierten Reste genannt seien: Cyano, Halogen wie Fluor oder Chlor, Hydroxy, $C_{1-4}$-Alkyl, $C_{1-4}$-Halogenalkyl,Phenyl, $C_{1-4}$-Alkoxy, $C_{1-4}$-Halogenalkoxy, $C_{1-4}$-Alkylthio, $C_{1-4}$-Halogenalkylthio, für den Fall, daß die Substituenten an einem Phenylrest sitzen, zusätzlich Methylendioxy, Ethylendioxy, halogensubstituiertes Methylendioxy, halogensubstituiertes Ethylendioxy, ferner Phenyl, Phenoxy, die ihrerseits einen oder mehrere der obengenannten Substituenten tragen können,

sowie ihre physiologisch verträglichen Salze und ihre N-Oxide,

ausgenommen 6-Chlor-α(isopropylaminomethyl)-3-pyridinmethanol und 2-Chlor-α-(isopropylaminomethyl)-4-pyridinmethanol.

3. Verfahren zur Herstellung der Heteroarylethylamine der Formel I,

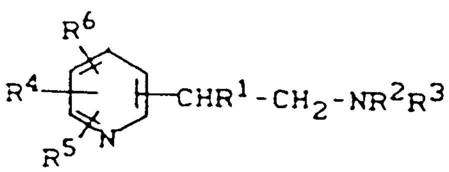

(I)

in welcher

$R^1$ für OH, $C_{1-6}$-Alkoxy oder für Oxycarbonyl-$C_{1-6}$-alkyl, gegebenenfalls substituiertes Oxycarbonylphenyl,Oxysulfonyl-$C_{1-6}$-alkyl, gegebenenfalls substituiertes Oxysulfonylphenyl steht,

$R^2$ für Wasserstoff oder $C_{1-6}$-Alkyl steht,

$R^3$ für Wasserstoff, gegebenenfalls substituiertes $C_{1-6}$-Alkyl, $C_{3-6}$-Cycloalkyl, substituiertes $C_{1-6}$-Alkylphenyl oder Phenyl steht,

$R^4$ für Wasserstoff, $C_{1-4}$-Alkyl, Fluor, Chlor, Brom, $C_{1-4}$-Halogenalkyl, Cyano, $C_{1-4}$-Alkoxycarbonyl, Mono- oder -di-$C_{1-4}$-alkylaminocarbonyl, $C_{1-6}$-Alkoxy, $C_{1-6}$-Halogenalkoxy, $C_{1-6}$-Halogenalkylthio, -$NHSO_2$-$C_{1-6}$-Alkyl steht,

$R^5$ für Wasserstoff, $C_{1-6}$-Alkoxy oder den Rest -$NR^7R^8$ steht,

$R^6$ für Wasserstoff, Alkyl, Halogen, Halogenalkyl, Cyano, Alkoxycarbonyl, Alkoxy, Halogenalkoxy, Halogenalkylthio, $NHSO_2$-Alkyl steht,

unter der Voraussetzung, daß $R^4$, $R^5$, $R^6$ nicht gleichzeitig für Wasserstoff stehen,

$R^7$ für Wasserstoff oder $C_{1-6}$-Alkyl steht,

$R^8$ für Wasserstoff, $C_{1-6}$-Alkyl, $C_{1-6}$-Halogenalkyl oder Acyl steht

Wobei als Substituenten der gegebenenfalls substituierten Reste genannt seien: Cyano, Halogen wie Fluor oder Chlor, Hydroxy, $C_{1-4}$-Alkyl, $C_{1-4}$-Halogenalkyl,Phenyl, $C_{1-4}$-Alkoxy, $C_{1-4}$-Halogenalkoxy, $C_{1-4}$-Alkylthio, $C_{1-4}$-Halogenalkylthio, für den Fall, daß die Substituenten an einem Phenylrest sitzen, zusätzlich Methylendioxy, Ethylendioxy, halogensubstituiertes Methylendioxy, halogensubstituiertes Ethylendioxy, ferner Phenyl, Phenoxy, die ihrerseits einen oder mehrere der obengenannten Substituenten tragen können,

sowie ihre physiologisch verträglichen Salze und ihre N-Oxide,

ausgenommen 6-Chlor-α(isopropylaminomethyl)-3-pyridinmethanol und 2-Chlor-α-(isopropylaminomethyl)-4-pyridinmethanol,

dadurch gekennzeichnet, daß man

a) Halogenmethylketone der Formel (II)

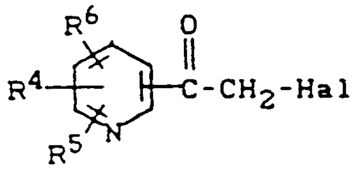

(II)

in welcher

$R^4$, $R^5$, $R^6$ die oben angegebene Bedeutung haben und Hal für Halogen steht,

mit Aminen der Formel (III)

$HNR^2R^3$ (III)

in welcher

$R^2$ und $R^3$ die oben angegebene Bedeutung haben,

umsetzt und anschließend die Carbonylgruppe reduziert, oder

b) Epoxide der Formel (IV)

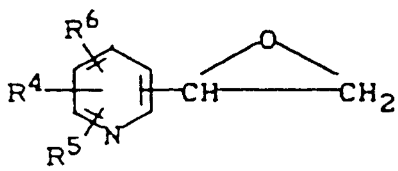

(IV)

in welcher

$R^4$, $R^5$, $R^6$ die oben angegebene Bedeutung haben

mit Aminen der Formel (III)

$HNR^2R^3$ (III)

in welcher

$R^2$ und $R^3$ die oben angegebene Bedeutung haben,

umsetzt oder

c) beta-Halogenethylverbindungen der Formel (V)

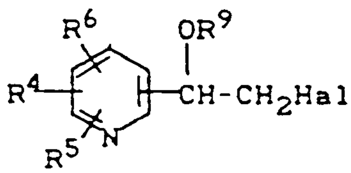

(V)

in welcher

$R^4$, $R^5$, $R^6$ die oben angegebene Bedeutung haben,

Hal für Halogen steht,

$R^9$ für Wasserstoff steht,

mit Aminen der Formel (III)

$HNR^2R^3$ (III)

in welcher

$R^2$ und $R^3$ die oben angegebene Bedeutung haben

umsetzt oder

d) für den Fall, daß in Formel Ia $R^2$ für Wasserstoff steht und $R^3$ für substituiertes Alkyl oder Aralkyl steht, man Verbindungen der Formel (VI)

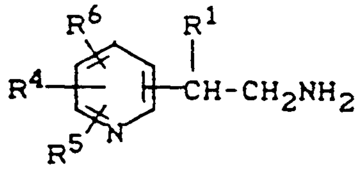

(VI)

in welcher

$R^1$, $R^4$, $R^5$, $R^6$ die oben angegebene Bedeutung haben

mit Verbindungen der Formel (VII)

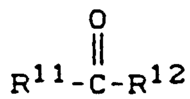

(VII)

in welcher

$R^{11}$ für substituiertes Alkyl, Aryl oder Aralkyl steht,

$R^{12}$ für Wasserstoff oder $C_{1-4}$-Alkyl steht,

unter reduzierenden Bedingungen umsetzt, oder

e) für den Fall, daß in Formel I $R^2$ für Wasserstoff steht, man Verbindungen der Formel (VIII)

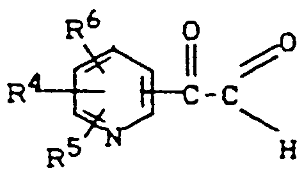

(VIII)

in welcher

$R^4$, $R^5$, $R^6$ die oben angegebene Bedeutung haben

mit Aminen der Formel (IX)

$R^3$-$NH_2$ (IX)

in welcher

$R^3$ die oben angegebene Bedeutung hat,

unter reduzierenden Bedingungen umsetzt, oder

f) für den Fall, daß in Formel I $R^2$ für Wasserstoff steht, Verbindungen der Formel X

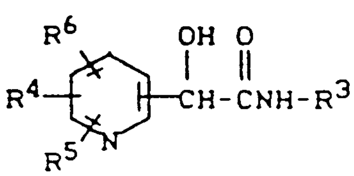

(X)

in welcher

$R^3$, $R^4$, $R^5$, $R^6$ die oben angegebene Bedeutung haben,

reduziert.

4. Pyridinderivate der Formel

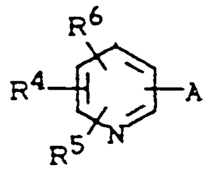

in welcher

$R^4$ für Wasserstoff, $C_{1-4}$-Alkyl, Fluor, Chlor, Brom,$C_{1-4}$-Halogenalkyl, Cyano, $C_{1-4}$-Alkoxycarbonyl, Mono- oder di-$C_{1-4}$ -Alkylaminocarbonyl, $C_{1-6}$-Alkoxy, $C_{1-6}$-Halogenalkoxy,$C_{1-6}$-Halogenalkylthio, $NHSO_2$-Alkyl steht,

$R^5$ für Wasserstoff, $C_{1-6}$-Alkoxy, oder den Rest $-NR^7R^8$ steht,

$R^6$ für Wasserstoff, $C_{1-4}$-Alkyl, Fluor, Chlor, Brom, $C_{1-4}$-Halogenalkyl, Cyano, $C_{1-4}$-Alkoxycarbonyl, $C_{1-6}$-Alkoxy, $C_{1-6}$-Halogenalkoxy, $C_{1-6}$-Halogenalkylthio, $-NH-SO_2$-Alkyl steht,

unter der Voraussetzung, daß $R^4$, $R^5$, $R^6$ nicht gleichzeitig für Wasserstoff stehen und

A für die Reste -CHO, -COOH, -COO($C_{1-4}$-Alkyl), -COCl,

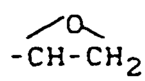,

$-COCH_3$, -CO-CHO, $-COCH_2$-Halogen, $-CH=CH-NO_2$, $-CO-CH_2-COO(C_{1-4}$-Alkyl),

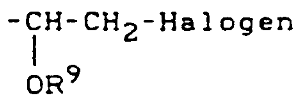, 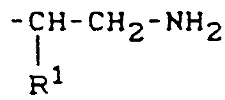, 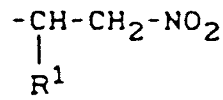,

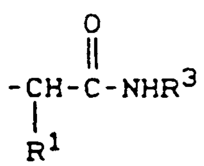

steht und

$R^9$ für Wasserstoff steht,

$R^1$ für Hydroxyl oder Acyloxy steht,

$R^3$ für Wasserstoff, $C_{1-6}$-Alkyl, substituiertes $C_{1-6}$-Alkyl, Cycloalkyl, substituiertes $C_{1-6}$-Alkylphenyl oder Phenyl steht,

$R^7$ für Wasserstoff oder $C_{1-6}$-Alkyl steht,

$R^8$ für Wasserstoff, $C_{1-6}$-Alkyl, $C_{1-6}$-Halogenalkyl oder Acyl steht,

ausgenommen α-(Brommethyl)-2-chlor-4-pyridinmethanol, Brommethyl-2-chlor-4-pyridinketon, 2-chlor-4-pyridylmethylketon, Brommethyl-6-chlor-3-pyridylketon, α-(Brommethyl)-2-chlor-4-pyrdinmethanol, 5-Acetyl-2-fluorpyridin, 5-Brom-acetyl-2-fluorpyridin, (2-Fluorpyrid-5-yl)oxiran.

Die Verbindungen der Formel I können in Form ihrer Tautomeren vorliegen. Beispiele dafür sind für den Fall, daß $R^5$ für $NH_2$ steht:

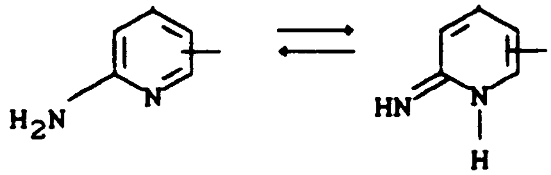

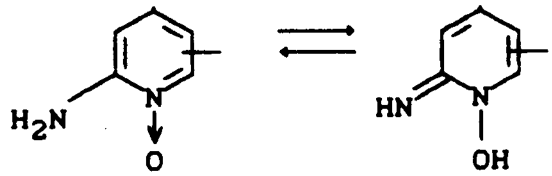

Die Verbindungen der Formeln I können auch in Form ihrer Racemate oder enantiomeren Formen vorliegen.

Physiologisch verträgliche Salze der Verbindungen der Formel I können mit folgenden Säuren gebildet werden:
Salzsäure, Schwefelsäure, Phosphorsäure, Perchlorsäure, Brom-, Iodwasserstoffsäure, Salpetersäure, Essigsäure, Oxalsäure, Malonsäure, Bernsteinsäure, Ascorbinsäure, Äpfelsäure, Weinsäure, Maleinsäure, Fumarsäure, Methansulfonsäure, Benzoesäure, substituierte Benzoesäuren, Ameisensäure, Toluolsulfonsäure, Benzolsulfonsäure, Phthalsäure, Naphthalinsulfonsäure, Nikotinsäure, Palmitinsäure, Embonsäure.

Bevorzugt sind Verbindungen der Formel I
in welcher

- $R^1$ für OH, $C_{1-6}$-Alkoxy oder Acyloxy steht und Acyloxy für Oxycarbonyl-$C_{1-6}$-alkyl, gegebenenfalls substituiertes Oxycarbonylphenyl, Oxysulfonyl-$C_{1-6}$-alkyl, gegebenenfalls substituiertes Oxysulfonylphenyl steht,
- $R^2$ für Wasserstoff oder $C_{1-6}$-Alkyl steht,
- $R^3$ für Wasserstoff, gegebenenfalls substituiertes $C_{1-6}$-Alkyl, $C_{3-6}$-Cycloalkyl, $C_{1-6}$-Alkylphenyl, Phenyl steht,
- $R^2$ und $R^3$ können gemeinsam mit dem Stickstoffatom, an das sie gebunden sind, für einen gegebenenfalls substituierten 5- bis 6-gliedrigen gesättigten oder ungesättigten heterocyclischen Rest stehen, der gegebenenfalls noch weitere Heteroatome aus der Reihe N, O, S enthalten kann,
- $R^4$ für Wasserstoff, $C_{1-4}$-Alkyl, Fluor, Chlor, Brom, $C_{1-4}$-Halogenalkyl, Hydroxyl, Cyano, $C_{1-4}$-Alkoxycarbonyl, Mono- oder -di-$C_{1-4}$-alkylaminocarbonyl, $C_{1-6}$-Alkoxy, $C_{1-6}$-Halogenalkoxy, $C_{1-6}$-Halogenalkylthio, -$NHSO_2$-$C_{1-6}$-Alkyl steht,
- $R^5$ für Wasserstoff, Hydroxyl, $C_{1-6}$-Alkoxy oder den Rest -$NR^7R^8$ steht,
- $R^6$ für die bei $R^4$ angegebenen Reste steht,
- $R^7$ für Wasserstoff oder $C_{1-6}$-Alkyl steht,
- $R^8$ für Wasserstoff, $C_{1-6}$-Alkyl, $C_{1-4}$-Halogenalkyl, $C_{1-6}$-Alkylcarbonyl, gegebenenfalls substituiertes Benzoyl, $C_{1-6}$-Alkylsulfonyl, gegebenenfalls substituiertes Phenylsulfonyl steht.

Als Substituenten der gegebenenfalls substituierten Reste kommen bevorzugt infrage: Cyano, Halogen wie Fluor oder Chlor, Hydroxy, $C_{1-4}$-Alkyl, $C_{1-4}$-Halogenalkyl,Phenyl, $C_{1-4}$-Alkoxy, $C_{1-4}$-Halogenalkoxy, $C_{1-4}$-Alkylthio, $C_{1-4}$-Halogenalkylthio, für den Fall, daß die Substituenten an einem Phenylrest sitzen, zusätzlich bevorzugt Methylendioxy, Ethylendioxy, halogensubstituiertes Methylendioxy, halogensubstituiertes Ethylendioxy, ferner Phenyl, Phenoxy, die ihrerseits einen oder mehrere der obengenannten Substituenten tragen können.

Besonders bevorzugt sind Verbindungen der Formel I
in welcher

- $R^1$ für OH, $C_{1-6}$-Alkoxy, insbesondere Methoxy oder Ethoxy steht,
- $R^2$ für Wasserstoff oder $C_{1-4}$-Alkyl, insbesondere Methyl oder Ethyl steht,
- $R^3$ für Wasserstoff, gegebenenflls durch 1 bis 5 Halogenatome substituiertes $C_{1-6}$-Alkyl, insbesondere Methyl, Ethyl, Propyl, Butyl, Pentyl, Hexyl, wobei insbesondere sekundäre und tertiäre Alkylreste genannt seien, sowie $C_{1-6}$-Alkylphenyl, das gegebenenfalls durch Halogen, insbesondere Fluor oder Chlor, $C_{1-4}$-Alkyl, Hydroxy, $C_{1-4}$-Alkoxy, gegebenenfalls halogensubstituiertes Methylendioxy oder Ethylendioxy substituiert sein kann, Phenyl, das gegebenenfalls durch Halogen, insbesondere Fluor oder Chlor, $C_{1-4}$-Alkyl, Hydroxy, $C_{1-4}$-Alkoxy, gegebenenfalls halogensubstituiertes Methylendioxy oder Ethylendioxy substituiert sein kann, steht,

$R^4$ für Wasserstoff, $C_{1-4}$-Alkyl, Halogen, insbesondere Fluor, Chlor, Brom, Cyano, Hydroxy, $C_{1-4}$-Halogenalkyl mit 1 bis 5 Halogenatomen, $C_{1-4}$-Alkoxy, $C_{1-4}$-Halogenalkoxy mit 1 bis 5 Halogenatomen, $NHSO_2$-$C_{1-4}$-Alkyl, -COO-$C_{1-4}$-Alkyl, -$CONH_2$, -CONH-$C_{1-4}$-Alkyl, -CON($C_{1-4}$-Alkyl)$_2$ steht,

$R^5$ für Wasserstoff, OH, $C_{1-4}$-Alkoxy, -$NR^7R^8$ steht,

$R^6$ für die bei $R^4$ angegebenen Reste steht,

$R^7$ für Wasserstoff, $C_{1-4}$-Alkyl steht,

$R^8$ für Wasserstoff, $C_{1-4}$-Alkyl steht.

Im einzelnen seien folgende Verbindungen der Formel I genannt:

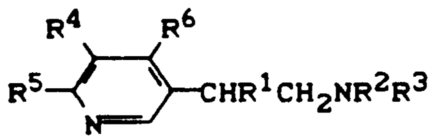

| $R^1$ | $R^2$ | $R^3$ | $R^4$ | $R^5$ | $R^6$ |
|---|---|---|---|---|---|
| OH | H | $i-C_3H_7$ | Cl | $NH_2$ | H |
| OH | H | $i-C_3H_7$ | CN | $NH_2$ | H |
| OH | H | $t-C_4H_9$ | Cl | $NH_2$ | H |
| OH | H | $t-C_4H_9$ | CN | $NH_2$ | H |
| OH | H | $i-C_3H_7$ | CN | OH | H |
| OH | H | $t-C_4H_9$ | CN | OH | H |

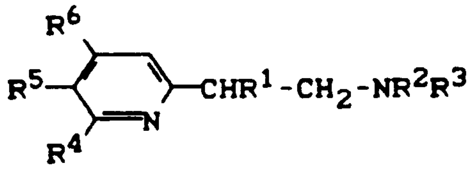

| $R^1$ | $R^2$ | $R^3$ | $R^4$ | $R^5$ | $R^6$ |
|---|---|---|---|---|---|
| OH | H | $i-C_3H_7$ | Cl | $NH_2$ | Cl |
| $OCH_3$ | H | $i-C_3H_7$ | Cl | $NH_2$ | Cl |
| OH | H | $t-C_4H_9$ | Cl | $NH_2$ | Cl |
| OH | H | $t-C_4H_8F$ | Cl | $NH_2$ | Cl |
| OH | H | $i-C_3H_7$ | Cl | $NH_2$ | CN |
| OH | H | $i-C_3H_7$ | CN | $NH_2$ | Cl |
| OH | H | $t-C_4H_9$ | Cl | $NH_2$ | CN |
| OH | H | $t-C_4H_9$ | CN | $NH_2$ | Cl |
| OH | H | $i-C_3H_7$ | H | $NH_2$ | CN |
| OH | H | $i-C_3H_7$ | CN | $NH_2$ | H |
| OH | H | $i-C_3H_7$ | Cl | $NH_2$ | $CF_3$ |
| OH | H | $t-C_4H_9$ | $CF_3$ | $NH_2$ | F |
| OH | H | $i-C_3H_7$ | CN | $NH_2$ | F |
| OH | H | $t-C_4H_9$ | Br | $NH_2$ | CN |

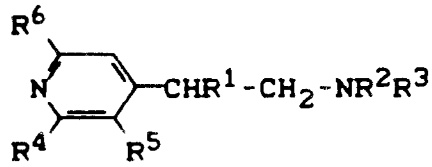

| $R^1$ | $R^2$ | $R^3$ | $R^4$ | $R^5$ | $R^6$ |
|---|---|---|---|---|---|
| OH | H | $i-C_3H_7$ | Cl | H | Cl |
| OH | H | $t-C_4H_9$ | Cl | H | Cl |
| OH | H | $i-C_3H_7$ | CN | H | H |
| OH | H | $t-C_4H_9$ | Cl | H | H |

Bevorzugt seien die Salze mit Salzsäure, Schwefelsäure, Phosphorsäure, Oxalsäure, Maleinsäure, Fumarsäure, Malonsäure genannt.

Wie bereits erwähnt, sind die Verbindungen der Formel I zum Teil bereits bekannt oder lassen sich analog zu bekannten Verfahren herstellen.

Die neuen Verbindungen der Formel I lassen sich nach den oben angegebenen Verfahren a) bis f) herstellen.

Setzt man bei Verfahren a) als Halogenmethylketon der Formel II 2-Chloracetyl-6-methoxypyridin und als Amin der Formel III t-Butylamin ein, läßt sich Verfahren a) durch folgendes Reaktionsschema wiedergeben:

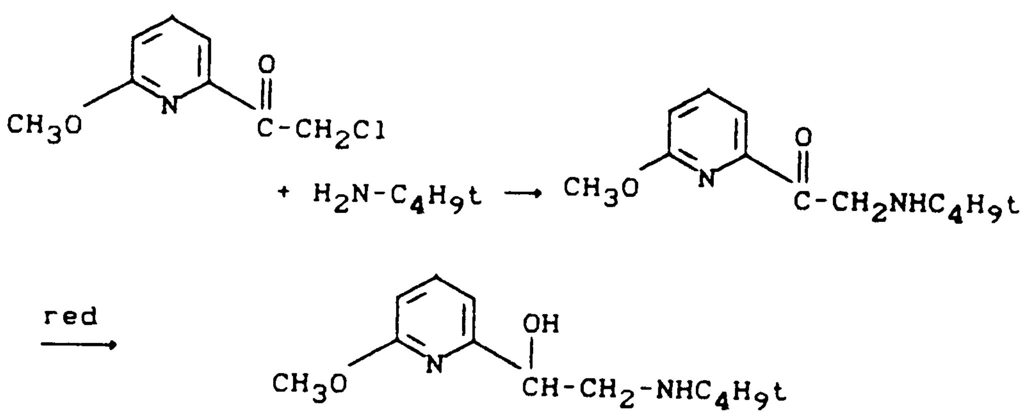


Die Verbindungen der Formel II sind bekannt (C.T.Gnewuch und H.L. Friedman, J. Med. Chem. 15, 1321 (1972)) oder lassen sich analog zu bekannten Verfahren herstellen. Die Substituenten $R^4$, $R^5$ und $R^6$ in Formel II besitzen bevorzugt die weiter oben bei den Verbindungen der Formel I angegebenen bevorzugten Bedeutungen. Im einzelnen seien folgende Verbindungen der Formel II genannt:
(2-Amino-3-chlor-5-pyridyl)-chlormethylketon,
(2-Amino-3-cyano-5-pyridyl)-chlormethylketon,
(2,4-Dichlor-3-amino-6-pyridyl)-brommethylketon,
(2-Cyano-3-amino-6-pyridyl)-brommethylketon,
(3-Amino-4-cyano-6-pyridyl)-brommethylketon,
(2-Amino-3-cyano-5-pyridyl)-brommethylketon,
(2-Cyano-3-amino-4-chlor-6-pyridyl)-brommethylketon,
(2-Cyano-3-amino-4-chlor-6-pyridyl)-chlormethylketon,
(2-Chlor-3-amino-4-trifluormethyl-6-pyridyl)-brommethylketon,
(2-Trifluormethyl-3-amino-4-cyano-6-pyridyl)-brommethylketon,
(2-Fluor-3-amino-4-cyano-6-pyridyl)-chlormethylketon.

Die Amine der Formel III sind bekannt oder lassen sich analog zu bekannten Verfahren herstellen. Die Substituenten $R^2$ und $R^3$ besitzen bevorzugt die weiter oben bei den Verbindungen der Formel I angegebenen bevorzugten Bedeutungen. Im einzelnen seien folgende Verbindungen der Formel III genannt:
Ammoniak, Methylamin, Dimethylamin, Ethylamin, Diethylamin, Methylethylamin, n-Propylamin, i-Propylamin, n-Butylamin, i-Butylamin, tert.-Butylamin, Cyclopentylamin, Cyclohexylamin, Benzylamin, Anilin, Piperidin, Morpholin, 2-Aminopyridin.

Als Reduktionsmittel zur Durchführung des Verfahrens a) seien folgende Reduktionsmittel genannt:
$H_2$/Katalysator, als Katalysator seien beispielsweise genannt: $PtO_2$, Pd-Aktivkohle;
komplexe Metallhydride wie z.B. $LiAlH_4$, $NaBH_4$, $NaBH_3CN$.

Besonders bevorzugt werden folgende Reduktionsmittel eingesetzt:
$NaBH_4$ und $NaBH_3CN$
Verfahren a) wird durchgeführt indem man Verbindungen II und III in einem Verdünnungsmittel in etwa äquimolaren Verhältnis zusammengibt.

Die Reaktion wird bei Temperaturen von -20 °C bis +100 °C bevorzugt durchgeführt.

Es wird bevorzugt bei Normaldruck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Petrolether, Ligroin, Benzol, Toluol; chlorierte Kohlenwasserstoffe wie Methylenchlorid, Ethylenchlorid, Chloroform; Ether wie Diethylether und Glykoldimethylether; Nitrile wie Acetonitril, Propionitril und Benzonitril; Alkohole wie Methanol, Ethanol, n- und i-Propanol.

Bevorzugt sind Alkohole, wobei ohne Isolierung der Zwischenstufen sofort die Reduktion durchgeführt werden kann.

Setzt man bei Verfahren b) als Epoxid der Formel IV 5-Methoxypyridin-3-epoxid und als Amin der Formel III t-Butylamin ein, läßt sich Verfahren b) durch folgendes Reaktionsschema wiedergeben:

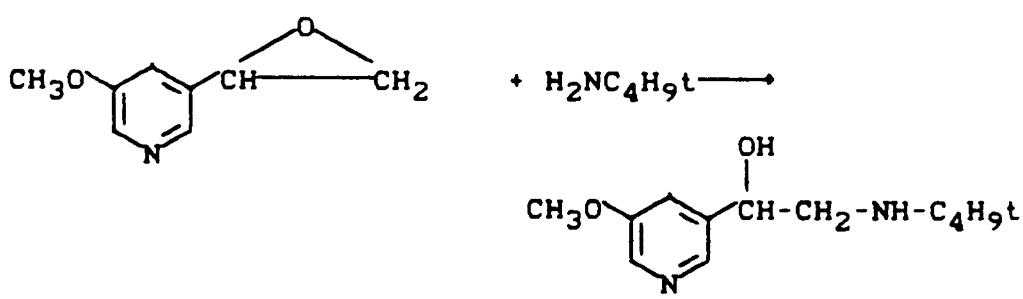

Epoxide der Formel IV sind bekannt (US-Pat. 3 948 919; US-Pat. 4 011 231; US-Pat. 4 031 108), oder lassen sich analog zu bekannten Verfahren herstellen.

Im einzelnen seien die folgenden Epoxide genannt:
2-Amino-3-chlorpyridin-5-epoxid,
2-Amino-3-cyanopyridin-5-epoxid,
2,4-Dichlor-3-aminopyridin-6-epoxid,
2-Chlor-3-amino-4-cyanopyridin-6-epoxid,
2-Cyano-3-amino-4-chlorpyridin-6-epoxid,
2-Cyano-3-aminopyridin-6-epoxid,
2-Chlor-3-amino-4-trifluormethylpyridin-6-epoxid,
2-Brom-3-amino-4-cyanopyridin-6-epoxid.

Verfahren b) wird durchgeführt indem man etwa äquimolare Mengen des Epoxids der Formel (IV) und des Amine der Formel (III) in einem Verdünnungsmittel umsetzt.

Im allgemeinen wird ein Überechuß an Amin (1-3 molar, bevorzugt 1-1,5 molar) bezogen auf das Epoxid der Formel IV verwendet.

Die Reaktion wird bei Temperaturen von +20 bis +150 °C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, ferner Nitrile wie Acetonitril und Benzonitril, Amide wie Dimethylformamid, Alkohole wie Methanol, Ethanol, n- und i-Propanol.

Bevorzugt sind Alkohole.

Setzt man bei Verfahren c) als beta-Halogenmethylverbindung der Formel V 5-Methoxy-3-(1-hydroxy-2-chlorethyl)pyridin und als Amin der Formel III t-Butylamin ein, läßt sich Verfahren c) durch folgendes Formelschema wiedergeben:

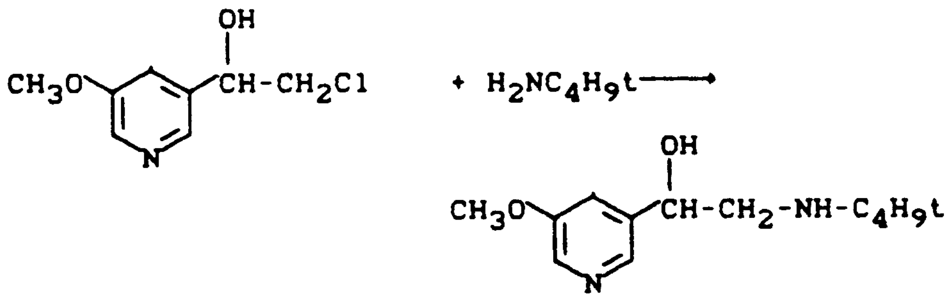

Beta-Halogenmethylverbindungen der Formel V sind bekannt (C.T.Gnewuch u. H.L. Friedman, J. Med. Chem. 15, 1321 (1972) oder lassen sich analog zu bekannten Verfahren herstellen.

Im einzelnen seien folgende Verbindungen der Formel V genannt:

1-(2-Amino-3-chlor-5-pyridyl)-2-chlorethanol,
1-(2-Amino-3-cyano-5-pyridyl)-2-chlorethanol,
1-(2,4-Dichlor-3-amino-6-pyridyl)-2-chlorethanol,
1-(2-Chlor-3-amino-4-cyano-6-pyridyl)-2-chlorethanol,
1-(2-Cyano-3-amino-4-chlor-6-pyridyl)-2-bromethanol,
1-(2-Cyano-3-amino-6-pyridyl)-2-chlorethanol,
1-(3-Amino-4-cyano-6-pyridyl)-2-bromethanol,
1-(2-Chlor-3-amino-4-trifluormethyl-6-pyridyl)-2-chlorethanol,
1-(2-Cyano-3-amino-4-fluor-6-pyridyl)-2-bromethanol.

Verfahren c) wird durchgeführt indem man die beta-Halogenmethylverbindung der Formel V mit überschüssigem Amin der Formel III gegebenenfalls in Gegenwart eines Verdünnungsmittels umsetzt.

Die Reaktion wird bei Temperaturen von +20 bis +150 °C durchgeführt.

Es wird bei Normaldruck oder unter erhöhtem Druck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Cyclohexan, Benzol, Toluol, Methylenchlorid, Chloroform, ferner Ether wie Diethylether, Tetrahydrofuran und Dioxan, weiterhin Nitrile wie Acetonitril und Benzonitril, ferner Amide wie Dimethylformamid, ferner Alkohole wie Methanol, Ethanol, n- und i-Propanol.

Bevorzugt eingesetzt werden Alkohole.

Setzt man bei Verfahren d) als Verbindung der Formel VI 5-Fluor-3(1-hydroxy-2-aminoethyl)-pyridin und als Verbindung der Formel VII Benzaldehyd ein, läßt sich Verfahren d) durch folgendes Formelschema wiedergeben:

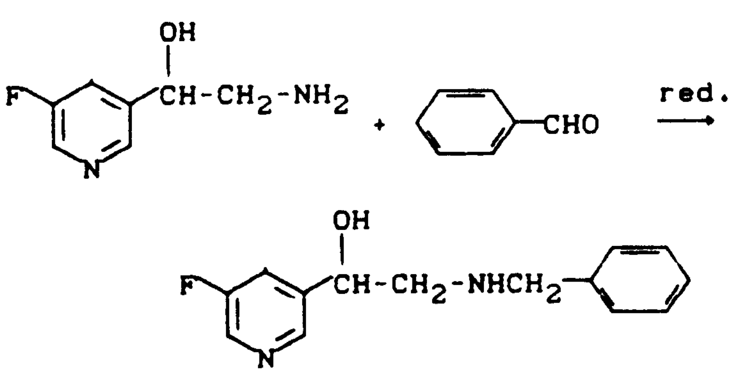


Verbindungen der Formeln VI sind bekannt (F. Zymalkowski, Arch. Pharm. 291, 12 (1958)) oder lassen sich analog zu bekannten Verfahren herstellen.

Im einzelnen seien folgende Verbindungen der Formeln VI genannt:
1-(2-Amino-3-chlor-5-pyridyl)-2-aminoethanol,
1-(2-Amino-3-cyano-5-pyridyl)-2-aminoethanol,
1-(2,4-Dichlor-3-amino-6-pyridyl)-2-aminoethanol,
1-(2-Chlor-3-amino-4-cyano-6-pyridyl)-2-aminoethanol,
1-(2-Cyano-3-amino-6-pyridyl)-2-aminoethanol,
1-(2-Chlor-3-amino-4-trifluormethyl-6-pyridyl)-2-aminoethanol.

Verbindungen der Formel VII sind bekannt oder lassen sich analog zu bekannten Verfahren herstellen. Die Substituenten $R^{11}$ und $R^{12}$ besitzen die weiter oben angegebenen Bedeutungen.

Im einzelnen seien folgende Verbindungen der Formel VII genannt: Acetaldehyd, Propionaldehyd, Benzaldehyd, 2-Methylbenzaldehyd, 3-Chlorbenzaldehyd, 2,4-Dichlorbenzaldehyd, Phenylacetaldehyd, 4-Nitrophenylacetaldehyd, Aceton, Methylethylketon, Methylisopropylketon, Diethylketon, Ethylpropylketon, Acetophenon, 4-Chloracetophenon, Phenylaceton, Phenoxyaceton.

Verfahren d) wird durchgeführt indem man etwa äquimolare Mengen der Verbindungen der Formeln VI und VII in einem Verdünnungsmittel vorlegt und die Mischung reduziert.

Die Reaktion wird bei Temperaturen von 0 ° C bis 150 ° C durchgeführt.

Es wird bevorzugt bei Normaldruck gearbeitet.

Als Verdünnungsmittel dienen alle inerten organischen Lösungsmittel. Hierzu gehören aliphatische und aromatische, gegebenenfalls halogenierte Kohlenwasserstoffe wie Pentan, Hexan, Cyclohexan, Benzol, Toluol, Methylenchlorid, Ethylenchlorid, Chloroform, Chlorbenzol, ferner Ether wie Diethylether, Tetrahydrofuran, Dioxan, weiterhin Nitrile wie Acetonitril, Benzonitril, Amide wie Dimethylformamid, Alkohole wie Methanol, Ethanol.

Als Reduktionsmittel dienen:
$H_2$/Katalysator, als Katalysator sei beispielsweise $PtO_2$ genannt; komplexe Metallhydride wie z.B. $LiAlH_4$, $NaBH_4$, $NaBH_3CN$

Setzt man bei Verfahren e) als Verbindung der Formel VIII 5-Methyl-3-pyridylglyoxal und als Amin der Formel IX t-Butylamin ein, läßt sich Verfahren e) durch folgendes Formelschema wiedergeben.

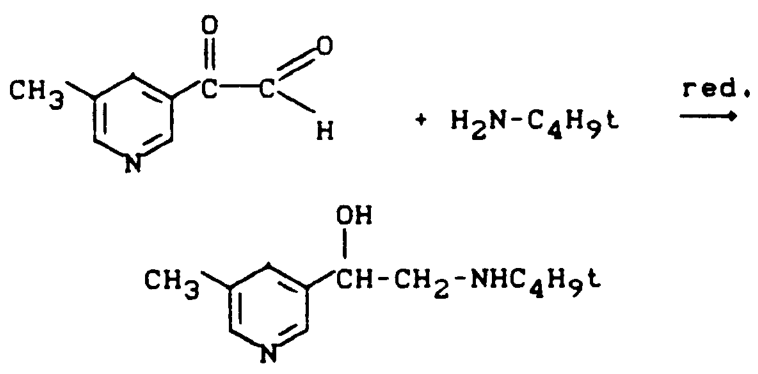


Die Substituenten $R^4$, $R^5$, $R^6$ in Formel VIII besitzen bevorzugt die weiter oben bei den Verbindungen der Formel I angegebenen bevorzugten Bedeutungen. Im einzelnen seien folgende Verbindungen der Formel VIII genannt:
2-Amino-3-chlor-5-pyridylglyoxal,
2-Amino-3-cyano-5-pyridylglyoxal,
2,4-Dichlor-3-amino-6-pyridylglyoxal,
2-Cyano-3-amino-6-pyridylglyoxal,
2-Chlor-3-amino-4-trifluormethyl-6-pyridylglyoxal,

Verfahren e) wird durchgeführt, indem man zur Verbindung der Formel VIII in einem Verdünnungsmittel etwa die äquivalente Menge des Amins der Formel IX zusetzt und anschließend reduziert.

Die Reaktion wird bei Temperaturen durchgeführt von 0 ° C bis 100 ° C.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B, Dimethylformamid, Dimethylacetamid und N-Methylpyrrolidon, sowie Tetramethylensulfon und Hexamethylphosphorsäuretriamid, weiterhin Alkohole wie Methanol, Ethanol, n- und i-Propanol.

Als Reduktionsmittel dienen

$H_2$/Katalysator; als Katalysator seien $PtO_2$ und Pd-Kohle genannt, ferner komplexe Metallhydride wie $LiAlH_4$ und $NaBH_4$.

Setzt man bei Verfahren f) als Verbindung der Formel X (5-Chlor-3-pyridyl)-hydroxyessigsäureisopropylamid ein, läßt sich Verfahren f) durch folgendes Reaktionsschema wiedergeben:

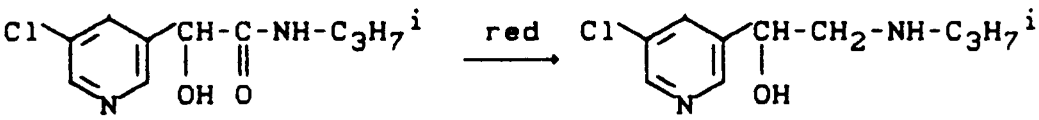


Verbindungen der Formel X sind neu. Sie lassen sich analog zu bekannten Verbindungen herstellen (T. Jen et al., J. Med. Chem. 20, 1258 (1977) und DE-OS 2 603 600).

Im einzelnen seien die folgenden Verbindungen der Formel X genannt:

(2-Amino-3-chlor-5-pyridyl)hydroxyessigsäureisopropylamid,
(2-Amino-3-cyano-5-pyridyl)hydroxyessigsäureisopropylamid,
(2-Amino-3-chlor-5-pyridyl)hydroxyessigsäure-tert.-butylamid,
(2-Chlor-3-amino-4-cyano-6-pyridyl)hydroxyessigsäureisopropylamid,
(2-Cyano-3-amino-4-chlor-6-pyridyl)hydroxyessigsäure-tert.-butylamid
(2-Cyano-3-amino-6-pyridyl)hydroxyessigsäureisopropylamid,
(2,4-Dichlor-3-amino-6-pyridyl)hydroxyessigsäure-tert.-butylamid
(2,6-Dichlor-4-pyridyl)hydroxyessigsäureisopropylamid (2,6-Dichlor-4-pyridyl)hydroxyessigsäure-tert.-butylamid,
(2-Cyano-4-pyridyl)hydroxyessigsäureisopropylamid, (2-Chlor-6-cyano-4-pyridyl)hydroxyessigsäure-tert.-butylamid.

Verfahren f) wird durchgeführt, indem man die Verbindung der Formel X in einem Verdünnungsmittel mit überschüssigem Reduktionsmittel umsetzt.

Die Reaktion wird bei Temperaturen von 0 ° C bis + 150 ° C durchgeführt.

Es wird bevorzugt bei Normaldruck gearbeitet.

Als Verdünnungsmittel dienen alle inerten organischen Losungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan.

Als Reduktionsmittel dienen komplexe Metallhydride wie $LiAlH_4$, Borane wie Diboran.

4. Es wurden ferner die neuen Halogenmethylketone der Formel II, in der $R^4$, $R^5$, $R^6$ die bei Formel I angegebene Bedeutung haben und $R^5$ zusätzlich zu den angegebenen Resten für $NO_2$ stehen kann, wobei $R^4$, $R^5$, $R^6$ aber nicht alle gleichzeitig für Wasserstoff stehen dürfen, gefunden.

5. Es wurde gefunden, daß sie sich herstellen lassen, indem man Acetylverbindungen der Formel XI, in welcher $R^4$, $R^5$, $R^6$ die oben (4) angegebene Bedeutung haben

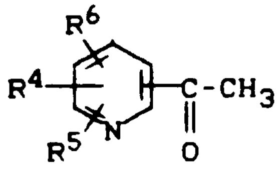

(XI)

a) mit elementarem Halogen umsetzt

b) mit Kupferhalogeniden der Formel $CuHal_2$ umsetzt.

Die Verbindungen der Formel XI sind bekannt (C.T. Gnewuch et al, J. Med. Chem. 15, 1321 (1972), US-Pat. 4 358 455) oder lassen sich analog zu bekannten Verfahren herstellen.

Im einzelnen seien folgende Verbindungen der Formel XI genannt:
2,6-Dichlor-4-acetylpyridin,
2-Cyano-4-acetylpyridin,
2-Amino-3-chlor-5-acetylpyridin,
2,4-Dichlor-3-amino-6-acetylpyridin,
2-Cyano-3-amino-6-acetylpyridin.

Setzt man bei Verfahren a) als Verbindung der Formel XI 2-Fluor-5-acetylpyridin und als Halogen Hal Brom ein, läßt sich die Reaktion durch folgendes Schema darstellen:

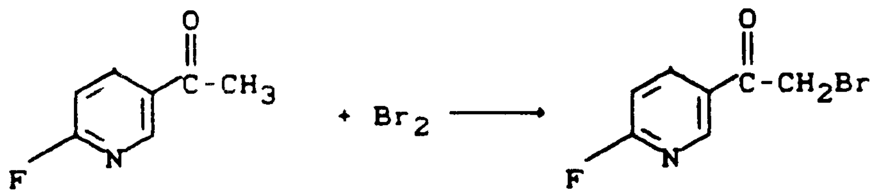


Verfahren a) wird durchgeführt, indem man zu Verbindung XI in einem Verdünnungsmittel die äquivalente Menge Halogen, eventuell in einem Verdünnungsmittel gelöst, zugibt.

Die Reaktion wird bei +20°C bis +150°C durchgeführt, bevorzugt bei der Siedetemperatur des verwendeten Verdünnungsmittels.

Es wird bevorzugt bei Normaldruck gearbeitet.

Als Verdünnungsmittel seien genannt: aliphatische, gegebenenfalls halogenierte Kohlenwasserstoffe wie Pentan, Hexan, Heptan, Cyclohexan, Methylenchlorid, Ethylenchlorid, Chloroform, Tetrachlorkohlenstoff, Alkohole wie Methanol, Ethanol, Ester wie Essigsäureethylester und Mischungen aus diesen Verdünnungsmitteln.

Setzt man bei Verfahren b) als Verbindung der Formel XI 3-Methoxy-5-acetylpyridin und als Verbindung der Formel $CuHal_2$ Kupfer(II)bromid ein, läßt sich die Reaktion durch folgendes Schema darstellen:

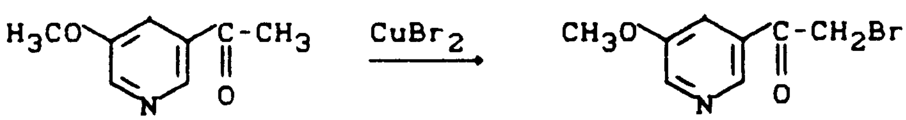


Verfahren b) wird durchgeführt, indem man äquivalente Mengen der Verbindung der Formel XI und der Verbindung $CuHal_2$ im Verdünnungsmittel 1-24 h, bevorzugt 6-12 h unter Rückfluß erhitzt.

Reaktionsparameter und Verdünnungsmittel wie bei Verfahren a).

6. Es wurden ferner die neuen Epoxide der Formel IV in welcher $R^4$, $R^5$, $R^6$ die bei den Verbindungen der Formel (I) angegebenen Bedeutungen besitzen, $R^5$ zusätzlich zu diesen Bedeutungen für $NO_2$ stehen kann und $R^4$, $R^5$, $R^6$ nicht gleichzeitig für Wasserstoff stehen dürfen, gefunden.

7. Es wurde gefunden, daß man sie erhält,

a) indem man Halogenmethylverbindungen der Formel V

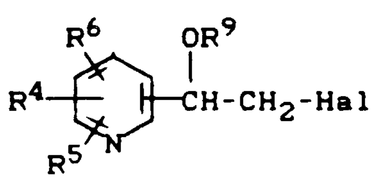

(V)

in welcher

| | |
|---|---|
| $R^4$, $R^5$, $R^6$ | und Hal die bei Verbindungen der Formel I angegebene Bedeutung haben und |
| $R^9$ | für Wasserstoff steht, |

mit Basen umsetzt, oder

b) indem man Aldehyde der Formel XII

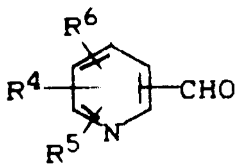

(XII)

in welcher

$R^4$, $R^5$, $R^6$ die bei Verbindungen der Formel I angegebene Bedeutung haben, mit Methylgruppenübertragenen Reagenzien in Gegenwart von Basen unter den Bedingungen der Corey-Epoxidierung (E.J. Corey und M. Chaykorsky, JACS 87, 1353 (1955))

umsetzt.

Verfahren a) wird durchgeführt, indem man eine Verbindung der Formel V in einem Verdünnungsmittel mit der 2-5 molaren, bevorzugt 2-4 molaren Menge einer Base umsetzt. Setzt man als Verbindung der Formel V 1-(2-Chlor-4-pyridyl)-2-bromethanolund als Base NaOH ein, läßt sich die Reaktion durch folgendes Schema darstellen:

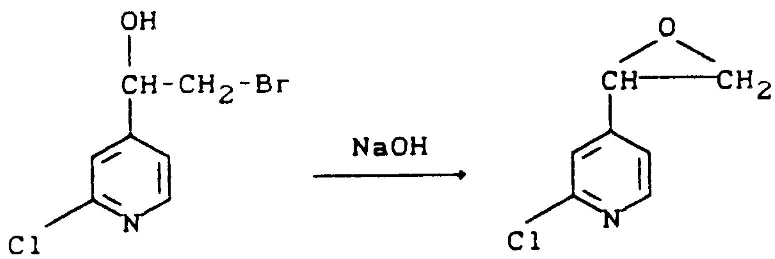


Im einzelnen seien folgende Verbindungen der Formel V genannt:
1-(2-Chlor-3-acetamido-6-pyridyl)-2-bromethanol,
1-(2-Acetamido-3-cyano-5-pyridyl)-2-chlorethanol.

Als Basen seien genannt: Alkali- und Erdalkalihydroxide wie Natriumhydroxid, Kaliumhydroxid; -carbonate und -hydrogencarbonate wie Natriumcarbonat, Natriumhydrogencarbonat, Bariumcarbonat; -alkoholate wie Natriummethylat und Natriumethylat.

Als Verdünnungsmittel seien genannt Alkohole, wie Methanol, Ethanol, Wasser, sowie Mischungen von Alkoholen mit Wasser.

Die Reaktion wird bei Temperaturen von 0 °C bis +100 °C durchgeführt, es wird bevorzugt unter Normaldruck gearbeitet.

Setzt man bei Verfahren b) als Verbindung der Formel XII 3-Methoxypyridin-5-aldehyd und als methylengruppenübertragendes Reagenz Trimethylsulfoniumiodid sowie als Base Natriumhydrid ein, läßt sich die Umsetzung durch folgendes Schema darstellen:

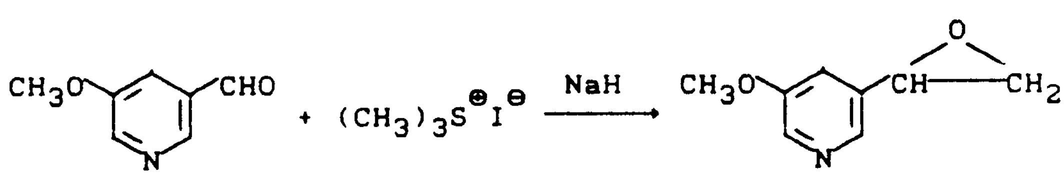


Im einzelnen seien folgende Verbindungen der Formel XII genannt:
2-Chlorpyridin-4-aldehyd,
2-Brompyridin-4-aldehyd,
2-Cyanopyridin-4-aldehyd,
2,6-Dichlorpyridin-4-aldehyd.

Als methylengruppenübertragende Reagentien seien genannt:
Trimethylsulfoniumhalogenide wie Trimethylsulfoniumchlorid, -bromid und -iodid,
Trimethylsulfoxoniumhalogenide wie Trimethylsulfoxoniumchlorid, -bromid und -iodid.

Als Basen werden verwendet: Alkali- und Erdalkalihydride wie Natriumhydrid, Alkali- und Erdalkalialkoholate wie Kalium-tert.-butylat.

Verfahren b) wird durchgeführt, indem man 1,1-Äquivalente der Base in Dimethylsulfoxid vorlegt, dann das methylengruppenübertragende Agens (1,1 Äquivalente) zufügt und zuletzt 1 Äquivalent der Verbindung der Formel XII zugibt.

Die Reaktion wird bei Temperaturen von 0 ° C bis 100 ° C, bevorzugt bei 50-70 ° C durchgeführt, es wird bevorzugt bei Normaldruck gearbeitet.

Als Verdünnungsmittel werden Dimethylsulfoxid oder Mischungen von Dimethylsulfoxid mit inerten organischen Lösungsmitteln eingesetzt.

Als inerte organische Lösungsmittel seien genannt: Ether wie Diethylether, Tetrahydrofuran, Dioxan.

8. Es wurden die neuen Aldehyde der Formel XII gefunden, in welcher $R^4$, $R^5$, $R^6$ die bei den Verbindungen der Formel I angegebenen Bedeutungen besitzen, und $R^5$ zusätzlich zu den angegebenen Resten für $NO_2$ stehen kann, wobei $R^4$, $R^5$, $R^6$ nicht gleichzeitig für Wasserstoff stehen dürfen.

9. Es wurde gefunden, daß man die neuen Aldehyde der Formel XII erhält, indem man

a) Alkohole der Formel XIII

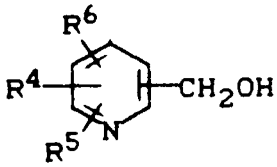

(XIII)

in welcher

$R^4$, $R^5$, $R^6$ die bei den Verbindungen der Formel I angegebene Bedeutung haben,

oxidiert, oder

b) indem man Säurechloride der Formel XIV

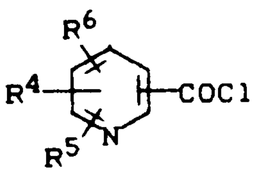

(XIV)

in welcher

$R^4$, $R^5$, $R^6$ die bei den Verbindungen der Formel I angegebene Bedeutung haben, reduziert.

Setzt man bei Verfahren a) als Alkohol der Formel XIII (2-Methoxy-4-pyridyl)methanol ein, läßt sich die Reaktion durch folgendes Schema darstellen:

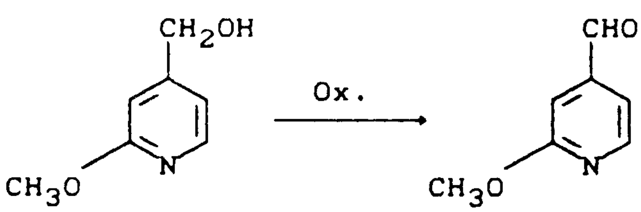


Die Verbindungen der Formel XIII sind bekannt oder lassen sich analog zu bekannten Verbindungen herstellen.

Im einzelnen seien folgende Verbindungen der Formel XIII genannt:

(2-Cyano-4-pyridyl)-methanol,
(2,6-Dichlor-4-pyridyl)-methanol,
(2-Acetamido-3-chlor-5-pyridyl)methanol,
(2,4-Dichlor-3-acetamido-6-pyridyl)-methanol,
(2-Amino-3-chlor-5-pyridyl)methanol,
(2,4-Dichlor-3-amino-6-pyridyl)methanol.

Als Oxidationsmittel zur Durchführung des Verfahrens a) seien genannt: aa) aktiviertes DMSO wie DMSO/Acetanhydrid, DMSO/Thionylchlorid, DMSO/Oxalylchlorid sowie ab) Mangandioxid.

Verfahren aa) wird durchgeführt, indem man den Alkohol der Formel XIII mit 1-1,5 Äquivalenten des Oxidationsmittels umsetzt.

Die Reaktion wird bei Temperaturen von -70 ° C bis +25 ° C durchgeführt.

Es wird bevorzugt bei Normaldruck gearbeitet.

Als Verdünnungsmittel dienen inerte organ. Lösungsmittel, beispielhaft seien genannt:
Gegebenenfalls chlorierte Kohlenwasserstoffe wie Methylenchlorid, Chlorform, Ether wie Diethylether und Tetrahydrofuran.

Verfahren ab) wird durchgeführt, indem man den Alkohol der Formel XIII mit überschüssigem Mangandioxid umsetzt.

Die Reaktion wird bei Temperaturen von +20 ° C bis +150 ° C durchgeführt, es wird bevorzugt bei Normaldruck gearbeitet.

Als Verdünnungsmittel werden verwendet: inerte organische Lösungsmittel, insbesondere aliphatische und aromatische gegebenenfalls halogenierte Kohlenstoffe wie Pentan, Hexan, Cyclohexan, Benzol, Toluol, Methylenchlorid, Chloroform, Ether wie Diethylether und Tetrahydrofuran, Ketone wie Aceton und Methylethylketon.

Setzt man bei Verfahren b) als Verbindung der Formel XIV 2-Chlor-4-pyridin-carbonsäurechlorid ein, läßt sich die Reaktion durch das folgende Schema darstellen:

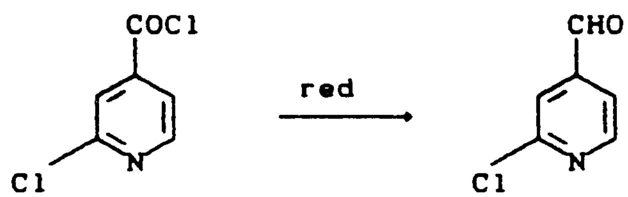


Verbindungen der Formel XIV sind bekannt (R. Graf. J. pr. Chem. 134, 177 (1932)) oder lassen sich analog zu bekannten Verfahren herstellen.

Im einzelnen seien folgende Verbindungen der Formel XIV genannt:
2-Chlornicotinsäurechlorid,
2-Chlorisonicotinsäurechlorid,
2,6-Dichlorisonicotinsäurechlorid.

Als Reduktionsmittel dient $H_2$/Katalysator, als Katalysator sei beispielhaft Palladium auf Bariumsulfat genannt.

Verfahren b) wird durchgeführt, indem man durch eine Lösung der Verbindung XIV in einem siedenden Verdünnungsmittel nach Zugabe von 5-10 mol-% Katalysator einen Wasserstoffstrom leitet.

Die Reaktion wird bei Temperaturen von 100-200 ° C durchgeführt, es wird bevorzugt bei Normaldruck gearbeitet.

Als Verdünnungsmittel dienen aliphatische und aromatische Kohlenwasserstoffe. Beispielhaft seien genannt: Hexan, Heptan, Cyclohexan, Benzol, Toluol, Xylole.

Verbindungen der Formel XIII sind zum Teil bekannt (G.R. Newcome, H.W. Lee, J. Org. Chem. 47, 2800 (1982)).

10. Es wurden die neuen Verbindungen der Formel XIII in welcher $R^4$, $R^5$, $R^6$ die bei den Verbindungen der Formel (I) angegebenen Bedeutungen besitzen, $R^5$ zusätzlich zu diesen Bedeutungen für $NO_2$ stehen kann und $R^4$, $R^5$, $R^6$ nicht gleichzeitig für Wasserstoff stehen dürfen, sowie der Rest $R^5$ nicht für $NH_2$ oder $-CH_2-OH$ stehen darf, wenn $R^4$ und $R^6$ für Wasserstoff stehen.

11. Es wurde gefunden, daß man die neuen Verbindungen der Formel XIII erhält, indem man Verbindungen der Formel XV bzw. XVI

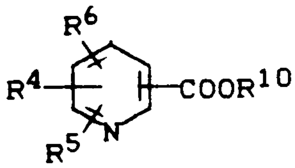 (XV) 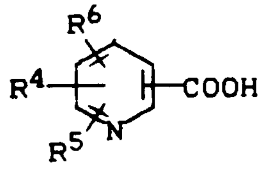 (XVI)

in welcher

$R^4$, $R^5$, $R^6$ die oben angegebene Bedeutung haben, und

$R^{10}$ für $C_1$-$C_4$-Alkyl steht, reduziert.

Im einzelnen seien folgende Verbindungen der Formel XV und XVI genannt:

2,6-Dichlorpyridin-4-carbonsäure,
2,6-Dichlorpyridin-4-carbonsäureethylester,
2-Amino-5-pyridin-carbonsäure,
2-Amino-3-chlor-5-pyridincarbonsäure,
2,4-Dichlor-3-amino-6-pyridincarbonsäure.

Als Reduktionsmittel dienen:

für Verbindungen der Formel XV, komplexe Metallhydride wie z.B. $LiAlH_4$,
für Verbindungen der Formel XVI, Borane wie z.B. Diboran, komplexe Metallhydride, wie z.B. $LiAlH_4$.

Das Verfahren wird durchgeführt, indem man die Verbindungen der Formel XV bzw. XVI in einem Verdünnungsmittel mit der 1-4 molaren Menge Reduktionsmittel umsetzt.

Die Reaktion wird bei Temperaturen von -50°C bis +100°C durchgeführt, es wird bevorzugt bei Normaldruck gearbeitet.

Als Verdünnungsmittel dienen:

Ether wie Diethylether, Tetrahydrofuran, Dioxan.

12. Es wurden die neuen Verbindungen der Formel XVI gefunden, in der $R^4$, $R^5$, $R^6$ die bei den Verbindungen der Formel I angegebene Bedeutung besitzen und $R^5$ zusätzlich zu den angegebenen Resten für $NO_2$ stehen kann, wobei $R^4$, $R^5$, $R^6$ nicht gleichzeitig für Wasserstoff stehen dürfen.

13. Es wurde gefunden, daS man die neuen Verbindungen der Formel XVI erhält, indem man Methylpyridine der Formel XVII

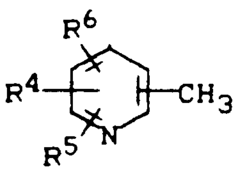 (XVII)

in welcher

$R^4$, $R^5$, $R^6$ die oben angegebene Bedeutung haben, oxidiert.

Setzt man bei dem Verfahren als Verbindung der Formel XVII 2,3-Dichlor-4-methylpyridin ein, läßt sich die Reaktion durch das folgende Schema darstellen:

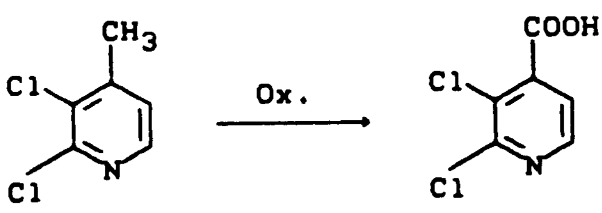


Die Verbindungen der Formel XVII sind bekannt (E.C. Taylor et al., J. Org. Chem. 48, 4852 (1983).

Im einzelnen seien folgende Verbindungen der Formel XVII genannt:

2-Amino-3-chlor-5-methylpyridin,
2-Amino-3-cyano-5-methylpyridin,

2,4-dichlor-3-amino-6-methylpyridin,
2-Chlor-3-amino-4-cyano-6-methylpyridin,
2-Chlor-3-amino-4-trifluormethyl-6-methylpyridin.

Als Oxidationsmittel werden die literaturbekannten Reagenzien (H. Henecka in Houben-Weyl, Bd. 8, S. 385ff, G. Thieme-Verlag, Stuttgart, 1972) zur Umwandlung von Methyl- in Carboxylgruppen eingesetzt.

Beispielhaft seien genannt: Chromsäure in Eisessig, Chromsäure in Schwefelsäure, Dichromat/Schwefelsäure, alkalische Permanganat-Lösung.

Die Reaktion wird durchgeführt, indem man die Verbindung der Formel XVII in wäßrig-saurer oder wäßrig alkalischer Lösung mit überschüssigem Oxidationsmittel behandelt.

Die Umsetzung wird bei Temperaturen von +20 bis +120°C durchgeführt, es wird bevorzugt bei Normaldruck gearbeitet.

14. Es wurden die neuen Verbindungen der Formel XIV gefunden, in der die Reste $R^4$, $R^5$, $R^6$ die bei den Verbindungen der Formel I angegebene Bedeutung besitzen, und $R^5$ zusätzlich zu den angegebenen Resten für $NO_2$ stehen kann, wobei $R^4$, $R^5$, $R^6$ nicht gleichzeitig für Wasserstoff stehen dürfen und zwei der Reste $R^4$, $R^5$, $R^6$ nicht gleichzeitig für Halogen stehen dürfen.

15. Es wurde gefunden, daß man die neuen Verbindungen der Formel XIV erhält, indem man Carbonsäuren der Formel XVI

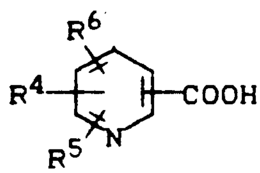

(XVI)

in welcher

$R^4$, $R^5$, $R^6$ die oben angegebene Bedeutung haben, nach bekannten Verfahren (H. Henecka in Houben-Weyl, Bd. 8, S. 359 ff, G. Thieme-Verlag, Stuttgart 1952) mit Halogenierungsmitteln umsetzt.

Setzt man bei dem Verfahren als Carbonsäure der Formel XVI 2-Brompyridin-5-carbonsäure ein, läßt sich die Reaktion durch das folgende Schema darstellen:

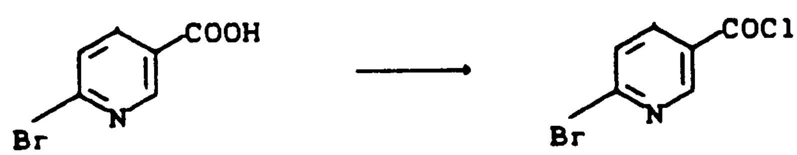

Im einzelnen seien die folgenden Verbindungen der Formel XVI genannt:
2,6-Dichlorpyridin-4-carbonsäure,
2-Acetamidopyridin-5-carbonsäure,
2-Acetamido-3-chlorpyridin-5-carbonsäure,
2,4-Diclor-3-acetamidopyridin-6-carbonsäure.

Als Halogenierungsmittel werden anorganische Säurechloride verwendet. Beispielhaft seien genannt: Phosphoroxichlorid, Phosphorpentachlorid, Thionylchlorid.

Die Reaktion wird durchgeführt, indem man eine Verbindung der Formel XVI mit 0,5-1,5 Äquivalenten des anorganischen Säurechlorids, gegebenenfalls in einem Verdünnungsmittel, behandelt.

Die Umsetzung wird bei Temperaturen von 20°C bis 100°C durchgeführt, es wird bevorzugt bei Normaldruck gearbeitet. Als Verdünnungsmittel können alle inerten organischen Lösungsmittel verwendet werden. Hierzu zählen insbesondere aliphatische und aromatische gegebenenfalls halogenierte Kohlenwasserstoffe wie Pentan, Hexan, Cylcohexan, Benzol, Toluol, Methylenchlorid, Chloroform, Chlorbenzol; Ether wie Diethylether, Tetrahydrofuran, Dioxan sowie Phosphoroxichlorid.

Bevorzugt wird ohne Verdünnungsmittel gearbeitet.

Verbindungen der Formel XV sind z.T. bekannt (F. Parravicini et al., Eur. J. Med. Chem.-Chim. Ther. 10, 252 (1975)).

16. Es wurden die neuen Verbindungen der Formel XV gefunden, in welcher $R^4$, $R^5$, $R^6$ die bei den

Verbindungen der Formel I angegebene Bedeutung besitzen, und $R^5$ zusätzlich zu den angegebenen Resten für $NO_2$ stehen kann, wobei $R^4$, $R^5$, $R^6$ nicht gleichzeitig für Wasserstoff stehen.

17. Es wurde gefunden, daß man die neuen Verbindungen der Formel XV erhält,

a) indem man Carbonsäurechloride der Formel XIV,

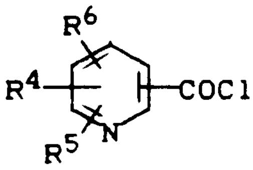

(XIV)

in welcher

$R^4$, $R^5$, $R^6$ die oben angegebene Bedeutung haben,

mit Alkoholen der Formel XVIII

$R^{10}$-OH (XVIII)

in welcher

$R^{10}$ die oben angegebene Bedeutung hat,

in Gegenwart von Säureakzeptoren umsetzt,

b) indem man Carbonsäuren der Formel XVI

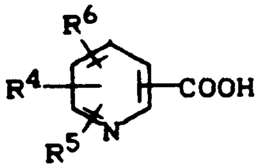

(XVI)

in welcher

$R^4$, $R^5$, $R^6$ die oben angegebene Bedeutung haben,

wie weiter oben beschrieben in die Carbonsäurechloride der Formel (XIV) überführt und diese direkt, wie bei Verfahren a) beschrieben, ohne Isolierung zu den Estern XV umsetzt,

c) indem man Carbonsäuren der Formel XVI

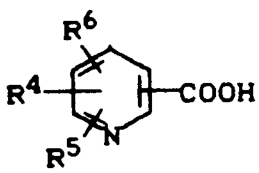

(XVI)

in welcher

$R^4$, $R^5$, $R^6$ die oben angegebene Bedeutung haben,

mit Alkoholen der Formel XVIII

$R^{10}$-OH (XVIII)

in welcher

$R^{10}$ die oben angegebene Bedeutung hat,

in Gegenwart wasserentziehender Mittel umsetzt.

Setzt man bei Verfahren a) als Carbonsäurechlorid der Formel XIV 3-Acetamidopyridin-6-carbonsäurechlorid und als Alkohol der Formel XVIII Ethanol ein, läßt sich die Reaktion durch das folgende Schema darstellen:

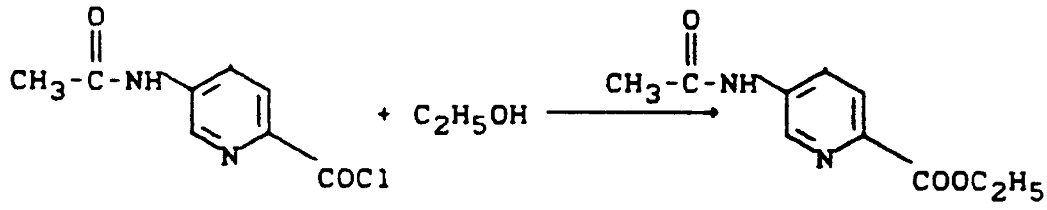

Die Substituenten $R^4$, $R^5$ und $R^6$ in Formel XIV besitzen bevorzugt die weiter oben bei den Verbindungen der Formel I angegebenen bevorzugten Bedeutungen. Im einzelnen seien folgende Verbindungen der Formel XIV genannt:

2,6-Dichlorpyridin-4-carbonsäurechlorid,
2-Acetamidopyridin-5-carbonsäurechlorid,
2-Acetamido-3-chlorpyridin-5-carbonsäurechlorid,
2-Acetamido-3-cyanopyridin-5-carbonsäurchlorid,
2,4-Dichlor-3-acetamidopyridin-6-carbonsäurechlorid.

Alkohole der Formel XVIII sind bekannt. Der Substituent $R^{10}$ besitzt die weiter oben angegebenen bevorzugten Bedeutungen. Im einzelnen seien folgende Verbindungen der Formel XVIII genannt: Methanol, Ethanol, n-Propanol.

Als Säureakzeptoren können alle üblichen Säurebindemittel verwendet werden. Hierzu gehören vorzugsweise Alkalicarbonate, -hydroxide wie Natrium- und Kaliumcarbonat, Natrium- und Kaliumhydroxid, ferner aliphatische, aromatische oder heterocyclische Amine, beispielsweise Trimethylamin, Triethylamin, Dimethylbenzylamin, Pyridin und 4-Dimethylaminopyridin.

Verfahren a) wird durchgeführt, indem man zur Verbindung XIV in einem Verdünnungsmittel eine Mischung von äquivalenten Mengen der Verbindung XVIII und des Säureakzeptors zugibt. Die Reaktion wird bei Temperaturen von 0 °C bis 100 °C durchgeführt.

Es wird bevorzugt bei Normaldruck gearbeitet.

Als Verdünnungsmittel dienen entweder der Alkohol XVIII selbst oder andere inerte organische Lösungsmittel.

Hierzu gehören insbesondere gegebenenfalls halogenierte aliphatische und aromatische Kohlenwasserstoffe wie Methylenchlorid, Chloroform, Chlorbenzol; Ether wie Diethylether, Tetrahydrofuran, Dioxan; Nitrile wie Acetonitril und Benzonitril.

Setzt man bei Verfahren b) als Carbonsäure der Formel XVI 2-Chlorpyridin-4-carbonsäure, als anorganisches Säurechlorid Thionylchlorid und als Alkohol der Formel XVIII Methanol ein, läßt sich die Reaktion durch das folgende Schema darstellen:

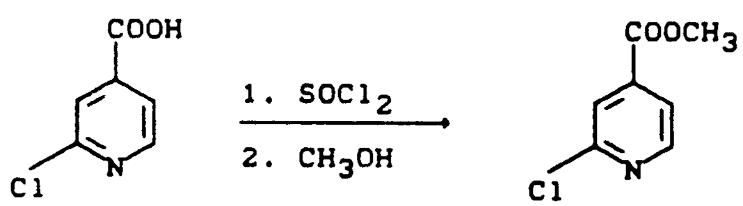


Die Substituenten $R^4$, $R^5$, $R^6$ besitzen die weiter oben angegebene Bedeutung. Bevorzugt sind die bei Verfahren 15 genannten Carbonsäuren der Formel XVI.

Als Verbindungen der Formel XVIII werden die weiter oben als bevorzugt genannten Alkohole eingesetzt.

Als Halogenierungsmittel dienen die weiter oben als bevorzugt genannten anorganischen Säurechloride.

Als Säureakzeptoren werden die weiter oben als bevorzugt genannten Verbindungen eingesetzt.

Verfahren b) wird durchgeführt, indem man zunächst, wie oben beschrieben, die Carbonsäure der Formel XVI in das Säurechlorid XIV überführt. Dann wird überschüssiges Halogenierungsmittel abgezogen, das rohe Säurechlorid in einem Verdünnungsmittel aufgenommen und, wie bei Verfahren a) beschrieben, mit der Mischung von äquivalenten Mengen Alkohol der Formel XVIII und Säureakzeptor umgesetzt.

Als Verdünnungsmittel dienen bevorzugt die bei Verfahren a) genannten.

Setzt man bei Verfahren c) als Carbonsäure der Formel XVI 3-Fluorpyridin-5-carbonsäure und als Alkohol der Formel XVIII Methanol ein, läßt sich Verfahren c) durch folgendes Reaktionsschema wiederge-

ben:

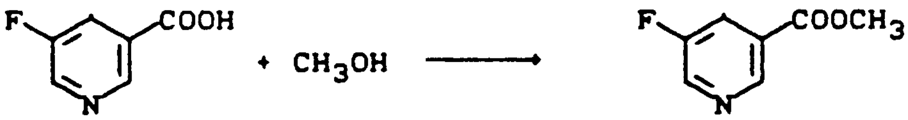

Als Verbindungen der Formel XVI werden die weiter oben genannten bevorzugt eingesetzt.

Als Alkohole der Formel XVIII werden die weiter oben genannten bevorzugt eingesetzt.

Als Kondensationsmittel dienen Carbodiimide, wie z.B. Dicyclohexylcarbodiimid.

Verfahren c) wird durchgeführt, indem man äquivalente Mengen der Verbindungen XVI, XVIII und des Kondensationsmittels in einem Verdünnungsmittel umsetzt.

Die Reaktion wird bei Temperaturen von -30 °C bis +100 °C durchgeführt.

Es wird bevorzugt bei Normaldruck gearbeitet.

Als Verdünnungmittel dienen inerte organische Lösungsmittel. Hierzu gehören gegebenenfalls halogenierte Kohlenwasserstoffe wie Methylenchlorid, Chloroform; Ether wie Diethylether, Tetrahydrofuran, Nitrile wie Acetonitril und Benzonitril, Amide wie Dimethylformamid.

Die Verbindungen der Formel V sind z.T. bekannt (L. P. Friz, Fanuaco XVII, 972 (1963), EP 120 770).

18. Es wurden die neuen Verbindungen der Formel V gefunden, in welcher die Reste $R^4$, $R^5$, $R^6$ und $R^9$ die bei den Verbindungen der Formel I angegebenen Bedeutungen besitzen und $R^5$ zusätzlich zu den angegebenen Resten für $NO_2$ stehen kann, wobei $R^4$, $R^5$, $R^6$ nicht gleichzeitig für Wasserstoff stehen dürfen und der Rest $R^5$ nicht für $NH_2$ oder $CH_2$-OH stehen darf, wenn $R^4$ und $R^6$ für Wasserstoff stehen.

19. Es wurde gefunden, daß man die neuen Verbindungen der Formel V erhält, indem man

a) wenn $R^9$ für Wasserstoff steht, Halogenmethylketone der Formel II

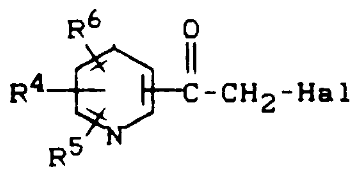

(II)

in welcher

$R^4$, $R^5$, $R^6$ und Hal die oben angegebene Bedeutung haben,

reduziert,

b) wenn $R^9$ für $C_1$-$C_4$-Alkyl steht, Vinylpyridine der Formel XIX

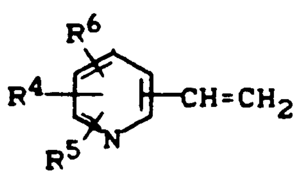

(XIX)

in welcher

$R^4$, $R^5$, $R^6$ die oben angegebene Bedeutung haben,

mit N-Halogenacetamiden der Formel XX,

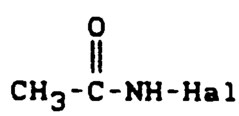

(XX)

in welcher

Hal für Halogen steht,

und Alkoholen der Formel $R^9$-OH
umsetzt.

Setzt man bei Verfahren a) als Verbindung der Formel II (2-Cyano-4-pyridyl)chlormethylketon ein, läßt sich Verfahren a) durch das folgende Reaktionsschema darstellen:

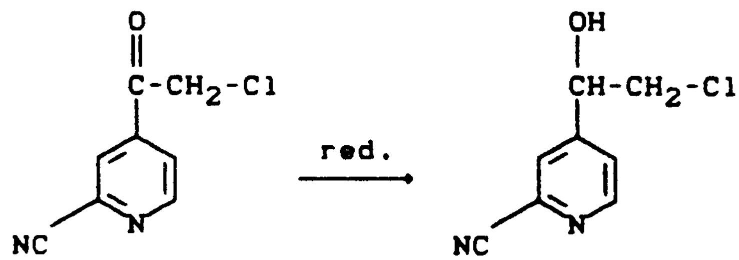


Die Substituenten $R^4$, $R^5$, $R^6$ und Hal in den Verbindungen der Formel II haben die weiter oben angegebenen bevorzugten Bedeutung.

Als Reduktionsmittel zur Durchführung des Verfahrens a) seien genannt:
$H_2$/Katalysator, als Katalysatoren seien genannt: $PtO_2$, Pd/Kohle; komplexe Metallhydride, wie z.B. $LiAlH_4$, $NaBH_4$, $NaBH_3CN$. Bevorzugt werden $NaBH_4$ und $NaBH_3CN$ eingesetzt.

Verfahren a) wird durchgeführt, indem die Verbindung II in einem Verdünnungsmittel mit dem Reduktionsmittel umsetzt.

Die Reaktion wird bei Temperaturen von -20 °C bis +100 °C durchgeführt.

Es wird bevorzugt bei Normaldruck gearbeitet.

Als Verdünnungsmittel dienen alle inerten organischen Lösungsmittel. Hierzu zählen insbesondere gegebenenfalls halogenierte aliphatische und aromatische Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril, Benzonitril; Alkohole wie Methanol, Ethanol, n- und i-Propanol. Bevorzugt werden Alkohole eingesetzt.

Setzt man bei Verfahren b) als Verbindung der Formel XIX 2-Brom-4-vinyl-pyridin, als N-Halogenacetamid der Formel XX N-Bromacetamid ein und als Alkohol der Formel $R^9$-OH Methanol, läßt sich Verfahren b) durch das folgende Reaktionsschema darstellen:

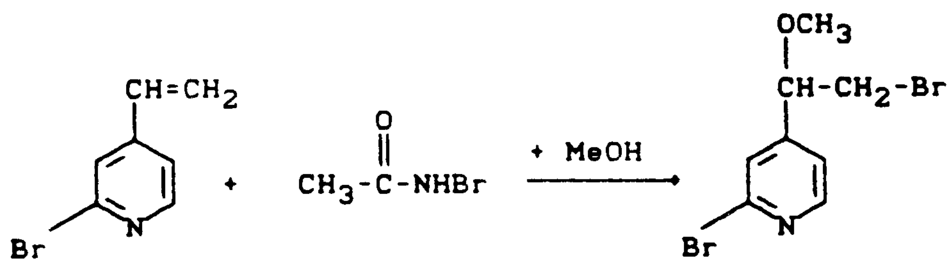


Verbindungen der Formel XIX sind z.T. bekannt (EP 120 770) oder lassen sich nach bekannten Verfahren herstellen.

Im einzelnen seien folgende Verbindungen der Formel XIX genannt:
2,6-Dichlor-4-vinylpyridin,
2-Acetamido-3-chlor-5-vinylpyridin,
2-Acetamido-3-cyano-5-vinylpyridin.

N-Halogenacetamide der Formel XX sind bekannt.

Im einzelnen seien folgende Verbindungen der Formel XX genannt:
N-Chloracetamid,
N-Bromacetamid.

Verfahren b) wird durchgeführt, indem man äquimolare Mengen der Verbindungen XIX und XX mit dem entsprechendem Alkohol(überschuß), 5-10 Mol, mit oder ohne Verdünnungsmittel zusammengibt.

Die Reaktion wird bei Temperaturen von -20 °C bis +80 °C durchgeführt.

Als Verdünnungsmittel dienen alle inerten organischen Lösungsmittel. Hierzu gehören insbesondere

aliphatische und aromatische gegebenenfalls halogenierte Kohlenwasserstoffe wie Pentan, Hexan, Heptan, Cyclohexan, Benzol, Toluol, Methylenchlorid, Ethylenchlorid, Chloroform; Ether wie Diethylether, Tetrahydrofuran, Dioxan; Nitrile wie Acetonitril, Benzonitril.

Verbindungen der Formel VI sind z.T. bekannt (F. Zymalkowski et al., Arch. Pharm. 294, 29 (1961)).

20. Es wurden die neuen Verbindungen der Formel VI gefunden, in welcher die Reste $R^4$, $R^5$, $R^6$ die bei den Verbindungen der Formel I angegebenen Bedeutungen besitzt, und $R^5$ zusätzlich zu den angegebenen Resten für $NO_2$ stehen kann, wobei $R^4$, $R^5$, $R^6$ nicht gleichzeitig für Wasserstoff stehen dürfen.

21. Es wurde gefunden, daß man die neuen Verbindungen der Formel VI erhält, indem man Nitroverbindungen der Formel XXI

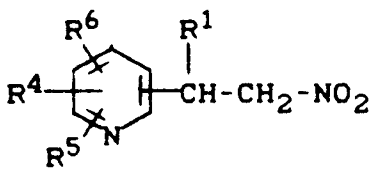

(XXI)

in welcher

$R^1$, $R^4$, $R^5$, $R^6$ die oben angegebene Bedeutung haben,

reduziert.

Setzt man bei dem Verfahren als Nitroverbindung der Formel XXI 1-(2-Brom-4-pyridyl)-2-nitroethanol ein, läßt sich die Reaktion durch das folgende Schema darstellen:

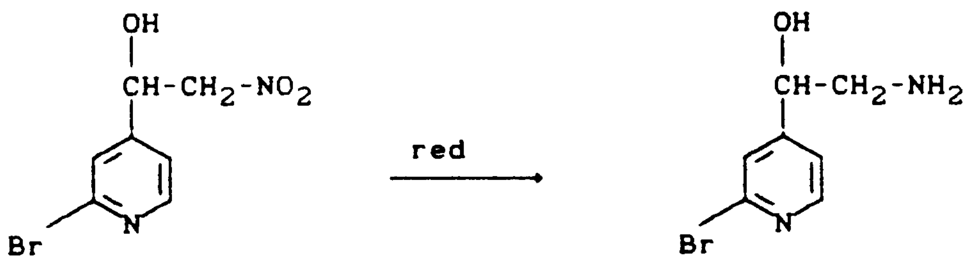


Die Verbindungen der Formel XXI sind bekannt (K.W. Merz et al., Arch. Pharm. 297, 10, (1964)) oder lassen sich analog zu bekannten Verfahren herstellen. Die Substituenten $R^1$, $R^4$, $R^5$ und $R^6$ in Formel XXI besitzen bevorzugt die weiter oben bei den Verbindungen der Formel I angegebenen bevorzugten Bedeutungen.

Im einzelnen seien folgende Verbindungen der Formel XXI genannt:

1-(2-Cyano-4-pyridyl)-2-nitroethanol,
1-(2-Amino-3-chlor-5-pyridyl)-2-nitroethanol,
1-(2-Amino-3-cyano-5-pyridyl)-2-nitroethanol,
1-(2,4-Dichlor-3-amino-6-pyridyl)-2-nitroethanol.

Als Reduktionsmittel für das Verfahren dient Wasserstoff/Katalysator. Als Katalysatoren seien beispielhaft genannt: Raney-Nickel, $PtO_2$, Pd/Kohle.

Das Verfahren wird durchgeführt, indem man Verbindung XXI in einem Verdünnungsmittel unter Zusatz einer Säure katalytisch hydriert.

Die Reaktion wird bei Temperaturen von +20 ° C bis +150 ° C durchgeführt.

Es wird unter Normaldruck oder erhöhtem Druck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Benzol, Toluol; Ether wie Diethylether, Tetrahydrofuran; Alkohole wie Methanol, Ethanol.

Als Säuren werden verwendet: anorganische Säuren wie Kohlensäure; Halogenwasserstoffsäuren wie Chlorwasserstoff; Schwefelsäure, organische Säuren wie Essigsäure, Propionsäure.

22. Es wurden die neuen Verbindungen der Formel XXI gefunden, in welcher $R^1$, $R^4$, $R^5$, $R^6$ die bei den Verbindungen der Formel I angegebenen Bedeutungen besitzen, und $R^5$ zusätzlich zu den angegebenen Resten für $NO_2$ stehen kann, wobei $R^4$, $R^5$, $R^6$ nicht gleichzeitig für Wasserstoff stehen dürfen.

23. Es wurde gefunden, daß man die neuen Verbindungen der Formel XXI erhält,

a) wenn $R^1$ für OH steht, indem man Aldehyde der Formel XII

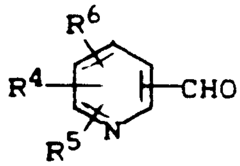

(XII)

in welcher

$R^4$, $R^5$, $R^6$ die oben angegebene Bedeutung haben,

mit Nitromethan umsetzt,

b) wenn $R^1$ für Acyloxy steht, indem man die nach Verfahren a) erhaltenen Nitroalkohole acyliert,

c) wenn $R^1$ für Alkoxy steht, indem man Nitroolefine der Formel XXII,

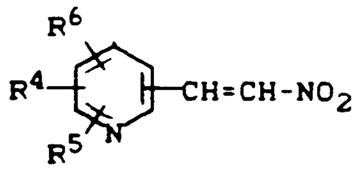

(XXII)

in welcher $R^4$, $R^5$, $R^6$ die oben angegebene Bedeutung haben, mit Alkoholaten umsetzt.

Setzt man bei Verfahren a) als Aldehyd der Formel XII 2,3-Dichlorpyridin-4-aldehyd ein, läßt sich Verfahren a) durch das folgende Schema darstellen:

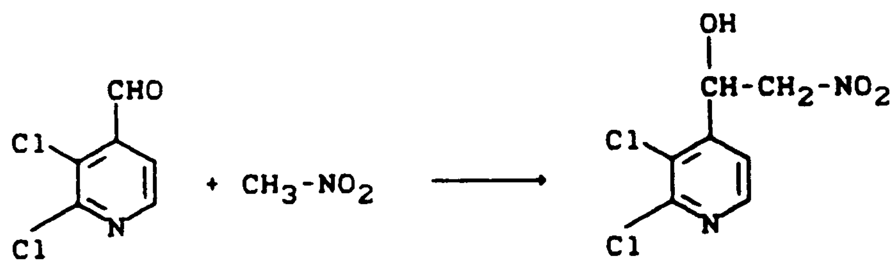

Als Aldehyde der Formel XII werden bevorzugt die weiter oben genannten Verbindungen eingesetzt.

Das Verfahren a) wird durchgeführt, indem man äquivalente Mengen der Verbindung XII und Nitromethan in einem Verdünnungsmittel in Gegenwart einer Base umsetzt.

Die Reaktion wird bei Temperaturen von -20 ° C bis +50 ° C durchgeführt.

Es wird bevorzugt bei Normaldruck gearbeitet.

Als Verdünnungsmittel dienen alle inerten organischen Lösungsmittel. Hierzu gehören insbesondere Ether wie Diethylether, Tetrahydrofuran; Alkohole wie Methanol, Ethanol.

Als Basen werden verwendet: Alkali- und Erdalkalihydroxide wie Natrium- und Kaliumhydroxid, -alkoholate wie Natrium- und Kaliummethylat, Natrium- und Kaliumethylat.

Setzt man bei Verfahren b) als Nitroalkohol der Formel XII ($R^1$ = OH)1-(2-Brom-4-pyridyl)-2-nitroethanol und als Acylierungsmittel Acetylchlorid ein, läßt sie die Reaktion durch das folgende Schema darstellen:

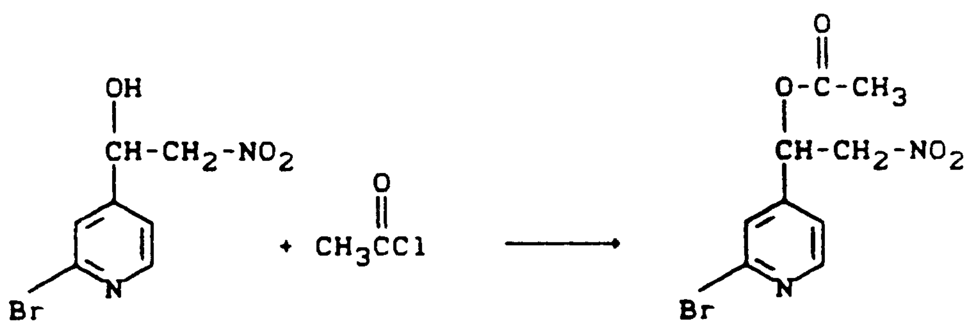

Als Nitroalkohole der Formel XII ($R^1$ = OH) werden bevorzugt die weiter oben genannten Verbindungen eingesetzt.

Verfahren b) wird durchgeführt, indem man äquivalente Mengen der Verbindung XII ($R^1$ = OH) und des Acylierungsmittels in einem Verdünnungsmittel in Gegenwart eines Säureakzeptors umsetzt.

Die Reaktion wird bei Temperaturen von -20 ° C bis +100 ° C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische gegebenenfalls halogenierte Kohlenwasserstoffe wie Pentan, Hexan, Cyclohexan, Benzol, Toluol, Methylenchlorid, Ethylenchlorid, Chloroform, Chlorbenzol; Ether wie Diethylether, Tetrahydrofuran, Dioxan; Nitrile wie Acetonitril, Benzonitril.

Als Säureakzeptoren können alle üblichen Säurebindemittel verwendet werden. Hierzu zählen Alkali- und Erdalkalimetallcarbonate wie Natrium- und Kaliumcarbonat, -hydroxide wie Natrium- und Kaliumhydroxid, sowie aliphatische, aromatische und heterocyclische Amine wie Trimethylamin, Triethylamin, Diethylbenzylamin, Pyridin, 4-Dimethylaminopyridin.

Setzt man bei Verfahren c) als Nitroolefin der Formel XXII 1-(2-Acetamido-5-pyridyl)-2-nitroethen und als Alkoholat Natriummethylat ein, läßt sich Verfahren c) durch das folgende Schema darstellen:

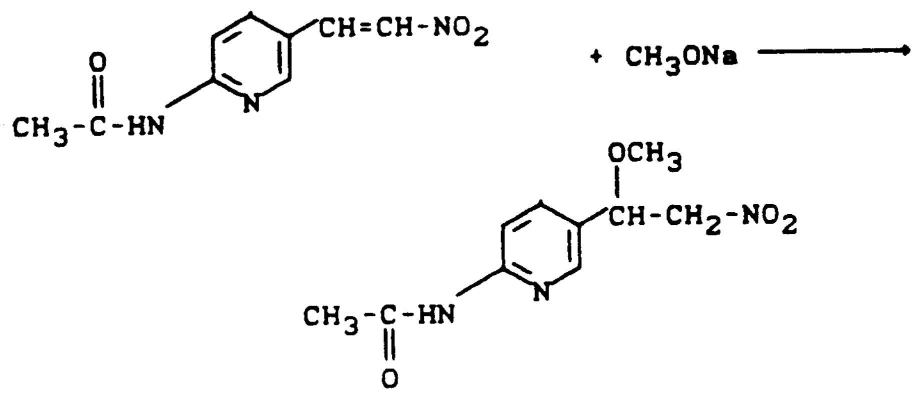

Die Substituenten $R^4$, $R^5$, $R^6$ der Nitroolefine der Formel XXII besitzen die weiter oben bei den Verbindungen der Formel I angegebenen bevorzugten Bedeutungen.

Im einzelnen seien die folgenden Verbindungen der Formel XXII genannt:

1-(2-Chlor-4-pyridyl)-2-nitroethen

1-(2-Acetamido-3-chlor-5-pyridyl)-2-nitroethen,

1-(2-Acetamido-3-cyano-5-pyridyl)-2-nitroethen.

Die bei der Reaktion eingesetzten Alkoholate sind bekannt. Im einzelnen seien genannt: Natrium- und Kaliummethylat, Natrium- und Kaliumethylat.

Verfahren c) wird durchgeführt, indem man äquivalente Mengen der Verbindung XXII und des Alkoholats in einem Verdünnungsmittel umsetzt.

Die Reaktion wird bei Temperaturen von 0 ° C bis 80 ° C durchgeführt.

Es wird bevorzugt bei Normaldruck gearbeitet.

Als Verdünnungsmittel dienen alle inerten organischen Lösungsmittel. Hierzu zählen insbesondere Ether, wie Diethylether, Tetrahydrofuran; Ester wie Essigsäuremethylester, Essigsäureethylester; Alkohole, wie Methanol, Ethanol.

Es wird bevorzugt in Alkoholen gearbeitet.

Verbindungen der Formel XXII sind z.T. bekannt (G. Jones Org. React. 15, 254 (1967).

24. Es wurden die neuen Verbindungen der Formel XXII gefunden, in welcher $R^4$, $R^5$, $R^6$ die bei den Verbindungen der Formel I angegebenen Bedeutungen besitzen, und $R^5$ zusätzlich zu den angegebenen Resten für $NO_2$ stehen Kann, wobei $R^4$, $R^5$, $R^6$ nicht gleichzeitig für Wasserstoff stehen dürfen.

25. Es wurde gefunden, daß man die neuen Verbindungen der Formel XXII erhält, indem man Aldehyde der Formel XII,

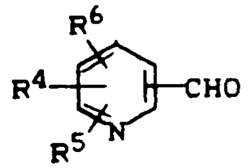

(XII)

in welcher

$R^4$, $R^5$, $R^6$ die oben angegebene Bedeutung haben, mit

Nitromethan in Gegenwart von Basen kondensiert.

Setzt man bei dem Verfahren als Aldehyd der Formel XII 2-Brom-pyridin-5-aldehyd ein, läßt sich das Verfahren durch das folgende Schema darstellen:

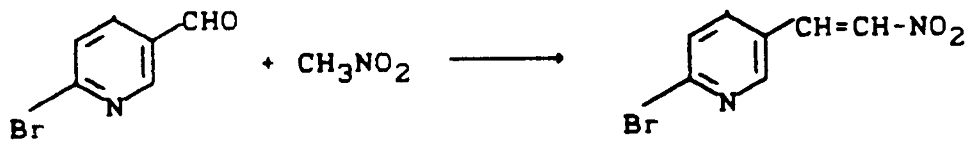

Als Aldehyde der Formel XII werden bevorzugt die weiter oben genannten Verbindungen eingesetzt.

Das Verfahren wird durchgeführt, indem man äquivalente Mengen der Verbindung XII und Nitromethan in einem Verdünnungsmittel unter Zusatz einer Base umsetzt.

Die Reaktion wird bei Temperaturen von 0 ° C bis +150 ° C durchgeführt.

Es wird bevorzugt bei Normaldruck gearbeitet.

Als Verdünnungsmittel dienen aromatische Kohlenwasserstoffe wie Benzol, Toluol, Xylol; Alkohole wie Methanol, Ethanol, organische Carbonsäuren wie Essigsäure.

Als Basen werden verwendet: Primäre Amine wie Methylamin, Ethylamin, Kombinationen von Hydrochloriden primärer Amine wie Methylaminhydrochlorid, Ethylaminhydochlorid mit Natriumcarbonat; sekundäre Amine wie Piperidin, tertiäre Amine wie Triethylamin; Ammoniumsalze organischer Carbonsäuren wie Ammoniumacetat.

26. Es wurden die neuen Verbindungen der Formel VIII gefunden, in welcher $R^4$, $R^5$, $R^6$ die bei den Verbindungen der Formel I angegebenen Bedeutungen besitzen und $R^5$ zusätzlich zu den angegebenen Resten für $NO_2$ stehen kann, wobei $R^4$, $R^5$, $R^6$ nicht gleichzeitig für Wasserstoff stehen dürfen.

27. Es wurde gefunden, daß man die neuen Verbindungen der Formel VIII erhält, indem man Halogenmethylketone der Formel II

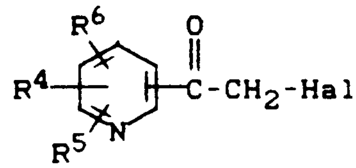

(II)

in welcher

$R^4$, $R^5$, $R^6$ und Hal die oben angegebene Bedeutung haben,

oxidiert.

Setzt man bei dem Verfahren als Halogenmethylketon der Formel II (2-Acetamido-5-pyridyl)-brommethylketon ein, läßt sich das Verfahren durch das folgende Schema darstellen:

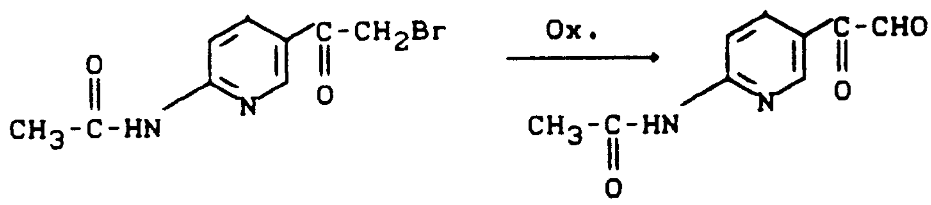


Als Halogenmethylketone der Formel II werden bevorzugt die weiter oben genannten Verbindungen eingesetzt.

Das Verfahren wird durchgeführt, indem man die Verbindung II, gegebenenfalls in Gegenwart eines Verdünnungsmittels, oxidiert.

Die Reaktion wird bei Temperaturen von +20°C bis +100°C durchgeführt.

Es wird bevorzugt bei Normaldruck gearbeitet.

Als Oxidationsmittel wird bevorzugt Dimethylsulfoxid verwendet (N. Kornblum et. al., JACS 79, 6562 (1957).

Wird die Umsetzung in Gegenwart eines Verdünnungsmittels durchgeführt, können alle inerten organischen Lösungsmittel verwendet werden. Hierzu gehören insbesondere aliphatische und aromatische, gegebenenfalls halogenierte Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril Benzonitril. Bevorzugt wird in Dimethylsulfoxid ohne ein weiteres Lösungsmittel gearbeitet.

28. Es wurden die neuen Verbindungen der Formel X gefunden, in welcher $R^3$, $R^4$, $R^5$, $R^6$ die bei den Verbindungen der Formel I angegebenen Bedeutungen besitzen und $R^5$ zusätzlich zu den angegebenen Resten für $NO_2$ stehen kann, wobei $R^4$, $R^5$, $R^5$ nicht gleichzeitig für Wasserstoff stehen dürfen.

29. Es wurde gefunden, daß man die neuen Verbindungen der Formel X erhält, indem man Verbindungen der Formel XXIII

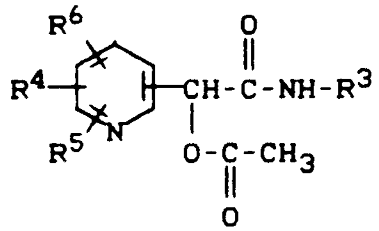

(XXIII)

in welcher

$R^3$-$R^6$ die oben angegebene Bedeutung haben,

hydrolysiert.

Setzt man bei dem Verfahren als Verbindung der Formel XXIII (2-Cyano-5-pyridyl)-acetoxyessigsäure-ethylamid ein, läßt sich das Verfahren durch das folgende Schema darstellen:

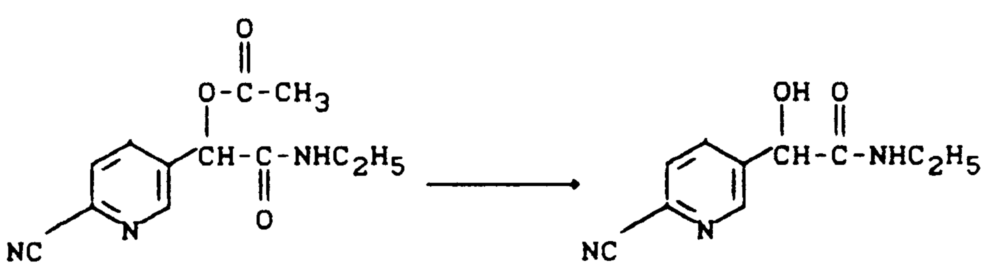

Die Substituenten $R^3$-$R^6$ in den Verbindungen XXIII besitzen bevorzugt die bei den Verbindungen der Formel I angegebenen bevorzugten Bedeutungen. Im einzelnen seien folgende Verbindungen der Formel XXIII genannt:

(2-Amino-3-chlor-5-pyridyl)acetoxyessigsäureisopropylamid,

(2-Amino-3-chlor-5-pyridyl)acetoxyessigsäure-tert.-butylamid,

(2-Amino-3-cyano-5-pyridyl)acetoxyessigsäureisopropylamid,
(2,4-Dichlor-3-amino-6-pyridyl)acetoxyessigsäure-tert.-butylamid,
(2-Chlor-3-amino-4-cyano-6-pyridyl)acetoxyessigsäureisopropylamid,
(2-Cyano-3-amino-4-chlor-6-pyridyl)acetoxyessigsäuretert.-butylamid.

Zur Abspaltung der Acetylgruppe werden anorganische Säuren verwendet. Hierzu zählen Halogenwasserstoffsäuren wie Salzsäure; Schwefelsäure, Phosphorsäure.

Das Verfahren wird durchgeführt, indem man die Verbindung XXIII in einem Verdünnungsmittel als Lösungsvermittler mit überschüssiger wäßriger Lösung der anorganischen Säure behandelt.

Die Reaktion wird bei Temperaturen von +20 °C bis +150 °C durchgeführt.

Es wird bevorzugt bei Normaldruck gearbeitet.

Als Verdünnungsmittel können alle inerten organischen Lösungsmittel, die mit Wasser mischbar sind, verwendet werden. Hierzu zählen Ether wie Tetrahydrofuran, Dioxan; Nitrile, wie Acetonitril; Amide wie Dimethylformamid; Alkohole wie Methanol, Ethanol; Dimethylsulfoxid.

30. Es wurden die neuen Verbindungen der Formel XXIII gefunden, in welcher $R^3$, $R^4$, $R^5$, $R^6$ die bei den Verbindungen der Formel I angegebenen Bedeutungen besitzen und $R^5$ zusätzlich zu den angegebenen Resten für $NO_2$ stehen kann, wobei $R^4$, $R^5$, $R^6$ nicht gleichzeitig für Wasserstoff stehen dürfen.

31. Es wurde gefunden, daß man die neuen Verbindungen der Formel XXIII erhält, indem man Aldehyde der Formel XII

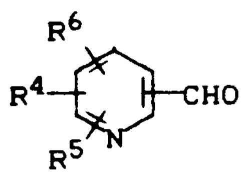

(XII)

in welcher

$R^4$, $R^5$, $R^6$ die oben angegebene Bedeutung haben,

mit Isonitrilen der Formel XXIV

$R^3$-NC (XXIV)

in welcher

$R^3$ die oben angegebene Bedeutung hat,

in Gegenwart von Essigsäure umsetzt.

Setzt man bei dem Verfahren als Aldehyd der Formel XII 2-Fluorpyridin-5-aldehydund als Isonitril der Formel XXIV Ethylisonitril ein, so läßt sich das Verfahren durch das folgende Schema darstellen:

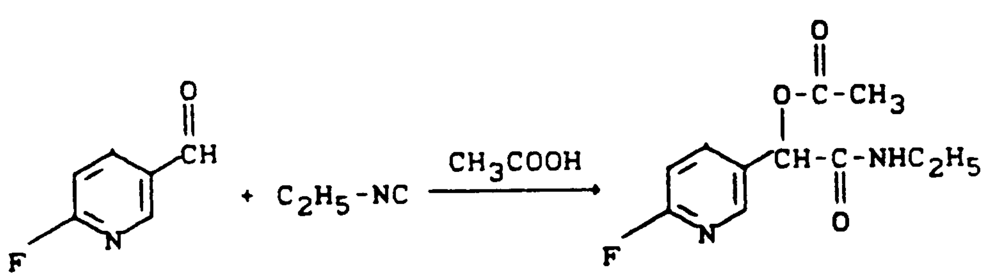


Bevorzugt werden die weiter oben genannten Aldehyde der Formel XII eingesetzt.

Isonitrile der Formel XXIV sind bekannt oder lassen sich analog zu bekannten Verfahren herstellen. Der Substituent $R^3$ besitzt bevorzugt die weiter oben bei den Verbindungen der Formel I angegebenen bevorzugten Bedeutungen. Im einzelnen seien folgende Verbindungen der Formel XXIV genannt; Methylisonitril, Ethylisonitril, n-Propylisonitril, Isopropylisonitril, n-Butylisonitril, sec.-Butylisonitril, Isobutylisonitril, tert.-Butylisonitril.

Das Verfahren wird durchgeführt, indem man die Verbindung XII mit der doppelt molaren Menge von

Isonitril der Formel XXIV und Essigsäure in einem Verdünnungsmittel zusammengibt.

Die Reaktion wird bei Temperaturen von +20°C bis +150°C durchgeführt.

Es wird bevorzugt bei Normaldruck gearbeitet.

Als Verdünnungsmittel dienen alle inerten organischen Lösungsmittel. Hierzu gehören insbesondere gegebenenfalls halogenierte aliphatische und aromatische Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril und Benzonitril.

32. Es wurden die neuen Verbindungen der Formel XI gefunden, in welcher $R^4$, $R^5$, $R^6$ die bei den Verbindungen der Formel I angegebenen Bedeutungen besitzen, und $R^5$ zusätzlich zu den angegebenen Resten für $NO_2$ stehen kann, wobei $R^4$, $R^5$, $R^6$ nicht gleichzeitig für Wasserstoff stehen dürfen.

33. Es wurde gefunden, daß man die neuen Verbindungen der Formel XI erhält, indem man Verbindungen der Formel XXV

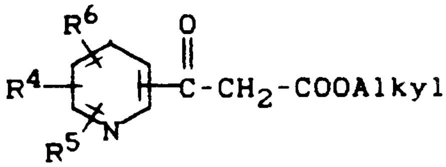

(XXV)

in welcher

$R^4$, $R^5$, $R^6$ die oben angegebene Bedeutung haben

und Alkyl für $C_1$-$C_4$-Alkyl steht, verseift und decarboxyliert.

Als Verbindungen der Formel XXV werden bevorzugt die unten genannten Verbindungen einsetzt.

Setzt man bei dem Verfahren als Verbindung der Formel XXV (2-Chlorpyridin-5-carbonyl)-essigsäureethylester ein, läßt sich das Verfahren durch das folgende Schema darstellen:

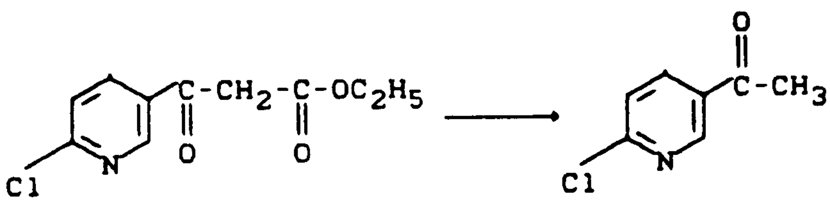

Das Verfahren wird durchgeführt, indem man die Verbindung der Formel XXV in einem Verdünnungsmittel in Gegenwart eines Überschusses einer Säure oder Base umsetzt.

Die Reaktion wird bei Temperaturen von +20°C bis +150°C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische gegebenenfalls halogenierte Kohlenwasserstoffe wie Pentan, Hexan, Cyclohexan, Benzol, Toluol, Methylenchlorid, Chloroform, Chlorbenzol; Ether wie Diethylether, Tetrahydrofuran, Ethylenglykolmonomethylether, Ethylenglykoldimethylether; Alkohole wie Methanol, Ethanol, sowie Wasser. Bevorzugt wird in Alkohol oder in Wasser gearbeitet.

Als Säuren können alle anorganischen Säuren verwendet werden. Hierzu zählen insbesondere Halogenwasserstoffsäuren wie Chlorwasserstoff, des weiteren Schwefelsäure, Phosphorsäure.

Als Basen können alle anorganischen Basen verwendet werden. Hierzu zählen Alkali- und Erdalkalimetallcarbonate wie Natrium- und Kaliumcarbonat sowie Hydroxide wie Natrium- und Kaliumhydroxid.

34. Es wurden die neuen Verbindungen der Formel XXV gefunden, in welcher $R^4$, $R^5$, $R^6$ die bei den Verbindungen der Formel I angegebenen Bedeutungen besitzen, und $R^5$ zusätzlich zu den angegebenen Resten für $NO_2$ stehen kann, wobei $R^4$, $R^5$, $R^6$ nicht gleichzeitig für Wasserstoff stehen dürfen.

35. Es wurde gefunden, daß man die neuen Verbindungen der Formel XXV erhält, indem man Verbindungen der Formel XV

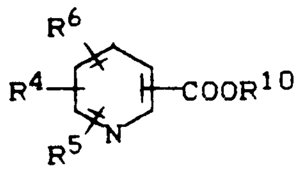

(XV)

in welcher

$R^4$, $R^5$, $R^6$, $R^{10}$ die oben angegebene Bedeutung haben,

mit Essigsäurederivaten der Formel XXVI

$CH_3COOAlkyl$ (XXVI)

in welcher Alkyl für $C_1$-$C_4$-Alkyl steht, umsetzt.

Setzt man bei dem Verfahren als Verbindung der Formel XV 2-Bromnicotinsäuremethylester und als Essigsäurederivat der Formel XXVI Essigsäuremethylester ein, läßt sich das Verfahren durch das folgende Schema darstellen:

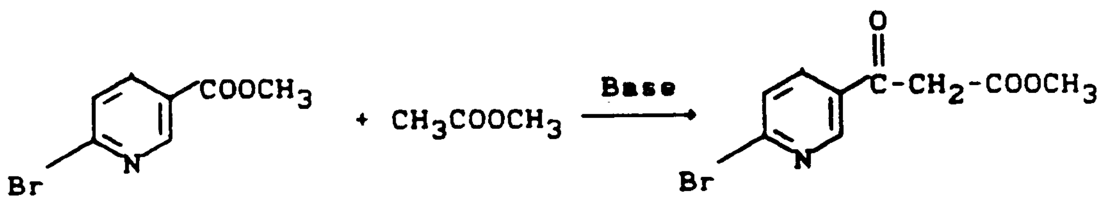


Als Verbindungen der Formel XV werden bevorzugt die weiter oben genannten Verbindungen eingesetzt.

Verbindungen der Formel XXVI sind bekannt oder lassen sich analog zu bekannten Verfahren herstellen.

Im einzelnen seien folgende Verbindungen der Formel XXVI genannt:
Essigsäuremethylester, Essigsäureethylester.

Das Verfahren wird durchgeführt, indem man äquivalente Mengen der Verbindungen XV, XXVI und einer Base in einem Verdünnungsmittel umsetzt.

Die Reaktion wird bei Temperaturen von 0°C bis +150°C durchgeführt.

Es wird bevorzugt bei Normaldruck gearbeitet.

Als Verdünnungsmittel dienen alle inerten organischen Lösungsmittel. Hierzu zählen insbesondere aliphatische und aromatische Kohlenwasserstoffe wie Pentan, Hexan, Heptan, Cyclohexan, Benzol, Toluol; Ether wie Diethylether, Tetrahydrofuran; Alkohole wie Methanol, Ethanol.

Als Basen dienen Alkali- und Erdalkalimetallhydride wie Natrium- und Calciumhydrid, Erdalkalimetallalkoholate wie Natrium- und Kaliummethylat, Natrium- und Kaliumethylat.

Die Wirkstoffe werden als Leistungsförderer bei Tieren zur Förderung und Beschleunigung des Wachstums, der Milch- und Wollproduktion, sowie zur Verbesserung der Futterverwertung, der Fleischqualität und zur Verschiebung des Fleisch-Fett-Verhältnisses zugunsten von Fleisch eingesetzt. Die Wirkstoffe werden bei Nutz-, Zucht-, Zier- und Hobbytieren verwendet.

Zu den Nutz- und Zuchttieren zählen Säugetiere wie z.B. Rinder, Schweine, Pferde, Schafe, Ziegen, Kaninchen, Hasen, Damwild, Pelztiere wie Nerze, Chinchilla, Geflügel wie z.B. Hühner, Gänse, Enten, Truthühner, Fische, wie z.B. Karpfen, Forellen, Lachse, Aale, Schleien, Hechte, Reptilien wie z.B. Schlangen und Krokodile.

Zu den Zier- und Hobbytieren zählen Säugetiere wie Hunde und Katzen, Vögel wie Papageien, Kanarienvögel, Fische wie Zier- und Aquarienfische z.B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge

des Wirkstoffs sowie die passende Dauer der Verabreichung hängen insbesondere von der Art, dem Alter, dem Geschlecht, dem Gesundsheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verarbreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstumsphase tamporär oder kontinuierlich verabreicht werden. Bei kontinuierlicher Verabreichung kann die Anwendung ein- oder mehrmals täglich in regelmäßigen oder unregelmäßigen Abständen erfolgen.

Die Verabreichung erfolgt oral oder parenteral in dafür geeigneten Formulierungen oder in reiner Form. Orale Formulierungen sind Pulver, Tabletten, Granulate, Drenche, Boli sowie Futtermittel, Prämixe für Futtermittel, Formulierungen zur Verabreichung über Trinkwasser.

Die oralen Formulierungen enthalten den Wirkstoff in Konzentrationen von 0,01 ppm - 100 %, bevorzugt von 0,01 ppm - 1 %.

Parenterale Formulierungen sind Injektionen in Form von Lösungen, Emulsionen und Suspensionen, sowie Implantate.

Die Wirkstoffe können in den Formulierungen allein oder in Mischung mit anderen Wirkstoffen, Mineralsalzen, Spurenelementen, Vitaminen, Eiweißstoffen, Farbstoffen, Fetten oder Geschmacksstoffen vorliegen.

Die Konzentration der Wirkstoffe im Futter beträgt normalerweise etwa 0,01-500 ppm, bevorzugt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das erfindungsgemäßen Wirkstoff enthält:
200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Prämix ergeben nach sorgfältigem Mischen 1 kg Futter.

In einem kg Futtermischung sind enthalten:
600 I.E. Vitamin A, 100 I.E. Vitamin, $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4$ x $H_2O$, 140 mg Zn $SO_4$ x 7 $H_2O$, 100 mg Fe $SO_4$ x 7 $H_2O$ und 20 mg $CuSO_4$ x 5 $H_2O$.

2,5 g Wirkstoff-Prämix enthalten z.B. 10 mg Wirkstoff, 1 g DL-Methionin, Rest Sojabohnenmehl.

Beispiel für die Zusammensetzung eines Schweineaufzuchtfutters, das erfindungsgemäßen Wirkstoff enthält:
630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z.B. wie beim Kükenfutter) ergeben nach sorgfältigem Mischen 1 kg Futter.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere, wie Schafe und Rinder, verwendet werden.

## Beispiel A

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Während des 13-tägigen Versuchs werden Gewichtszunahme und Futterverbrauch bestimmt.

Es werden die aus der Tabelle ersichtlichen Ergebnisse erhalten:

**Tabelle:** Ratten-Fütterungsversuch

| Wirkstoff | Dosis 100 ppm | Gewichtszunahme |
|---|---|---|
| Kontrolle, ohne Wirkstoff | | 100 |
| 1 | | 110 |
| 3 | | 115 |
| 6 | | 110 |
| 7 | | 120 |

## Beispiele

Allgemeine Vorschrift für Verfahren 3a

Herstellung der Verbindungen der Formel I nach Verfahren 3a

10 mmol der Verbindung der Formel II werden bei 0 °C portionsweise zu einer Lösung von 10 ml des Amins der Formel III in 15 ml absolutem Ethanol gegeben. Man läßt auf 10-15 °C kommen und rührt bei dieser Temperatur eine Stunde nach. Dann wird wieder auf 0 °C abgekühlt und portionsweise werden 600 mg (50 mmol) Natriumborhydrid zugegeben. Man rührt über Nacht bei Raumtemperatur nach. Nach Zugabe von 20 ml Wasser wird 30 min gerührt, eingedampft und zwischen Wasser und Essigester verteilt. Die organische Phase wird über Natriumsulfat getrocknet und eingedampft. Der Rückstand wird umkristallisiert.

Allgemeine Vorschrift für Verfahren 3b

Herstellung der Verbindungen der Formel I nach Verfahren 3b

0,1 mol der Verbindung der Formel IV und 0,11 mol des Amins der Formel III werden in 200 ml Methanol über Nacht unter Rückfluß erhitzt. Das Lösungsmittel und überschüssiges Amin werden abgezogen und der Rückstand umkristallisiert.

Allgemeine Vorschrift für Verfahren 3c

Herstellung der Verbindungen der Formel I nach Verfahren 3c

10 mmol der Verbindung der Formel V werden in 150 ml Ethanol gelöst und 20 ml des Amins der Formel III werden zugegeben, die Mischung wird 18 Stunden unter Rückfluß erhitzt. Dann werden Lösungsmittel und überschüssiges Amin abgezogen, und der Rückstand wird in 100 ml trockenem Ether aufgenommen. Das unlösliche Aminhydrohalogenid wird abfiltriert, die etherische Lösung mit Wasser gewaschen, über Natriumsulfat getrocknet und eingedampft. Das Rohprodukt wird umkristallisiert.

Allgemeine Vorschrift für Verfahren 3d

Herstellung der Verbindungen der Formel I nach Verfahren 3d

Zu 22 mmol der Verbindung VI in 10 ml absolutem Ethanol werden 22 mmol der Carbonylverbindung der Formel VII bei 0-5 °C zugegeben. Man läßt auf Raumtemperatur kommen und rührt noch 30 Minuten nach. Die Lösung wird dann zu 0,15 g Adams-Katalysator (vorhydriert in 10 ml absolutem Ethanol) gegeben

und die Mischung 4-5 Stunden bei 40 °C und 50 atm Wasserstoffdruck hydriert. Nach Abfiltrieren vom Katalysator wird eingedampft und umkristallisiert.

Allgemeine Vorschrift für Verfahren 3e

Herstellung der Verbindungen der Formel I nach Verfahren 3e

Zur Lösung von 10 mmol der Verbindung der Formel VIII in 50 ml Ethanol werden bei 10-15 °C 15 mmol des Amins der Formel IX getropft. Man läßt auf Raumtemperatur kommen und rührt noch 15 min nach. Dann verdünnt man mit weiteren 100 ml Ethanol und gibt bei 0-5 °C portionsweise 80 mmol Natriumborhydrid zu. Man läßt auf Raumtemperatur kommen und rührt über Nacht. Dann gibt man bei 10 °C 200 ml Wasser zu, rührt 30 min., dampft das Ethanol ab und extrahiert den Rückstand dreimal mit je 50 ml Dichlormethan. Die vereinigten organischen Phasen werden mit 100 ml Wasser gewaschen, über Natriumsulfat getrocknet und eingedampft.

Allgemeine Vorschrift für Verfahren 3f

Herstellung der Verbindungen der Formel I nach Verfahren 3f

Zu 12,4 ml einer 1 M Lösung von Boran in Tetrahydrofuran werden 2,3 mmol der Verbindung X in 30 ml absolutem Tetrahydrofuran getropft. Die Mischung wird eine 1 Stunde unter Rückfluß erhitzt, mit Eiswasser verdünnt und mit 50 ml 1N Salzsäure versetzt. Nach Abdampfen des organischen Lösungsmittels wird die saure wäßrige Lösung zweimal mit je 30 ml Ether extrahiert, dann mit gesättigter Natriumcarbonatlösung alkalisch gestellt und dreimal mit je 30 ml Essigester extrahiert. Die vereinigten Extrakte werden über Nariumsulfat getrocknet und eingedampft.

Analog zu den oben angegebenen Verfahren 3a - 3f werden die folgenden Verbindungen hergestellt:

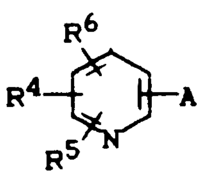

| Bsp. Nr. | A | $R^5$ | $R^4$ | $R^6$ | Physik. Daten Fp (°C) |
|---|---|---|---|---|---|
| 1. | $4-CH(OH)CH_2NHC_4H_9-t$ | H | 2-Cl | 6-Cl | 122° |
| 2. | $3-CH(OH)CH_2NHC_4H_9-t$ | $6-NH_2$ | H | H | 115 |
| 3. | $3-CH(OH)CH_2NHC_4H_9-t$ | $6-NH_2$ | 5-Cl | H | 140 |
| 4. | $2-CH(OH)CH_2NHC_4H_9-t$ | $5-NH_2$ | H | H | 115 |
| 5. | $2-CH(OH)CH_2NHC_4H_9-t$ | $5-NH_2$ | 6-Cl | 4-Cl | 124 |
| 6. | $2-CH(OH)CH_2NHC_4H_9-t$ | $5-NH_2$ | 6-Cl | H | 111 |
| 7. | $2-CH(OH)CH_2NHC_3H_7-i$ | $5-NH_2$ | H | H | 88 |
| 8. | $3-CH(OH)CH_2NHC_3H_7-i$ | $6-NH_2$ | 5-Cl | H | 146 |

Herstellung der Verbindungen der Formel XII nach Verfahren 9a

2,6-Dichlorpyridin-4-aldehyd

Unter Feuchtigkeitsausschluß und unter Stickstoff wird zu einer Lösung von 1 ml (11 mmol) Oxalylchlorid in 25 ml absolutem Methylenchlorid bei -50 bis -60 °C eine Lösung von 1,7 ml (22 mmol) absolutem

DMSO in 5 ml absolutem Methylenchlorid getropft. Man rührt 2 min nach, dann werden 1,78 g (10 mmol) (2,6-Dichlor-4-pyridyl)-methanol in 10 ml absolutem Methylenchlorid innerhalb von 5 min zugegeben. Man rührt 15 min nach, tropft 7 ml Triethylamin zu und läßt auf Raumtemperatur kommen. Dann gibt man 50 ml Wasser zu, trennt die organische Phase ab und extrahiert die wäßrige Phase nochmal mit 50 ml Methylenchlorid. Die vereinigten organischen Phasen werden nacheinander mit gesättigter NaCl-Lösung, 1 %iger Salzsäure, Wasser, 5 %iger $Na_2CO_3$-Lösung und Wasser gewaschen, über $Na_2SO_4$ getrocknet und eingedampft.
Ausbeute 1,6 g (91 %), Fp. 45° C.

Analog werden hergestellt:
2-Amino-3-chlorpyridin-5-aldehyd, Fp. 135° C
3-Amino-2,4-dichlorpyridin-6-aldehyd, Fp. 139-141° C

Herstellung der Verbindungen der Formel XIII nach Verfahren 11

<u>(2-Amino-5-pyridyl)methanol</u>

Zur Suspension von 6,1 g (45 mmol) 2-Aminopyridin-5-carbonsäure in 75 ml absolutem Tetrahydrofuran werden bei 0° C 180 ml 1 M Boran in Tetrahydrofuran (= 180 mmol) zugetropft. Man läßt auf Raumtemperatur kommen und rührt 4 Stunden nach. Dann kühlt man auf 0° C, gibt 50 ml 3N NaOH zu und rührt über Nacht bei Raumtemperatur.

Durch Zugabe von festem NaOH wird auf pH 11 gestellt, das Tetrahydrofuran abgezogen und der Rückstand mit Ether extrahiert. Die Etherphase wird mit Wasser gewaschen, über $Na_2SO_4$ getrocknet und eingedampft.
Ausbeute: 4,5 g (80 %); Fp. 101° C (Z.)

Analog werden hergestellt:
(2-Amino-3-chlor-5-pyridyl)methanol, Fp. 102° C
(3-Amino-2,4-dichlor-6-pyridyl)methanol, Fp. 95° C

Herstellung der Verbindungen der Formel XIII nach Verfahren 11

<u>(2,6-Dichlor-4-pyridyl)methanol</u>

Zu einer Suspension von 860 mg (23 mmol) $LiAlH_4$ und 3,03 g (23 mmol) $AlCl_3$ in 100 ml absolutem Ether werden bei 0° C unter Stickstoff 5 g (23 mmol) 2,6-Dichlorpyridin-4-carbonsäureethylester in kleinen Portionen gegeben. Dann wird 3 Stunden unter Rückfluß erhitzt, nach dem Abkühlen mit Wasser verdünnt und die organische Phase abgetrennt. Die Etherphase wird mit Wasser gewaschen, über $Na_2SO_4$ getrocknet und eingedampft.
Ausb.: 3,2 g (78 %), Fp. 131° C.

Herstellung der Verbindungen der Formel XVI nach Verfahren 13

<u>2-Acetamido-pyridin-5-carbonsäure</u>

Zu 15 g (0,1 mol) 2-Acetamido-5-methylpyridin in 250 ml Wasser werden bei 70-90° C portionsweise 42 g (0,265 mol) Kaliumpermanganat gegeben. Nach beendeter Zugabe wird heiß filtriert, auf etwa 150 ml eingeengt und mit Salzsäure auf pH 3 gestellt. Das ausgefallene Produkt wird abgesaugt und an der Luft getrocknet.
Ausb.: 12,6 g (70 %); Fp. > 230° C.

Analog werden hergestellt:
2-Amino-3-chlorpyridin-5-carbonsäure, Fp. >300° C
3-Amino-2,4-dichlorpyridin-6-carbonsäure, Fp. 134° C

Herstellung der Verbindungen der Formel XIV nach Verfahren 15

<u>2,6-Dichlorpyridin-4-carbonsäurechlorid</u>

10 g (52 mmol) 2,6-Dichlorpyridin-4-carbonsäure werden in 30 ml Thionylchlorid 4 Stunden unter Rückfluß erhitzt. Die jetzt homogene Lösung wird eingedampft und der Rückstand destilliert.

Ausb.: 8,2 g (75 %); Sdp 173° C (13 mm Hg).

Herstellung der Verbindungen der Formel XV nach Verfahren 17a

2,6-Dichlorpyridin-4-carbonsäureethylester

5,5 g (26 mmol) 2,6-Dichlorpyridin-4-carbonsäurechlorid werden in 100 ml absolutem Ethanol gelöst und mit 2,63 g (26 mmol) Triethylamin versetzt. Die Lösung wird 30 min unter Rückfluß erhitzt und eingedampft. Der Rückstand wird in 100 ml Methylenchlorid aufgenommen, mit gesättigter $NaHCO_3$-Lösung und Wasser gewaschen, über $Na_2SO_4$ getrocknet und eingedampft.
Ausb.: 5,35 g (93 %); Fp. 64° C.

Herstellung der Verbindungen der Formel XV nach Verfahren 17b

2,6-Dichlorpyridin-4-carbonsäureethylester

57,6 g (0,3 mmol) 2,6-Dichlorpyridin-4-carbonsäure werden in 200 ml Thionylchlorid 4 Stunden unter Rückfluß erhitzt. Dann wird eingedampft und der Rückstand zweimal in jeweils 100 ml Toluol aufgenommen und wieder eingedampft, um restliches Thionylchlorid zu entfernen. Das rohe Säurechlorid wird in 300 ml absolutem Ethanol aufgenommen, mit 30 g (0,3 mol) Triethylamin versetzt und 30 min unter Rückfluß erhitzt. Nach dem Eindampfen wird in 200 ml Methylenchlorid aufgenommen, über $Na_2SO_4$ getrocknet und eingedampft.
Ausb.: 39,6 g (60 %); Fp. 64° C.

Herstellung der Verbindungen der Formel X nach Verfahren 29

(2,6-Dichlor-4-pyridyl)-hydroxyessigsäure-tert.-butylamid

Eine Suspension von 2,7 g (8,5 mmol) (2,6-Dichlor-4-pyridyl)-acetoxyessigsäure-tert.-butylamid wird in einer Mischung aus 31 ml Methanol, 31 ml Wasser und 15,4 ml 2,5 N Salzsäure zum Sieden erhitzt. Nach 1 Stunde wird das Methanol abgezogen und der ausgefallene Feststoff abgesaugt.
Ausb.: 1,9 g (81 %); Fp. 168° C

Analog werden hergestellt:
(2-Amino-5-pyridyl)-hydroxyessigsäure-tert.-butylamid, Fp. 104° C
(5-Amino-2-pyridyl)-hydroxyessigsäure-tert.-butylamid, Fp. 158° C
(2-Amino-3-chlor-5-pyridyl)-hydroxyessigsäure-tert.-butylamid, Fp. 79° C
(2-Chlor-3-amino-6-pyridyl)-hydroxyessigsäure-tert.-butylamid, Fp. 136° C
(3-Amino-2,4-dichlor-6-pyridyl)-hydroxyessigsäure-tert.-butylamid, Fp. 176° C

Herstellung der Verbindungen der Formel XXIII nach Verfahren 31

(2,6-Dichlor-4-pyridyl)-acetoxyessigsäure-tert.-butylamid

Eine Mischung von 1,76 g (10 mmol) 2,6-Dichlorpyridin-4-aldehyd, 1,66 g (20 mmol) tert.-Butylisonitril und 1,2 g (20 mmol) Eisessig in 50 ml trockenem Chloroform wird 2 Stunden unter Rückfluß erhitzt. Dann wird mit 5 %iger wäßriger $NaHCO_3$-Lösung gewaschen, über $Na_2SO_4$ getrocknet und eingedampft.
Ausb.: 2,7 g (85 %); Fp. 156° C

**Patentansprüche**

**Patentansprüche für folgende Vertragsstaaten : AT, BE, CH, DE, FR, GB, IT, LI, NL, SE**

**1.** Verwendung von Heteroarylethylaminen der Formel (I)

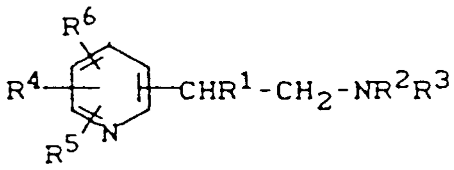

(I)

in welcher

| | |
|---|---|
| $R^1$ | für OH, Acyloxy oder Alkoxy steht |
| $R^2$ | für Wasserstoff oder Alkyl steht, |
| $R^3$ | für Wasserstoff, Alkyl, Cycloalkyl, substituiertes Alkyl, gegebenenfalls substituiertes Aralkyl, Aryl oder Heterocyclyl steht, |
| $R^2$ und $R^3$ | können gemeinsam mit dem Stickstoffatom, an das sie gebunden sind, für einen gegebenenfalls substituierten heterocyclischen Rest stehen, |
| $R^4$ | für Wasserstoff, Alkyl, Halogen, Halogenalkyl, Hydroxyl, Cyano, Alkoxycarbonyl, Aminocarbonyl, Mono- und Dialkylaminocarbonyl, Alkoxy, Halogenalkoxy, Halogenalkylthio, $NHSO_2$-Alkyl steht, |
| $R^5$ | für Wasserstoff, Hydroxyl, Alkoxy oder den Rest $-NR^7R^8$ steht, |
| $R^6$ | für die bei $R^4$ angegebenen Reste steht, |
| $R^7$ | für Wasserstoff oder Alkyl steht, |
| $R^8$ | für Wasserstoff, Alkyl, Halogenalkyl, Acyl steht, |

ausgenommen die Verbindung 6-Amino-α-(((1-methyl-3-phenylpropyl)amino)methyl)-3-pyridinmethanol,

(Die Verbindungen der Formel I können dabei in Form ihrer Tautomeren sowie in Form ihrer Racemate oder Enantiomeren vorliegen) zur Leistungsförderung bei Tieren.

2. Verwendung von Heteroarylethylaminen der Formel I gemäß Anspruch 1 zur Förderung und Beschleunigung des Wachstums, zur Verbesserung der Futterverwertung, der Fleischqualität und zur Verschiebung des Fleisch-Fett-Verhältnisses zugunsten von Fleisch bei Nutz-, Zucht-, Zier- und Hobbytieren.

3. Verwendung gemäß Ansprüchen 1 und 2 von Heteroarylethylaminen der Formel I gemäß Anspruch 1, in welcher

| | |
|---|---|
| $R^1$ | für OH, $C_{1-6}$-Alkoxy oder Acyloxy steht und Acyloxy für Oxycarbonyl-$C_{1-6}$-alkyl, gegebenenfalls substituiertes Oxycarbonylphenyl, Oxysulfonyl-$C_{1-6}$-alkyl, gegebenenfalls substituiertes Oxysulfonylphenyl steht, |
| $R^2$ | für Wasserstoff oder $C_{1-6}$-Alkyl steht, |
| $R^3$ | für Wasserstoff, gegebenenfalls substituiertes $C_{1-6}$-Alkyl, $C_{3-6}$-Cycloalkyl, $C_{1-6}$-Alkylphenyl, Phenyl steht, |
| $R^2$ und $R^3$ | können gemeinsam mit dem Stickstoffatom, an das sie gebunden sind, für einen gegebenenfalls substituierten 5- bis 6-gliedrigen gesättigten oder ungesättigten heterocyclischen Rest stehen, der gegebenenfalls noch weitere Heteroatome aus der Reihe N, O, S enthalten kann, |
| $R^4$ | für Wasserstoff, $C_{1-4}$-Alkyl, Fluor, Chlor, Brom, $C_{1-4}$-Halogenalkyl, Hydroxyl, Cyano, $C_{1-4}$-Alkoxycarbonyl, Mono- oder -di-$C_{1-4}$-alkylaminocarbonyl, $C_{1-6}$-Alkoxy, $C_{1-6}$-Halogenalkoxy, $C_{1-6}$-Halogenalkylthio, $-NHSO_2$-$C_{1-6}$-Alkyl steht, |
| $R^5$ | für Wasserstoff, Hydroxyl, $C_{1-6}$-Alkoxy oder den Rest $-NR^7R^8$ steht, |
| $R^6$ | für die bei $R^4$ angegebenen Reste steht, |
| $R^7$ | für Wasserstoff oder $C_{1-6}$-Alkyl steht, |
| $R^8$ | für Wasserstoff, $C_{1-6}$-Alkyl, $C_{1-4}$-Halogenalkyl, $C_{1-6}$-Alkylcarbonyl, gegebenenfalls substituiertes Benzoyl, $C_{1-6}$-Alkylsulfonyl, gegebenenfalls substituiertes Phenylsulfonyl steht. |

Als Substituenten der gegebenenfalls substituierten Reste kommen bevorzugt infrage: Cyano, Halogen wie Fluor oder Chlor, Hydroxy, $C_{1-4}$-Alkyl, $C_{1-4}$-Halogenalkyl,Phenyl, $C_{1-4}$-Alkoxy, $C_{1-4}$-Halogenalkoxy, $C_{1-4}$-Alkylthio, $C_{1-4}$-Halogenalkylthio, für den Fall, daß die Substituenten an einem Phenylrest sitzen, zusätzlich bevorzugt Methylendioxy, Ethylendioxy, halogensubstituiertes Methylendioxy, halogensubstituiertes Ethylendioxy, ferner Phenyl, Phenoxy, die ihrerseits einen oder mehrere der obengenannten Substituenten tragen können,

ausgenommen die Verbindung 6-Amino-α-(((1-methyl-3-phenylpropyl)amino)methyl)-3-pyridinmethanol.

4. Verwendung gemäß Ansprüchen 1 und 2 von Heteroarylethylaminen der Formel I gemäß Anspruch 1,

in welcher

$R^1$ für OH, $C_{1-6}$-Alkoxy, insbesondere Methoxy oder Ethoxy steht,

$R^2$ für Wasserstoff oder $C_{1-4}$-Alkyl, insbesondere Methyl oder Ethyl steht,

$R^3$ für Wasserstoff, gegebenenfalls durch 1-5 Halogenatome substituiertes $C_{1-6}$-Alkyl, insbesondere Methyl, Ethyl, Propyl, Butyl, Pentyl, Hexyl, wobei insbesondere sekundäre und tertiäre Alkylreste genannt seien, sowie $C_{1-6}$-Alkylphenyl, das gegebenenfalls durch Halogen, insbesondere Fluor oder Chlor, $C_{1-4}$-Alkyl, Hydroxy, $C_{1-4}$-Alkoxy, gegebenenfalls halogensubstituiertes Methylendioxy oder Ethylendioxy substituiert sein kann, Phenyl, das gegebenenfalls durch Halogen, insbesondere Fluor oder Chlor, $C_{1-4}$-Alkyl, Hydroxy, $C_{1-4}$-Alkoxy, gegebenenfalls halogensubstituiertes Methylendioxy oder Ethylendioxy substituiert sein kann, steht,

$R^4$ für Wasserstoff, $C_{1-4}$-Alkyl, Halogen, insbesondere Fluor, Chlor, Brom, Cyano, Hydroxy, $C_{1-4}$-Halogenalkyl mit 1 bis 5 Halogenatomen, $C_{1-4}$-Alkoxy, $C_{1-4}$-Halogenalkoxy mit 1 bis 5 Halogenatomen, $NHSO_2$-$C_{1-4}$-Alkyl, -COO-$C_{1-4}$-Alkyl, -$CONH_2$, -CONH-$C_{1-4}$-Alkyl, -CON-($C_{1-4}$-Alkyl)$_2$ steht,

$R^5$ für Wasserstoff, OH, $C_{1-4}$-Alkoxy, -$NR^7R^8$ steht,

$R^6$ für die bei $R^4$ angegebenen Reste steht,

$R^7$ für Wasserstoff, $C_{1-4}$-Alkyl steht,

$R^8$ für Wasserstoff, $C_{1-4}$-Alkyl steht,

ausgenommen die Verbindung 6-Amino-α-(((1-methyl-3-phenylpropyl)amino)methyl)-3-pyridinmethanol.

5. Verwendung gemäß Ansprüchen 1 und 2 des Heteroarylethylamins der Formel

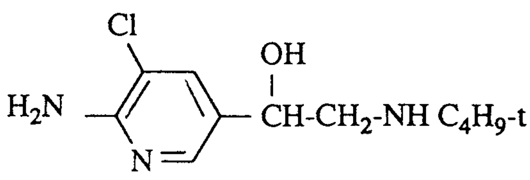

6. Verwendung gemäß Ansprüchen 1 und 2 des Heteroarylethylamins der Formel

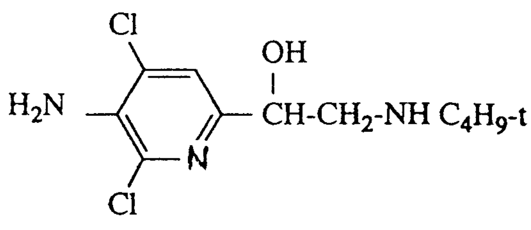

7. Tiernahrung und Premixe zur Herstellung von Tiernahrung enthaltend Heteroarylethylamine der allgemeinen Formel (I) gemäß Ansprüchen 1 - 6.

8. Leistungsfördernde Mittel für Tiere, enthaltend Heteroarylethylamine der allgemeinen Formel (I) gemäß Ansprüchen 1 - 6.

9. Verfahren zur Herstellung von leistungsfördernden Mitteln für Tiere, dadurch gekennzeichnet, daß man Heteroarylethylamine der allgemeinen Formel (I) gemäß Ansprüchen 1 - 6 mit Streck-, Verdünnungs- und Nahrungsmitteln sowie gegebenenfalls weiteren Hilfsstoffen mischt.

**10.** Verwendung von Heteroarylethylaminen der Formel (I) gemäß Ansprüchen 1 - 6 zur Herstellung von leistungsfördernden Mitteln.

**11.** Verwendung von Heteroarylethylaminen der Formel (I) gemäß Ansprüchen 1 - 6 zur Herstellung von Mitteln zur Förderung und Beschleunigung des Wachstums, zur Verbesserung der Futterverwertung, der Fleischqualität und zur Verschiebung des Fleisch-Fett-Verhältnisses zugunsten von Fleisch bei Nutz-, Zucht-, Zier- und Hobbytieren.

**12.** Heteroarylethylamine der Formel I,

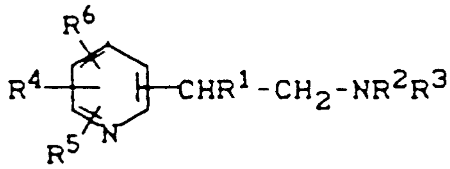

(I)

in welcher

$R^1$ für OH, $C_{1-6}$-Alkoxy oder für Oxycarbonyl-$C_{1-6}$-alkyl, gegebenenfalls substituiertes Oxycarbonylphenyl,Oxysulfonyl-$C_{1-6}$-alkyl, gegebenenfalls substituiertes Oxysulfonylphenyl steht,

$R^2$ für Wasserstoff oder $C_{1-6}$-Alkyl steht,

$R^3$ für Wasserstoff, gegebenenfalls substituiertes $C_{1-6}$-Alkyl, $C_{3-6}$-Cycloalkyl, substituiertes $C_{1-6}$-Alkylphenyl oder Phenyl steht,

$R^4$ für Wasserstoff, $C_{1-4}$-Alkyl, Fluor, Chlor, Brom, $C_{1-4}$-Halogenalkyl, Cyano, $C_{1-4}$-Alkoxycarbonyl, Mono- oder -di-$C_{1-4}$-alkylaminocarbonyl, $C_{1-6}$-Alkoxy, $C_{1-6}$-Halogenalkoxy, $C_{1-6}$-Halogenalkylthio, -$NHSO_2$-$C_{1-6}$-Alkyl steht,

$R^5$ für Wasserstoff, $C_{1-6}$-Alkoxy oder den Rest -$NR^7R^8$ steht,

$R^6$ für Wasserstoff, $C_{1-4}$-Alkyl, Fluor, Chlor, Brom, $C_{1-4}$-Halogenalkyl, Cyano, $C_{1-4}$-Alkoxycarbonyl, $C_{1-6}$-Alkoxy, $C_{1-6}$-Halogenalkoxy, $C_{1-6}$-Halogenalkylthio, $NHSO_2$-Alkyl steht,

unter der Voraussetzung, daß $R^4$, $R^5$, $R^6$ nicht gleichzeitig für Wasserstoff stehen,

$R^7$ für Wasserstoff oder $C_{1-6}$-Alkyl steht,

$R^8$ für Wasserstoff, $C_{1-6}$-Alkyl, $C_{1-6}$-Halogenalkyl oder Acyl steht.

Wobei als Substituenten der gegebenenfalls substituierten Reste genannt seien: Cyano, Halogen wie Fluor oder Chlor, Hydroxy, $C_{1-4}$-Alkyl, $C_{1-4}$-Halogenalkyl,Phenyl, $C_{1-4}$-Alkoxy, $C_{1-4}$-Halogenalkoxy, $C_{1-4}$-Alkylthio, $C_{1-4}$-Halogenalkylthio, für den Fall, daß die Substituenten an einem Phenylrest sitzen, zusätzlich Methylendioxy, Ethylendioxy, halogensubstituiertes Methylendioxy, halogensubstituiertes Ethylendioxy, ferner Phenyl, Phenoxy, die ihrerseits einen oder mehrere der obengenannten Substituenten tragen können,

sowie ihre physiologisch verträglichen Salze und ihre N-Oxide,

ausgenommen 6-Chlor-α(isopropylaminomethyl)-3-pyridinmethanol und 2-Chlor-α-(isopropylaminomethyl)-4-pyridinmethanol.

**13.** Heteroarylethylamine der Formel I gemäß Anspruch 12

in welcher

$R^1$ für OH, $C_{1-6}$-Alkoxy steht,

$R^2$ für Wasserstoff oder $C_{1-4}$-Alkyl steht,

$R^3$ für Wasserstoff, gegebenenfalls durch 1 bis 5 Halogenatome substituiertes $C_{1-6}$-Alkyl sowie $C_{1-6}$-Alkylphenyl, das durch Halogen, $C_{1-4}$-Alkyl, Hydroxy, $C_{1-4}$-Alkoxy, gegebenenfalls halogensubstituiertes Methylendioxy oder Ethylendioxy substituiert ist, steht,

$R^4$ für Wasserstoff, $C_{1-4}$-Alkyl, Halogen, Cyano, $C_{1-4}$-Halogenalkyl mit 1 bis 5 Halogenatomen, $C_{1-4}$-Alkoxy, $C_{1-4}$-Halogenalkoxy mit 1 bis 5 Halogenatomen, $NHSO_2$-$C_{1-4}$-Alkyl, -COO-$C_{1-4}$-Alkyl, -$CONH_2$, -CONH-$C_{1-4}$-Alkyl, -CON($C_{1-4}$-Alkyl)$_2$ steht,

$R^5$ für Wasserstoff, $C_{1-4}$-Alkoxy, -$NR^7R^8$ steht,

$R^6$ für die bei $R^4$ angegebenen Reste steht,

$R^7$ für Wasserstoff, $C_{1-4}$-Alkyl steht,

$R^8$ für Wasserstoff, $C_{1-4}$-Alkyl steht,
sowie ihre physiologisch verträglichen Salze und ihre N-Oxide,

ausgenommen 6-Chlor-α(isopropylaminomethyl)-3-pyridinmethanol und 2-Chlor-α-(isopropylaminomethyl)-4-pyridinmethanol.

**14.** Heteroarylethylamin der Formel

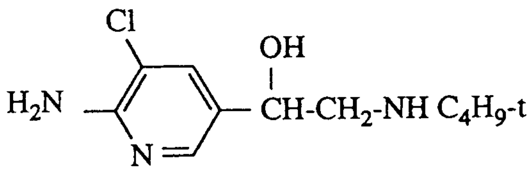

**15.** Heteroarylethylamin der Formel

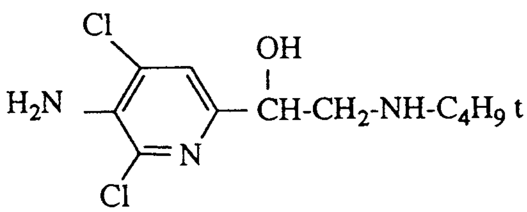

**16.** Verfahren zur Herstellung der Heteroarylethylamine der Formel I, gemäß Anspruch 12

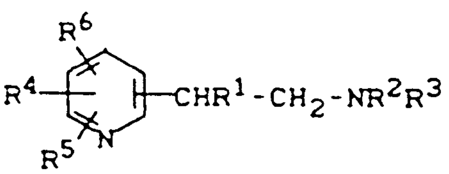

(I)

in welcher
$R^1$, $R^2$, $R^3$, $R^4$, $R^5$, $R^6$ die in Anspruch 12 angegebene Bedeutung besitzen.
dadurch gekennzeichnet, daß man
a) Halogenmethylketone der Formel (II)

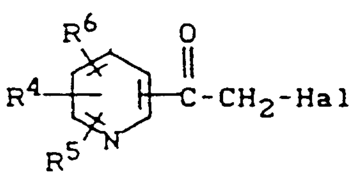

(II)

in welcher
$R^4$, $R^5$, $R^6$ die in Anspruch 12 angegebene Bedeutung haben und Hal für Halogen steht,
mit Aminen der Formel (III)

$HNR^2R^3$ (III)

in welcher

$R^2$ und $R^3$ die in Anspruch 12 angegebene Bedeutung haben,

umsetzt und anschließend die Carbonylgruppe reduziert, oder
b) Epoxide der Formel (IV)

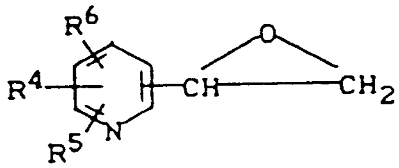

(IV)

in welcher
$R^4$, $R^5$, $R^6$ die oben angegebene Bedeutung haben
mit Aminen der Formel (III)

$HNR^2R^3$ (III)

in welcher
$R^2$ und $R^3$ die oben angegebene Bedeutung haben,
umsetzt oder
c) beta-Halogenethylverbindungen der Formel (V)

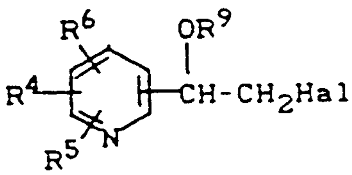

(V)

in welcher
$R^4$, $R^5$, $R^6$ die oben angegebene Bedeutung haben,
Hal für Halogen steht,
$R^9$ für Wasserstoff steht,
mit Aminen der Formel (III)

$HNR^2R^3$ (III)

in welcher
$R^2$ und $R^3$ die oben angegebene Bedeutung haben
umsetzt oder
d) für den Fall, daß in Formel I $R^2$ für Wasserstoff steht und $R^3$ für substituiertes Alkyl oder Aralkyl steht, man Verbindungen der Formel (VI)

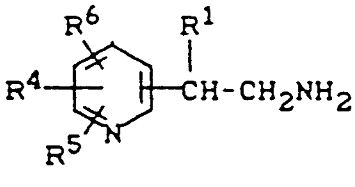

(VI)

in welcher
$R^1$, $R^4$, $R^5$, $R^6$ die die in Anspruch 12 angegebene Bedeutun haben
mit Verbindungen der Formel (VII)

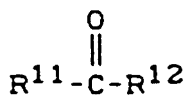 (VII)

in welcher

$R^{11}$ für substituiertes Alkyl, Aryl oder Aralkyl steht,

$R^{12}$ für Wasserstoff oder $C_{1-4}$-Alkyl steht,

unter reduzierenden Bedingungen umsetzt, oder

e) für den Fall, daß in Formel I $R^2$ für Wasserstoff steht, man Verbindungen der Formel (VIII)

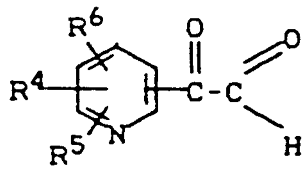 (VIII)

in welcher

$R^4$, $R^5$, $R^6$ die oben angegebene Bedeutung haben

mit Aminen der Formel (IX)

$R^3$-$NH_2$ (IX)

in welcher

$R^3$ die oben angegebene Bedeutung hat,

unter reduzierenden Bedingungen umsetzt, oder

f) für den Fall, daß in Formel I $R^2$ für Wasserstoff steht, Verbindungen der Formel X

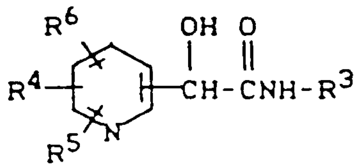 (X)

in welcher

$R^3$, $R^4$, $R^5$, $R^6$ die oben angegebene Bedeutung haben,

reduziert.

**17.** Pyridinderivate der Formel

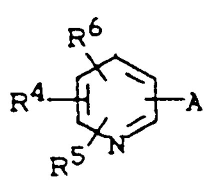

in welcher

$R^4$ für Wasserstoff, $C_{1-4}$-Alkyl, Fluor, Chlor, Brom,$C_{1-4}$-Halogenalkyl, Cyano, $C_{1-4}$-Alkoxycarbonyl, Mono- oder di-$C_{1-4}$-Alkylaminocarbonyl, $C_{1-6}$-Alkoxy, $C_{1-6}$-Halogenalkoxy,$C_{1-6}$-Halogenalkylthio, $NHSO_2$-Alkyl steht,

$R^5$ für Wasserstoff, $C_{1-6}$-Alkoxy, oder den Rest -$NR^7R^8$ steht,

$R^6$ für Wasserstoff, $C_{1-4}$-Alkyl, Fluor, Chlor, Brom, $C_{1-4}$-Halogenalkyl, Cyano, $C_{1-4}$-Alkoxycarbonyl, $C_{1-6}$-Alkoxy, $C_{1-6}$-Halogenalkoxy, $C_{1-6}$-Halogenalkylthio, -NH-$SO_2$-Alkyl steht,

unter der Voraussetzung, daß $R^4$, $R^5$, $R^6$ nicht gleichzeitig für Wasserstoff stehen und

A für die Reste -CHO, -COOH, -COO($C_{1-4}$-Alkyl), -COCl,

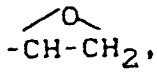,

$-COCH_3$, -CO-CHO, $-COCH_2$-Halogen, $-CH=CH-NO_2$, $-CO-CH_2-COO$($C_{1-4}$-Alkyl),

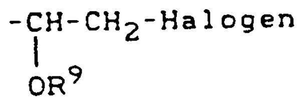, 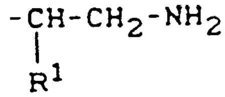, 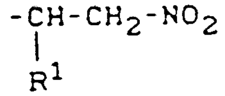,

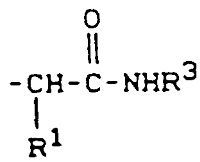

steht und

$R^9$ für Wasserstoff steht,

$R^1$ für Hydroxyl oder Acyloxy steht,

$R^3$ für Wasserstoff, $C_{1-6}$-Alkyl, substituiertes $C_{1-6}$-Alkyl, Cycloalkyl, substituiertes $C_{1-6}$-Alkylphenyl oder Phenyl steht,

$R^7$ für Wasserstoff oder $C_{1-6}$-Alkyl steht,

$R^8$ für Wasserstoff, $C_{1-6}$-Alkyl, $C_{1-6}$-Halogenalkyl oder Acyl steht,

ausgenommen α-(Brommethyl)-2-chlor-4-pyridinmethanol, Brommethyl-2-chlor-4-pyridinketon, 2-chlor-4-pyridylmethylketon, Brommethyl-6-chlor-3-pyridylketon, α-(Brommethyl)-2-chlor-4-pyrdinmethanol, 5-Acetyl-2-fluorpyridin, 5-Brom-acetyl-2-fluorpyridin, (2-Fluorpyrid-5-yl)oxiran.

**18.** Pyridinderivate gemäß Anspruch 17 dadurch gekennzeichnet, daß

$R^5$ für den Rest $-NR^7R^8$ steht,

wobei die Reste A, $R^4$, $R^6$, $R^7$ und $R^8$

die in Anspruch 17 angegebene Bedeutung haben.

**Patentansprüche für folgende Vertragsstaaten : ES, GR**

**1.** Verwendung von Heteroarylethylaminen der Formel (I)

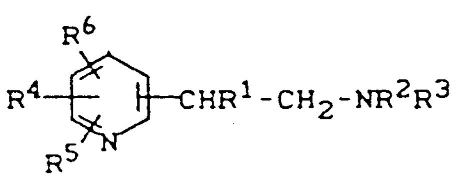

(I)

in welcher

$R^1$ für OH, Acyloxy oder Alkoxy steht

$R^2$ für Wasserstoff oder Alkyl steht,

$R^3$ für Wasserstoff, Alkyl, Cycloalkyl, substituiertes Alkyl, gegebenenfalls substituiertes Aralkyl, Aryl oder Heterocyclyl steht,

$R^2$ und $R^3$ können gemeinsam mit dem Stickstoffatom, an das sie gebunden sind, für einen gegebenenfalls substituierten heterocyclischen Rest stehen,

$R^4$ für Wasserstoff, Alkyl, Halogen, Halogenalkyl, Hydroxyl, Cyano, Alkoxycarbonyl, Aminocarbonyl, Mono- und Dialkylaminocarbonyl, Alkoxy, Halogenalkoxy, Halogenalkylthio, $NHSO_2$-Alkyl steht,

$R^5$ für Wasserstoff, Hydroxyl, Alkoxy oder den Rest $-NR^7R^8$ steht,

$R^6$ für die bei $R^4$ angegebenen Reste steht,
$R^7$ für Wasserstoff oder Alkyl steht,
$R^8$ für Wasserstoff, Alkyl, Halogenalkyl, Acyl steht,

ausgenommen die Verbindung 6-Amino-α-(((1-methyl-3-phenylpropyl)amino)methyl)-3-pyridinmethanol, (Die Verbindungen der Formel I können dabei in Form ihrer Tautomeren sowie in Form ihrer Racemate oder Enantiomeren vorliegen) zur Herstellung von leistungsfördernden Mitteln für Tiere.

**2.** Verwendung von Heteroarylethylaminen der Formel I gemäß Anspruch 1 zur Herstellung von Mitteln zur Förderung und Beschleunigung des Wachstums, zur Verbesserung der Futterverwertung, der Fleischqualität und zur Verschiebung des Fleisch-Fett-Verhältnisses zugunsten von Fleisch bei Nutz-, Zucht-, Zier- und Hobbytieren.

**3.** Verwendung gemäß Ansprüchen 1 und 2 von Heteroarylethylaminen der Formel I gemäß Anspruch 1 in welcher

$R^1$ für OH, $C_{1-6}$-Alkoxy oder Acyloxy steht und Acyloxy für Oxycarbonyl-$C_{1-6}$-alkyl, gegebenenfalls substituiertes Oxycarbonylphenyl, Oxysulfonyl-$C_{1-6}$-alkyl, gegebenenfalls substituiertes Oxysulfonylphenyl steht,
$R^2$ für Wasserstoff oder $C_{1-6}$-Alkyl steht,
$R^3$ für Wasserstoff, gegebenenfalls substituiertes $C_{1-6}$-Alkyl, $C_{3-6}$-Cycloalkyl, $C_{1-6}$-Alkylphenyl, Phenyl steht,
$R^2$ und $R^3$ können gemeinsam mit dem Stickstoffatom, an das sie gebunden sind, für einen gegebenenfalls substituierten 5- bis 6-gliedrigen gesättigten oder ungesättigten heterocyclischen Rest stehen, der gegebenenfalls noch weitere Heteroatome aus der Reihe N, O, S enthalten kann,
$R^4$ für Wasserstoff, $C_{1-4}$-Alkyl, Fluor, Chlor, Brom, $C_{1-4}$-Halogenalkyl, Hydroxyl, Cyano, $C_{1-4}$-Alkoxycarbonyl, Mono- oder -di-$C_{1-4}$-alkylaminocarbonyl, $C_{1-6}$-Alkoxy, $C_{1-6}$-Halogenalkoxy, $C_{1-6}$-Halogenalkylthio, -$NHSO_2$-$C_{1-6}$-Alkyl steht,
$R^5$ für Wasserstoff, Hydroxyl, $C_{1-6}$-Alkoxy oder den Rest -$NR^7R^8$ steht,
$R^6$ für die bei $R^4$ angegebenen Reste steht,
$R^7$ für Wasserstoff oder $C_{1-6}$-Alkyl steht,
$R^8$ für Wasserstoff, $C_{1-6}$-Alkyl, $C_{1-4}$-Halogenalkyl, $C_{1-6}$-Alkylcarbonyl, gegebenenfalls substituiertes Benzoyl, $C_{1-6}$-Alkylsulfonyl, gegebenenfalls substituiertes Phenylsulfonyl steht,

Als Substituenten der gegebenenfalls substituierten Reste kommen bevorzugt infrage: Cyano, Halogen wie Fluor oder Chlor, Hydroxy, $C_{1-4}$-Alkyl, $C_{1-4}$-Halogenalkyl,Phenyl, $C_{1-4}$-Alkoxy, $C_{1-4}$-Halogenalkoxy, $C_{1-4}$-Alkylthio, $C_{1-4}$-Halogenalkylthio, für den Fall, daß die Substituenten an einem Phenylrest sitzen, zusätzlich bevorzugt Methylendioxy, Ethylendioxy, halogensubstituiertes Methylendioxy, halogensubstituiertes Ethylendioxy, ferner Phenyl, Phenoxy, die ihrerseits einen oder mehrere der obengenannten Substituenten tragen können

ausgenommen die Verbindung 6-Amino-α-(((1-methyl-3-phenylpropyl)amino)methyl)-3-pyridinmethanol,
zur Herstellung von leistungsfördernden Mitteln für Tiere.

**4.** Verwendung von Heteroarylethylaminen der Formel I gemäß Anspruch 1,

in welcher

$R^1$ für OH, $C_{1-6}$-Alkoxy, insbesondere Methoxy oder Ethoxy steht,
$R^2$ für Wasserstoff oder $C_{1-4}$-Alkyl, insbesondere Methyl oder Ethyl steht,
$R^3$ für Wasserstoff, gegebenenfalls durch 1-5 Halogenatome substituiertes $C_{1-6}$-Alkyl, insbesondere Methyl, Ethyl, Propyl, Butyl, Pentyl, Hexyl, wobei insbesondere sekundäre und tertiäre Alkylreste genannt seien, sowie $C_{1-6}$-Alkylphenyl, das gegebenenfalls durch Halogen, insbesondere Fluor oder Chlor, $C_{1-4}$-Alkyl, Hydroxy, $C_{1-4}$-Alkoxy, gegebenenfalls halogensubstituiertes Methylendioxy oder Ethylendioxy substituiert sein kann, Phenyl, das gegebenenfalls durch Halogen, insbesondere Fluor oder Chlor, $C_{1-4}$-Alkyl, Hydroxy, $C_{1-4}$-Alkoxy, gegebenenfalls halogensubstituiertes Methylendioxy oder Ethylendioxy substituiert sein kann, steht,
$R^4$ für Wasserstoff, $C_{1-4}$-Alkyl, Halogen, insbesondere Fluor, Chlor, Brom, Cyano, Hydroxy, $C_{1-4}$-Halogenalkyl mit 1 bis 5 Halogenatomen, $C_{1-4}$-Alkoxy, $C_{1-4}$-Halogenalkoxy mit 1 bis 5

Halogenatomen, $NHSO_2$-$C_{1-4}$-Alkyl, -COO-$C_{1-4}$-Alkyl, -$CONH_2$, -CONH-$C_{1-4}$-Alkyl, -CON-($C_{1-4}$-Alkyl)$_2$ steht,

$R^5$ für Wasserstoff, OH, $C_{1-4}$-Alkoxy, -$NR^7R^8$ steht,

$R^6$ für die bei $R^4$ angegebenen Reste steht,

$R^7$ für Wasserstoff, $C_{1-4}$-Alkyl steht,

$R^8$ für Wasserstoff, $C_{1-4}$-Alkyl steht.

ausgenommen die Verbindung 6-Amino-α-(((1-methyl-3-phenylpropyl)amino)methyl)-3-pyridinmethanol zur Herstellung von leistungsfördernden Mitteln für Tiere.

**5.** Verwendung gemäß Ansprüchen 1 und 2 des Heteroarylethylamins der Formel

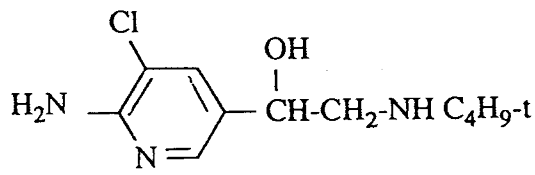

zur Herstellung von leistungsfördernden Mitteln für Tiere.

**6.** Verwendung gemäß Ansprüchen 1 und 2 des Heteroarylethylamins der Formel

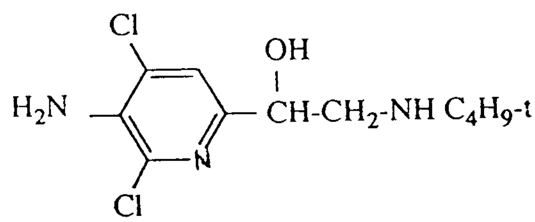

zur Herstellung von leistungsfördernden Mitteln für Tiere.

**7.** Verfahren zur Herstellung von leistungsfördernden Mitteln für Tiere, dadurch gekennzeichnet, daß man Heteroarylethylamine der allgemeinen Formel (I) gemäß Ansprüchen 1 - 6 mit Streck-, Verdünnungs- und Nahrungsmitteln sowie gegebenenfalls weiteren Hilfsstoffen mischt.

**8.** Verfahren zur Herstellung der Heteroarylethylamine der Formel I,

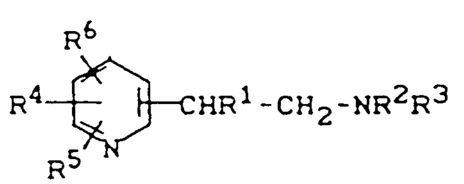

(I)

in welcher

$R^1$ für OH, $C_{1-6}$-Alkoxy oder für Oxycarbonyl-$C_{1-6}$-alkyl, gegebenenfalls substituiertes Oxycarbonylphenyl,Oxysulfonyl-$C_{1-6}$-alkyl, gegebenenfalls substituiertes Oxysulfonylphenyl steht,

$R^2$ für Wasserstoff oder $C_{1-6}$-Alkyl steht,

$R^3$ für Wasserstoff, gegebenenfalls substituiertes $C_{1-6}$-Alkyl, $C_{3-6}$-Cycloalkyl, substituiertes $C_{1-6}$-Alkylphenyl oder Phenyl steht,

$R^4$ für Wasserstoff, $C_{1-4}$-Alkyl, Fluor, Chlor, Brom, $C_{1-4}$-Halogenalkyl, Cyano, $C_{1-4}$-Alkoxycarbonyl, Mono- oder -di-$C_{1-4}$-alkylaminocarbonyl, $C_{1-6}$-Alkoxy, $C_{1-6}$-Halogenalkoxy, $C_{1-6}$-Halogenalkylthio, -$NHSO_2$-$C_{1-6}$-Alkyl steht,

$R^5$ für Wasserstoff, $C_{1-6}$-Alkoxy oder den Rest -$NR^7R^8$ steht,

$R^6$ für Wasserstoff, $C_{1-4}$-Alkyl, Fluor, Chlor, Brom, $C_{1-4}$-Halogenalkyl, Cyano, $C_{1-4}$-Alkoxycarbonyl, $C_{1-6}$-Alkoxy, $C_{1-6}$-Halogenalkoxy, $C_{1-6}$-Halogenalkylthio, $NHSO_2$-Alkyl steht,

unter der Voraussetzung, daß $R^4$, $R^5$, $R^6$ nicht gleichzeitig für Wasserstoff stehen,

$R^7$ für Wasserstoff oder $C_{1-6}$-Alkyl steht,

$R^8$ für Wasserstoff, $C_{1-6}$-Alkyl, $C_{1-6}$-Halogenalkyl oder Acyl steht.

Wobei als Substituenten der gegebenenfalls substituierten Reste genannt seien: Cyano, Halogen wie Fluor oder Chlor, Hydroxy, $C_{1-4}$-Alkyl, $C_{1-4}$-Halogenalkyl,Phenyl, $C_{1-4}$-Alkoxy, $C_{1-4}$-Halogenalkoxy, $C_{1-4}$-Alkylthio, $C_{1-4}$-Halogenalkylthio, für den Fall, daß die Substituenten an einem Phenylrest sitzen, zusätzlich Methylendioxy, Ethylendioxy, halogensubstituiertes Methylendioxy, halogensubstituiertes Ethylendioxy, ferner Phenyl, Phenoxy, die ihrerseits einen oder mehrere der obengenannten Substituenten tragen können,

sowie ihre physiologisch verträglichen Salze und ihre N-Oxide,

ausgenommen 6-Chlor-α(isopropylaminomethyl)-3-pyridinmethanol und 2-Chlor-α-(isopropylaminomethyl)-4-pyridinmethanol,

dadurch gekennzeichnet, daß man

a) Halogenmethylketone der Formel (II)

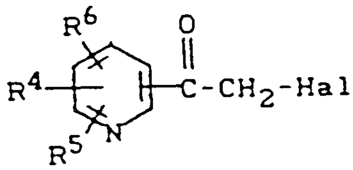

(II)

in welcher

$R^4$, $R^5$, $R^6$ die oben angegebene Bedeutung haben und Hal für Halogen steht,

mit Aminen der Formel (III)

$HNR^2R^3$ (III)

in welcher

$R^2$ und $R^3$ die oben angegebene Bedeutung haben,

umsetzt und anschließend die Carbonylgruppe reduziert, oder

b) Epoxide der Formel (IV)

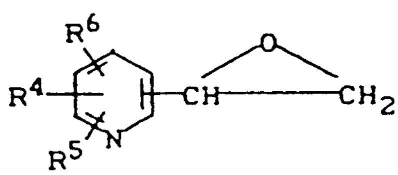

(IV)

in welcher

$R^4$, $R^5$, $R^6$ die oben angegebene Bedeutung haben

mit Aminen der Formel (III)

$HNR^2R^3$ (III)

in welcher

$R^2$ und $R^3$ die oben angegebene Bedeutung haben,

umsetzt oder

c) beta-Halogenethylverbindungen der Formel (V)

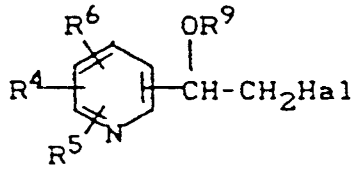

(V)

in welcher

$R^4$, $R^5$, $R^6$ die oben angegebene Bedeutung haben,

Hal für Halogen steht,

$R^9$ für Wasserstoff steht,

mit Aminen der Formel (III)

$HNR^2R^3$ (III)

in welcher

$R^2$ und $R^3$ die oben angegebene Bedeutung haben

umsetzt oder

d) für den Fall, daß in Formel Ia $R^2$ für Wasserstoff steht und $R^3$ für substituiertes Alkyl oder Aralkyl steht, man Verbindungen der Formel (VI)

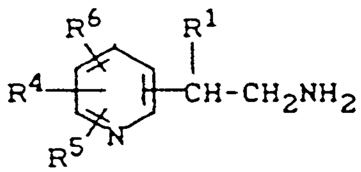

(VI)

in welcher

$R^1$, $R^4$, $R^5$, $R^6$ die oben angegebene Bedeutung haben

mit Verbindungen der Formel (VII)

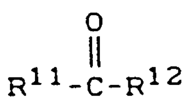

(VII)

in welcher

$R^{11}$ für substituiertes Alkyl, Aryl oder Aralkyl steht,

$R^{12}$ für Wasserstoff oder $C_{1-4}$-Alkyl steht,

unter reduzierenden Bedingungen umsetzt, oder

e) für den Fall, daß in Formel I $R^2$ für Wasserstoff steht, man Verbindungen der Formel (VIII)

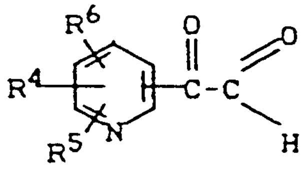

(VIII)

in welcher

$R^4$, $R^5$, $R^6$ die oben angegebene Bedeutung haben

mit Aminen der Formel (IX)

$R^3$-$NH_2$ (IX)

in welcher

$R^3$ die oben angegebene Bedeutung hat,

unter reduzierenden Bedingungen umsetzt, oder

f) für den Fall , daß in Formel I $R^2$ für Wasserstoff steht, Verbindungen der Formel X

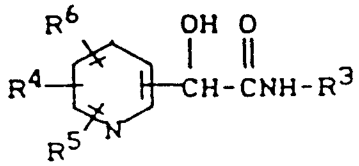

(X)

in welcher

$R^3$, $R^4$, $R^5$, $R^6$ die oben angegebene Bedeutung haben,

reduziert.

**9.** Verfahren gemäß Anspruch 8 zur Herstellung der Heteroarylethylamine der Formel I

in welcher

$R^1$ für OH, $C_{1-6}$-Alkoxy steht,

$R^2$ für Wasserstoff oder $C_{1-4}$-Alkyl steht,

$R^3$ für Wasserstoff, gegebenenfalls durch 1 bis 5 Halogenatome substituiertes $C_{1-6}$-Alkyl sowie $C_{1-6}$-Alkylphenyl, das durch Halogen, $C_{1-4}$-Alkyl, Hydroxy, $C_{1-4}$-Alkoxy, gegebenenfalls halogensubstituiertes Methylendioxy oder Ethylendioxy substituiert ist, steht,

$R^4$ für Wasserstoff, $C_{1-4}$-Alkyl, Halogen, Cyano, $C_{1-4}$-Halogenalkyl mit 1 bis 5 Halogenatomen, $C_{1-4}$-Alkoxy, $C_{1-4}$-Halogenalkoxy mit 1 bis 5 Halogenatomen, $NHSO_2$-$C_{1-4}$-Alkyl, -COO-$C_{1-4}$-Alkyl, $-CONH_2$, -CONH-$C_{1-4}$-Alkyl, -CON($C_{1-4}$-Alkyl)$_2$ steht,

$R^5$ für Wasserstoff, $C_{1-4}$-Alkoxy, $-NR^7R^8$ steht,

$R^6$ für die bei $R^4$ angegebenen Reste steht,

$R^7$ für Wasserstoff, $C_{1-4}$-Alkyl steht,

$R^8$ für Wasserstoff, $C_{1-4}$-Alkyl steht,

sowie ihre physiologisch verträglichen Salze und ihre N-Oxide,

ausgenommen 6-Chlor-α(isopropylaminomethyl)-3-pyridinmethanol und 2-Chlor-α-(isopropylaminomethyl)-4-pyridinmethanol.

**10.** Verfahren gemäß Anspruch 8 zur Herstellung des Heteroarylethylamins der Formel

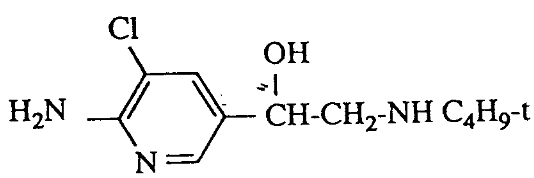

**11.** Verfahren gemäß Anspruch 8 zur Herstellung des Heteroarylethylamins der Formel

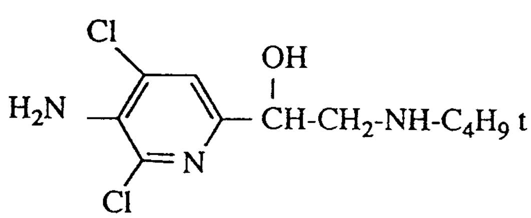

## Claims

**Claims for the following Contracting States : AT, BE, CH, DE, FR, GB, IT, LI, NL, SE**

**1.** Use of heteroarylethylamines of the formula (I)

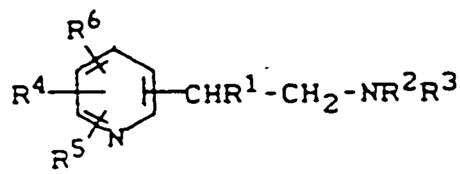

(I)

in which

| | |
|---|---|
| $R^1$ | represents OH, acyloxy or alkoxy, |
| $R^2$ | represents hydrogen or alkyl, |
| $R^3$ | represents hydrogen, alkyl, cycloalkyl, substituted alkyl, optionally substituted aralkyl, aryl or heterocyclyl, |
| $R^2$ and $R^3$, | together with the nitrogen atom to which they are bonded, can represent an optionally substituted heterocyclic radical, |
| $R^4$ | represents hydrogen, alkyl, halogen, halogenoalkyl, hydroxyl, cyano, alkoxycarbonyl, aminocarbonyl, mono- and dialkylaminocarbonyl, alkoxy, halogenoalkoxy, halogenoalkylthio or $NHSO_2$-alkyl, |
| $R^5$ | represents hydrogen, hydroxyl, alkoxy or the radical $-NR^7R^8$, |
| $R^6$ | represents the radicals mentioned for $R^4$, |
| $R^7$ | represents hydrogen or alkyl and |
| $R^8$ | represents hydrogen, alkyl, halogenoalkyl or acyl, |

excluding the compound 6-amino-α-(((1-methyl-3-phenylpropyl)amino)methyl)-3-pyridinemethanol, (the compounds of the formula I can be present here in the form of their tautomers and in the form of their racemates or enantiomers) for promoting the yield of animals.

**2.** Use of heteroarylethylamines of the formula I according to Claim 1, for promoting and accelerating growth, for improving feed conversion and meat quality and for shifting the meat/fat ratio in favour of meat in stock, breeding and ornamental animals and pets.

**3.** Use according to Claims 1 and 2 of heteroarylethylamines of the formula I according to Claim 1, in which

| | |
|---|---|
| $R^1$ | represents OH, $C_{1-6}$-alkoxy or acyloxy and acyloxy represents oxycarbonyl-$C_{1-6}$-alkyl, optionally substituted oxycarbonylphenyl, oxysulphonyl-$C_{1-6}$-alkyl or optionally substituted oxysulphonylphenyl, |
| $R^2$ | represents hydrogen or $C_{1-6}$-alkyl, |
| $R^3$ | represents hydrogen, optionally substituted $C_{1-6}$-alkyl, $C_{3-6}$-cycloalkyl, $C_{1-6}$-alkylphenyl or phenyl, |
| $R^2$ and $R^3$, | together with the nitrogen atom to which they are bonded, can represent an optionally substituted 5- or 6-membered saturated or unsaturated heterocyclic radical, which can optionally also contain further hetero atoms from the series comprising N, O and S, |
| $R^4$ | represents hydrogen, $C_{1-4}$-alkyl, fluorine, chlorine, bromine, $C_{1-4}$-halogenoalkyl, hydroxyl, cyano, $C_{1-4}$-alkoxycarbonyl, mono- or di-$C_{1-4}$-alkylaminocarbonyl, $C_{1-6}$-alkoxy, $C_{1-6}$-halogenoalkoxy, $C_{1-6}$-halogenoalkylthio or $-NHSO_2-C_{1-6}$-alkyl, |
| $R^5$ | represents hydrogen, hydroxyl, $C_{1-6}$-alkoxy or the radical $-NR^7R^8$, |
| $R^6$ | represents the radicals mentioned for $R^4$, |
| $R^7$ | represents hydrogen or $C_{1-6}$-alkyl and |
| $R^8$ | represents hydrogen, $C_{1-6}$-alkyl, $C_{1-4}$-halogenoalkyl, $C_{1-6}$-alkylcarbonyl, optionally substituted benzoyl, $C_{1-6}$-alkylsulphonyl or optionally substituted phenylsulphonyl; |

preferred possible substituents of the optionally substituted radicals being: cyano, halogen, such as fluorine or chlorine, hydroxyl, $C_{1-4}$-alkyl, $C_{1-4}$-halogenoalkyl, phenyl, $C_{1-4}$-alkoxy, $C_{1-4}$-halogenoalkoxy, $C_{1-4}$-alkylthio and $C_{1-4}$-halogenoalkylthio, and in the case where the substituents are on a

phenyl radical, additionally preferably methylenedioxy, ethylenedioxy, halogen-substituted methylenedioxy, halogen-substituted ethylenedioxy, and furthermore phenyl and phenoxy, which in turn can carry one or more of the abovementioned substituents,
excluding the compound 6-amino-α-(((1-methyl-3-phenylpropyl)amino)methyl)-3-pyridinemethanol.

**4.** Use according to Claims 1 and 2 of heteroarylethylamines of the formula I according to Claim 1, in which

$R^1$ represents OH or $C_{1-6}$-alkoxy, in particular methoxy or ethoxy,
$R^2$ represents hydrogen or $C_{1-4}$-alkyl, in particular methyl or ethyl,
$R^3$ represents hydrogen, $C_{1-6}$-alkyl, in particular methyl, ethyl, propyl, butyl, pentyl or hexyl, secondary and tertiary alkyl radicals being mentioned in particular, which is optionally substituted by 1-5 halogen atoms, or $C_{1-6}$-alkylphenyl, which can optionally be substituted by halogen, in particular fluorine or chlorine, $C_{1-4}$-alkyl, hydroxyl, $C_{1-4}$-alkoxy or optionally halogen-substituted methylenedioxy or ethylenedioxy, or phenyl, which can optionally be substituted by halogen, in particular fluorine or chlorine, $C_{1-4}$-alkyl, hydroxyl, $C_{1-4}$-alkoxy or optionally halogen-substituted methylenedioxy or ethylenedioxy,
$R^4$ represents hydrogen, $C_{1-4}$-alkyl, halogen, in particular fluorine, chlorine or bromine, cyano, hydroxyl, $C_{1-4}$-halogenoalkyl having 1 to 5 halogen atoms, $C_{1-4}$-alkoxy, $C_{1-4}$-halogenoalkoxy having 1 to 5 halogen atoms, $NHSO_2$-$C_{1-4}$-alkyl, -COO-$C_{1-4}$-alkyl, $-CONH_2$, -CONH-$C_{1-4}$-alkyl or -CON($C_{1-4}$-alkyl$)_2$,
$R^5$ represents hydrogen, OH, $C_{1-4}$-alkoxy or $-NR^7R^8$,
$R^6$ represents the radicals mentioned for $R^4$,
$R^7$ represents hydrogen or $C_{1-4}$-alkyl and
$R^8$ represents hydrogen or $C_{1-4}$-alkyl,

excluding the compound 6-amino-α-(((1-methyl-3-phenylpropyl)amino)methyl)-3-pyridinemethanol.

**5.** Use according to Claims 1 and 2 of the heteroarylethylamine of the formula

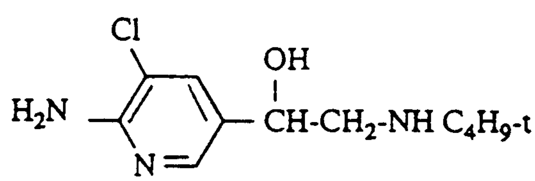

**6.** Use according to Claims 1 and 2 of the heteroarylethylamine of the formula

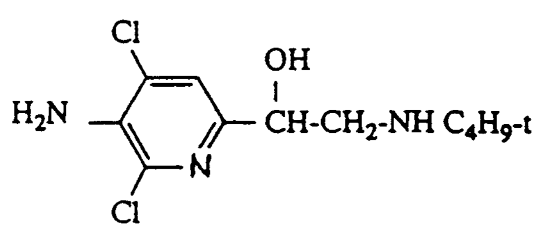

**7.** Animal food and premixes for the preparation of animal food, comprising heteroarylethylamines of the general formula (I) according to Claims 1 - 6.

**8.** Yield-promoting agents for animals, comprising heteroarylethylamines of the general formula (I) according to Claims 1-6.

**9.** Process for the preparation of yield-promoting agents for animals, characterised in that heteroarylethylamines of the general formula (I) according to Claims 1 - 6 are mixed with extenders, diluents and foodstuffs and if appropriate other auxiliaries.

**10.** Use of heteroarylethylamines of the formula (I) according to Claims 1 - 6 for the preparation of yield-promoting agents.

**11.** Use of heteroarylethylamines of the formula (I) according to Claims 1 - 6 for the preparation of agents for promoting and accelerating growth, for improving feed conversion and meat quality and for shifting the meat/fat ratio in favour of meat in stock, breeding and ornamental animals and pets.

**12.** Heteroarylethylamines of the formula I

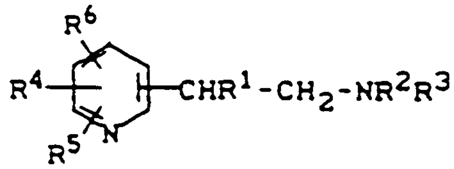

(I)

in which

$R^1$ represents OH or $C_{1-6}$-alkoxy, or represents oxycarbonyl-$C_{1-6}$-alkyl, optionally substituted oxycarbonylphenyl, oxysulphonyl-$C_{1-6}$-alkyl or optionally substituted oxysulphonylphenyl,

$R^2$ represents hydrogen or $C_{1-6}$-alkyl,

$R^3$ represents hydrogen, optionally substituted $C_{1-6}$-alkyl, $C_{3-6}$-cycloalkyl, substituted $C_{1-6}$-alkylphenyl or phenyl,

$R^4$ represents hydrogen, $C_{1-4}$-alkyl, fluorine, chlorine, bromine, $C_{1-4}$-halogenoalkyl, cyano, $C_{1-4}$-alkoxycarbonyl, mono- or di-$C_{1-4}$-alkylaminocarbonyl, $C_{1-6}$-alkoxy,$C_{1-6}$-halogenoalkoxy, $C_{1-6}$-halogenoalkylthio or -$NHSO_2$-$C_{1-6}$-alkyl,

$R^5$ represents hydrogen, $C_{1-6}$-alkoxy or the radical -$NR^7R^8$,

$R^6$ represents hydrogen, $C_{1-4}$-alkyl, fluorine, chlorine, bromine, $C_{1-4}$-halogenoalkyl, cyano, $C_{1-4}$-alkoxycarbonyl, $C_{1-6}$-alkoxy, $C_{1-6}$-halogenoalkoxy, $C_{1-6}$-halogenoalkylthio or $NHSO_2$-alkyl,

with the proviso that $R^4$, $R^5$ and $R^6$ do not simultaneously represent hydrogen,

$R^7$ represents hydrogen or $C_{1-6}$-alkyl and

$R^8$ represents hydrogen, $C_{1-6}$-alkyl, $C_{1-6}$-halogenoalkyl or acyl,

substituents of the optionally substituted radicals which may be mentioned being: cyano, halogen, such as fluorine or chlorine, hydroxyl, $C_{1-4}$-alkyl, $C_{1-4}$-halogenoalkyl, phenyl, $C_{1-4}$-alkoxy, $C_{1-4}$-halogenoalkoxy, $C_{1-4}$-alkylthio and $C_{1-4}$-halogenoalkylthio, and in the case where the substituents are on a phenyl radical, additionally methylenedioxy, ethylenedioxy, halogen-substituted methylenedioxy, halogen-substituted ethylenedioxy and furthermore phenyl and phenoxy, which in turn can carry one or more of the abovementioned substituents,

and their physiologically tolerated salts and their N-oxides,

excluding 6-chloro-α-(isopropylaminomethyl)-3-pyridinemethanol and 2-chloro-α-(isopropylaminomethyl)-4-pyridinemethanol.

**13.** Heteroarylethylamines of the formula I according to Claim 12,

in which

$R^1$ represents OH or $C_{1-6}$-alkoxy,

$R^2$ represents hydrogen or $C_{1-4}$-alkyl,

$R^3$ represents hydrogen, $C_{1-6}$-alkyl which is optionally substituted by 1 to 5 halogen atoms or $C_{1-6}$-alkylphenyl which is substituted by halogen, $C_{1-4}$-alkyl, hydroxyl, $C_{1-4}$-alkoxy or optionally halogen-substituted methylenedioxy or ethylenedioxy,

$R^4$ represents hydrogen, $C_{1-4}$-alkyl, halogen, cyano, $C_{1-4}$-halogenoalkyl having 1 to 5 halogen atoms, $C_{1-4}$-alkoxy, $C_{1-4}$-halogenoalkoxy having 1 to 5 halogen atoms, $NHSO_2$-$C_{1-4}$-alkyl, -COO-$C_{1-4}$-alkyl, -$CONH_2$, -CONH-$C_{1-4}$-alkyl or -CON($C_{1-4}$-alkyl)$_2$,

$R^5$ represents hydrogen, $C_{1-4}$-alkoxy or -$NR^7R^8$,

$R^6$ represents the radicals mentioned for $R^4$,

$R^7$ represents hydrogen or $C_{1-4}$-alkyl and

$R^8$ represents hydrogen or $C_{1-4}$-alkyl,

and their physiologically tolerated salts and their N-oxides,

excluding 6-chloro-α-(isopropylaminomethyl)-3-pyridinemethanol and 2-chloro-α-

(isopropylaminomethyl)-4-pyridinemethanol.

**14.** The heteroarylethylamine of the formula

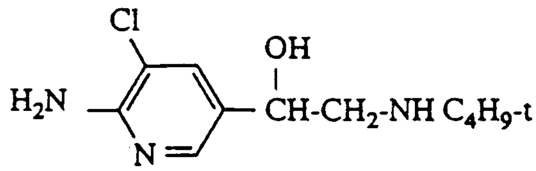

**15.** The heteroarylethylamine of the formula

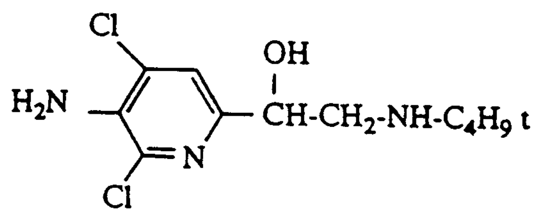

**16.** Process for the preparation of the heteroarylethylamines of the formula I according to Claim 12

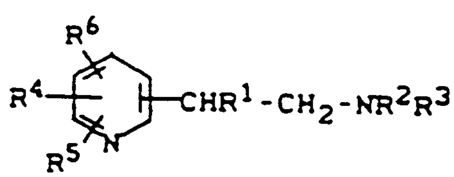

(I)

in which

$R^1$, $R^2$, $R^3$, $R^4$, $R^5$ and $R^6$ have the meaning given in Claim 12,

characterised in that

a) halogenomethyl ketones of the formula (II)

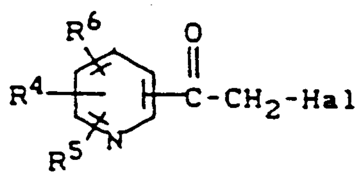

(II)

in which

$R^4$, $R^5$ and $R^6$ have the meaning given in Claim 12 and Hal represents halogen,

are reacted with amines of the formula (III)

$HNR^2R^3$ (III)

in which

$R^2$ and $R^3$ have the meaning given in Claim 12,

and the carbonyl group is then reduced, or

b) epoxides of the formula IV)

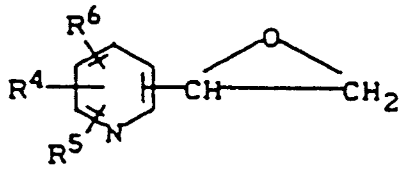

(IV)

in which

$R^4$, $R^5$ and $R^6$ have the abovementioned meaning,

are reacted with amines of the formula (III)

$HNR^2R^3$ (III)

in which

$R^2$ and $R^3$ have the abovementioned meaning,

or

c) beta-halogenoethyl compounds of the formula (V)

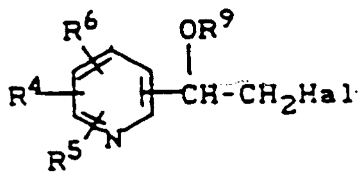

(V)

in which

$R^4$, $R^5$ and $R^6$ have the abovementioned meaning,

Hal represents halogen and

$R^9$ represents hydrogen,

are reacted with amines of the formula (III)

$HNR^2R^3$ (III)

in which

$R^2$ and $R^3$ have the abovementioned meaning,

or

d) in the case where, in formula I, $R^2$ represents hydrogen and $R^3$ represents substituted alkyl or aralkyl, compounds of the formula (VI)

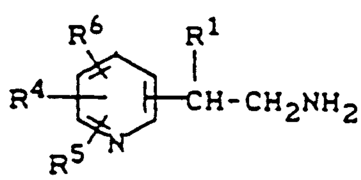

(VI)

in which

$R^1$, $R^4$, $R^5$ and $R^6$ have the meaning given in Claim 12,

are reacted with compounds of the formula (VII)

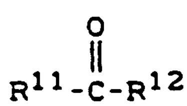

(VII)

in which

$R^{11}$ represents substituted alkyl, aryl or aralkyl and

$R^{12}$ represents hydrogen or $C_{1-4}$-alkyl,

under reducing conditions, or

e) in the case where, in formula I, $R^2$ represents hydrogen, compounds of the formula (VIII)

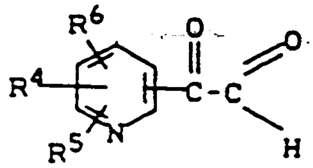

(VIII)

in which

$R^4$, $R^5$ and $R^6$ have the abovementioned meaning,

are reacted with amines of the formula (IX)

$R^3$-$NH_2$ (IX)

in which

$R^3$ has the abovementioned meaning,

under reducing conditions, or

f) in the case where, in formula I, $R^2$ represents hydrogen, compounds of the formula X

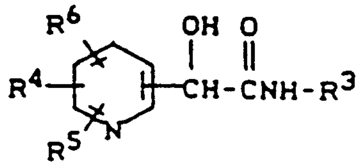

(X)

in which

$R^3$, $R^4$, $R^5$ and $R^6$ have the abovementioned meaning,

are reduced.

**17.** Pyridine derivatives of the formula

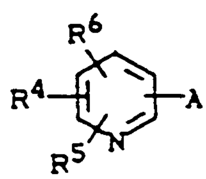

in which

$R^4$ represents hydrogen, $C_{1-4}$-alkyl, fluorine, chlorine, bromine, $C_{1-4}$-halogenoalkyl, cyano, $C_{1-4}$-alkoxycarbonyl, mono- or di-$C_{1-4}$-alkylaminocarbonyl, $C_{1-6}$-alkoxy,$C_{1-6}$-halogenoalkoxy, $C_{1-6}$-halogenoalkylthio or $NHSO_2$-alkyl,

$R^5$ represents hydrogen, $C_{1-6}$-alkoxy or the radical -$NR^7R^8$,

$R^6$ represents hydrogen, $C_{1-4}$-alkyl, fluorine, chlorine, bromine, $C_{1-4}$-halogenoalkyl, cyano, $C_{1-4}$-alkoxycarbonyl, $C_{1-6}$-alkoxy, $C_{1-6}$-halogenoalkoxy, $C_{1-6}$-halogenoalkylthio or -NH-$SO_2$-alkyl,

with the proviso that $R^4$, $R^5$ and $R^6$ do not simultaneously represent hydrogen, and

A represents the radicals -CHO, -COOH, -COO($C_{1-4}$-alkyl), -COCl,

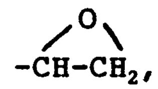

$-COCH_3$, -CO-CHO, $-COCH_2$-halogen, $-CH=CH-NO_2$, $-CO-CH_2-COO(C_{1-4}$-alkyl),

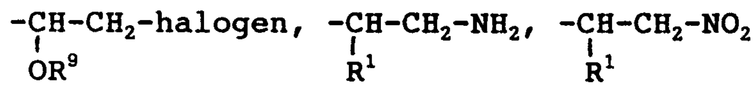 or

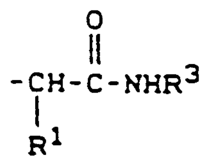

and

- $R^9$ represents hydrogen,
- $R^1$ represents hydroxyl or acyloxy,
- $R^3$ represents hydrogen, $C_{1-6}$-alkyl, substituted $C_{1-6}$-alkyl, cycloalkyl, substituted $C_{1-6}$-alkyl-phenyl or phenyl,
- $R^7$ represents hydrogen or $C_{1-6}$-alkyl and
- $R^8$ represents hydrogen, $C_{1-6}$-alkyl, $C_{1-6}$-halogenoalkyl or acyl,

excluding α-(bromomethyl)-2-chloro-4-pyridinemethanol, bromomethyl 2-chloro-4-pyridine ketone, 2-chloro-4-pyridyl methyl ketone, bromomethyl 6-chloro-3-pyridyl ketone, α-(bromomethyl)-2-chloro-4-pyridinemethanol, 5-acetyl-2-fluoropyridine, 5-bromo-acetyl-2-fluoropyridine and (2-fluoropyrid-5-yl)-oxirane.

**18.** Pyridine derivatives according to Claim 17, characterised in that

- $R^5$ represents the radical $-NR^7R^8$, wherein the radicals A, $R^4$, $R^6$, $R^7$ and $R^8$ have the meaning given in Claim 17.

**Claims for the following Contracting States : ES, GR**

**1.** Use of heteroarylethylamines of the formula (I)

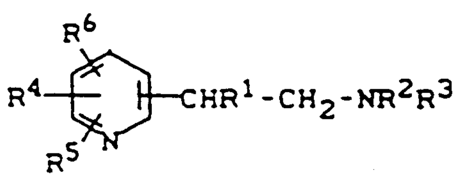 (I)

in which

- $R^1$ represents OH, acyloxy or alkoxy,
- $R^2$ represents hydrogen or alkyl,
- $R^3$ represents hydrogen, alkyl, cycloalkyl, substituted alkyl, optionally substituted aralkyl, aryl or heterocyclyl,
- $R^2$ and $R^3$, together with the nitrogen atom to which they are bonded, can represent an optionally substituted heterocyclic radical,
- $R^4$ represents hydrogen, alkyl, halogen, halogenoalkyl, hydroxyl, cyano, alkoxycarbonyl, aminocarbonyl, mono- and dialkylaminocarbonyl, alkoxy, halogenoalkoxy, halogenoalkylthio or $NHSO_2$-alkyl,
- $R^5$ represents hydrogen, hydroxyl, alkoxy or the radical $-NR^7R^8$,

$R^6$ represents the radicals mentioned for $R^4$,
$R^7$ represents hydrogen or alkyl and
$R^8$ represents hydrogen, alkyl, halogenoalkyl or acyl,

excluding the compound 6-amino-α-(((1-methyl-3-phenylpropyl)amino)methyl)-3-pyridinemethanol, (the compounds of the formula I can be present here in the form of their tautomers and in the form of their racemates or enantiomers) for the preparation of yield-promoting agents for animals.

**2.** Use of heteroarylethylamines of the formula I according to Claim 1, for the preparation of agents for promoting and accelerating growth, for improving feed conversion and meat quality and for shifting the meat/fat ratio in favour of meat in stock, breeding and ornamental animals and pets.

**3.** Use according to Claims 1 and 2 of heteroarylethylamines of the formula I according to Claim 1, in which

$R^1$ represents OH, $C_{1-6}$-alkoxy or acyloxy and acyloxy represents oxycarbonyl-$C_{1-6}$-alkyl, optionally substituted oxycarbonylphenyl, oxysulphonyl-$C_{1-6}$-alkyl or optionally substituted oxysulphonylphenyl,
$R^2$ represents hydrogen or $C_{1-6}$-alkyl,
$R^3$ represents hydrogen, optionally substituted $C_{1-6}$-alkyl, $C_{3-6}$-cycloalkyl, $C_{1-6}$-alkylphenyl or phenyl,
$R^2$ and $R^3$, together with the nitrogen atom to which they are bonded, can represent an optionally substituted 5- or 6-membered saturated or unsaturated heterocyclic radical, which can optionally also contain further hetero atoms from the series comprising N, O and S,
$R^4$ represents hydrogen, $C_{1-4}$-alkyl, fluorine, chlorine, bromine, $C_{1-4}$-halogenoalkyl, hydroxyl, cyano, $C_{1-4}$-alkoxycarbonyl, mono- or di-$C_{1-4}$-alkylaminocarbonyl, $C_{1-6}$-alkoxy, $C_{1-6}$-halogenoalkoxy, $C_{1-6}$-halogenoalkylthio or -$NHSO_2$-$C_{1-6}$-alkyl,
$R^5$ represents hydrogen, hydroxyl, $C_{1-6}$-alkoxy or the radical -$NR^7R^8$,
$R^6$ represents the radicals mentioned for $R^4$,
$R^7$ represents hydrogen or $C_{1-6}$-alkyl and
$R^8$ represents hydrogen, $C_{1-6}$-alkyl, $C_{1-4}$-halogenoalkyl, $C_{1-6}$-alkylcarbonyl, optionally substituted benzoyl, $C_{1-6}$-alkylsulphonyl or optionally substituted phenylsulphonyl;

preferred possible substituents of the optionally substituted radicals being: cyano, halogen, such as fluorine or chlorine, hydroxyl, $C_{1-4}$-alkyl, $C_{1-4}$-halogenoalkyl, phenyl, $C_{1-4}$-alkoxy, $C_{1-4}$-halogenoalkoxy, $C_{1-4}$-alkylthio and $C_{1-4}$-halogenoalkylthio, and in the case where the substituents are on a phenyl radical, additionally preferably methylenedioxy, ethylenedioxy, halogen-substituted methylenedioxy, halogen-substituted ethylenedioxy, and furthermore phenyl and phenoxy, which in turn can carry one or more of the abovementioned substituents, excluding the compound 6-amino-α-(((1-methyl-3-phenylpropyl)amino)methyl)-3-pyridinemethanol, for the preparation of yield-promoting agents for animals.

**4.** Use of heteroarylethylamines of the formula I according to Claim 1, in which

$R^1$ represents OH or $C_{1-6}$-alkoxy, in particular methoxy or ethoxy,
$R^2$ represents hydrogen or $C_{1-4}$-alkyl, in particular methyl or ethyl,
$R^3$ represents hydrogen, $C_{1-6}$-alkyl, in particular methyl, ethyl, propyl, butyl, pentyl or hexyl, secondary and tertiary alkyl radicals being mentioned in particular, which is optionally substituted by 1-5 halogen atoms, or $C_{1-6}$-alkylphenyl, which can optionally be substituted by halogen, in particular fluorine or chlorine, $C_{1-4}$-alkyl, hydroxyl, $C_{1-4}$-alkoxy or optionally halogen-substituted methylenedioxy or ethylenedioxy, or phenyl, which can optionally be substituted by halogen, in particular fluorine or chlorine, $C_{1-4}$-alkyl, hydroxyl, $C_{1-4}$-alkoxy or optionally halogen-substituted methylenedioxy or ethylenedioxy,
$R^4$ represents hydrogen, $C_{1-4}$-alkyl, halogen, in particular fluorine, chlorine or bromine, cyano, hydroxyl, $C_{1-4}$-halogenoalkyl having 1 to 5 halogen atoms, $C_{1-4}$-alkoxy, $C_{1-4}$-halogenoalkoxy having 1 to 5 halogen atoms, $NHSO_2$-$C_{1-4}$-alkyl, -COO-$C_{1-4}$-alkyl, -$CONH_2$, -CONH-$C_{1-4}$-alkyl or -CON ($C_{1-4}$-alkyl)$_2$,
$R^5$ represents hydrogen, OH, $C_{1-4}$-alkoxy or -$NR^7R^8$,
$R^6$ represents the radicals mentioned for $R^4$,
$R^7$ represents hydrogen or $C_{1-4}$-alkyl and
$R^8$ represents hydrogen or $C_{1-4}$-alkyl,

excluding the compound 6-amino-α-(((1-methyl-3-phenylpropyl)amino)methyl)-3-pyridinemethanol, for the preparation of yield-promoting agents for animals.

**5.** Use according to Claims 1 and 2 of the heteroarylethylamine of the formula

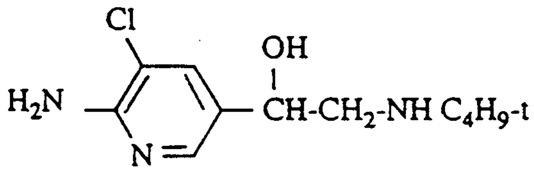

for the preparation of yield-promoting agents for animals.

**6.** Use according to Claims 1 and 2 of the heteroarylethylamine of the formula

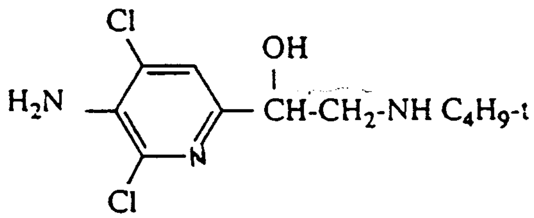

for the preparation of yield-promoting agents for animals.

**7.** Process for the preparation of yield-promoting agents for animals, characterised in that heteroarylethylamines of the general formula (I) according to Claims 1 - 6 are mixed with extenders, diluents and foodstuffs and if appropriate other auxiliaries.

**8.** Process for the preparation of the heteroarylethylamines of the formula I

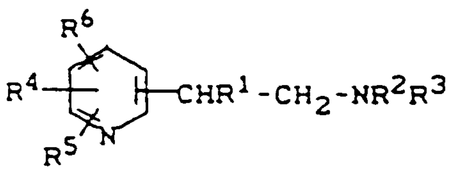

(I)

in which

$R^1$ represents OH or $C_{1-6}$-alkoxy, or represents oxycarbonyl-$C_{1-6}$-alkyl, optionally substituted oxycarbonylphenyl, oxysulphonyl-$C_{1-6}$-alkyl or optionally substituted oxysulphonylphenyl,

$R^2$ represents hydrogen or $C_{1-6}$-alkyl,

$R^3$ represents hydrogen, optionally substituted $C_{1-6}$-alkyl, $C_{3-6}$-cycloalkyl, substituted $C_{1-6}$-alkylphenyl or phenyl,

$R^4$ represents hydrogen, $C_{1-4}$-alkyl, fluorine, chlorine, bromine, $C_{1-4}$-halogenoalkyl, cyano, $C_{1-4}$-alkoxycarbonyl, mono- or di-$C_{1-4}$-alkylaminocarbonyl, $C_{1-6}$-alkoxy,$C_{1-6}$-halogenoalkoxy, $C_{1-6}$-halogenoalkylthio or -$NHSO_2$-$C_{1-6}$-alkyl,

$R^5$ represents hydrogen, $C_{1-6}$-alkoxy or the radical -$NR^7R^8$,

$R^6$ represents hydrogen, $C_{1-4}$-alkyl, fluorine, chlorine, bromine, $C_{1-4}$-halogenoalkyl, cyano, $C_{1-4}$-alkoxycarbonyl, $C_{1-6}$-alkoxy, $C_{1-6}$-halogenoalkoxy, $C_{1-6}$-halogenoalkylthio or $NHSO_2$-alkyl,

with the proviso that $R^4$, $R^5$ and $R^6$ do not simultaneously represent hydrogen,

$R^7$ represents hydrogen or $C_{1-6}$-alkyl and

$R^8$ represents hydrogen, $C_{1-6}$-alkyl, $C_{1-6}$-halogenoalkyl or acyl,

substituents of the optionally substituted radicals which may be mentioned being: cyano, halogen, such

as fluorine or chlorine, hydroxyl, $C_{1-4}$-alkyl, $C_{1-4}$-halogenoalkyl, phenyl, $C_{1-4}$-alkoxy, $C_{1-4}$-halogenoalkoxy, $C_{1-4}$-alkylthio and $C_{1-4}$-halogenoalkylthio, and in the case where the substituents are on a phenyl radical, additionally methylenedioxy, ethylenedioxy, halogen-substituted methylenedioxy, halogen-substituted ethylenedioxy and furthermore phenyl and phenoxy, which in turn can carry one or more of the abovementioned substituents,
and their physiologically tolerated salts and their N-oxides,
excluding 6-chloro-α-(isopropylaminomethyl)-3-pyridinemethanol and 2-chloro-α-(isopropylaminomethyl)-4-pyridinemethanol,
characterised in that

a) halogenomethyl ketones of the formula (II)

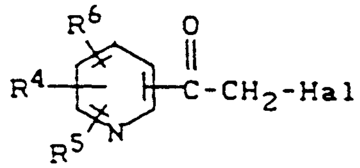

(II)

in which
$R^4$, $R^5$ and $R^6$ have the abovementioned meaning and Hal represents halogen,
are reacted with amines of the formula (III)

$HNR^2R^3$ (III)

in which
$R^2$ and $R^3$ have the abovementioned meaning,
and the carbonyl group is then reduced, or

b) epoxides of the formula IV)

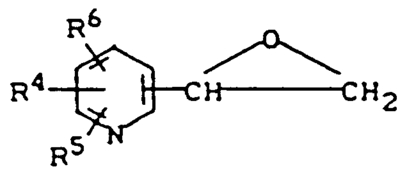

(IV)

in which
$R^4$, $R^5$ and $R^6$ have the abovementioned meaning,
are reacted with amines of the formula (III)

$HNR^2R^3$ (III)

in which
$R^2$ and $R^3$ have the abovementioned meaning,
or

c) beta-halogenoethyl compounds of the formula (V)

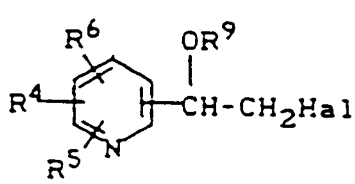

(V)

in which
$R^4$, $R^5$ and $R^6$ have the abovementioned meaning,
Hal represents halogen and

$R^9$ represents hydrogen,

are reacted with amines of the formula (III)

$HNR^2R^3$ (III)

in which

$R^2$ and $R^3$ have the abovementioned meaning,

or

d) in the case where, in formula Ia, $R^2$ represents hydrogen and $R^3$ represents substituted alkyl or aralkyl, compounds of the formula (VI)

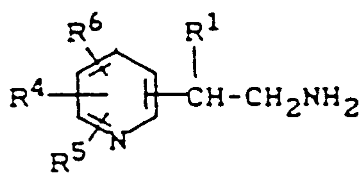

(VI)

in which

$R^1$, $R^4$, $R^5$ and $R^6$ have the abovementioned meaning,

are reacted with compounds of the formula (VII)

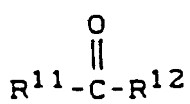

(VII)

in which

$R^{11}$ represents substituted alkyl, aryl or aralkyl and

$R^{12}$ represents hydrogen or $C_{1-4}$-alkyl,

under reducing conditions, or

e) in the case where, in formula I, $R^2$ represents hydrogen, compounds of the formula (VIII)

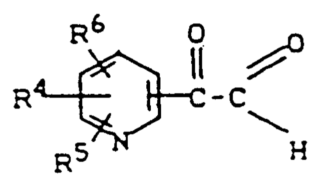

(VIII)

in which

$R^4$, $R^5$ and $R^6$ have the abovementioned meaning,

are reacted with amines of the formula (IX)

$R^3$-$NH_2$ (IX)

in which

$R^3$ has the abovementioned meaning,

under reducing conditions, or

f) in the case where, in formula I, $R^2$ represents hydrogen, compounds of the formula X

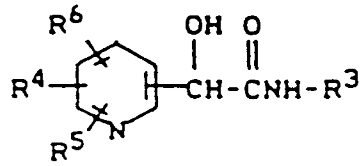

(X)

in which

$R^3$, $R^4$, $R^5$ and $R^6$ have the abovementioned meaning,

are reduced.

**9.** Process according to Claim 8 for the preparation of the heteroarylethylamines of the formula I in which

- $R^1$ represents OH or $C_{1-6}$-alkoxy,
- $R^2$ represents hydrogen or $C_{1-4}$-alkyl,
- $R^3$ represents hydrogen, $C_{1-6}$-alkyl which is optionally substituted by 1 to 5 halogen atoms or $C_{1-6}$-alkylphenyl which is substituted by halogen, $C_{1-4}$-alkyl, hydroxyl, $C_{1-4}$-alkoxy or optionally halogen-substituted methylenedioxy or ethylenedioxy,
- $R^4$ represents hydrogen, $C_{1-4}$-alkyl, halogen, cyano, $C_{1-4}$-halogenoalkyl having 1 to 5 halogen atoms, $C_{1-4}$-alkoxy, $C_{1-4}$-halogenoalkoxy having 1 to 5 halogen atoms, $NHSO_2$-$C_{1-4}$-alkyl, -COO-$C_{1-4}$-alkyl, -$CONH_2$, -CONH-$C_{1-4}$-alkyl or -CON($C_{1-4}$-alkyl)$_2$,
- $R^5$ represents hydrogen, $C_{1-4}$-alkoxy or -$NR^7R^8$,
- $R^6$ represents the radicals mentioned for $R^4$,
- $R^7$ represents hydrogen or $C_{1-4}$-alkyl and
- $R^8$ represents hydrogen or $C_{1-4}$-alkyl,

and their physiologically tolerated salts and their N-oxides, excluding 6-chloro-α-(isopropylaminomethyl)-3-pyridinemethanol and 2-chloro-α-(isopropylaminomethyl)-4-pyridinemethanol.

**10.** Process according to Claim 8, for the preparation of the heteroarylethylamine of the formula

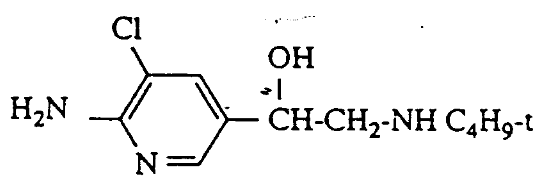

**11.** Process according to Claim 8, for the preparation of the heteroarylethylamine of the formula

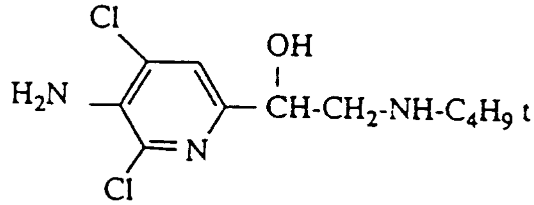

**Revendications**

**Revendications pour les Etats contractants suivants : AT, BE, CH, DE, FR, GB, IT, LI, NL, SE**

**1.** Mise en oeuvre d'hétéroaryléthylamines de formule (I):

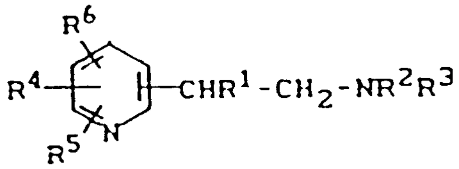

(I)

dans laquelle

$R^1$ désigne un radical OH, acyloxy ou alcoxy;

$R^2$ désigne l'hydrogène ou un radical alkyle;

$R^3$ désigne l'hydrogène, un radical alkyle ou cycloalkyle, un radical alkyle substitué, un radical aralkyle, aryle ou hétérocyclyle éventuellement substitué;

$R^2$ et $R^3$ peuvent désigner ensemble avec l'atome d'azote auquel ils sont liés un radical hétérocyclique éventuellement substitué;

$R^4$ désigne l'hydrogène, un radical alkyle, un halogène, un radical halogénoalkyle, hydroxyle, cyano, alcoxycarbonyle, aminocarbonyle, monoalkylaminocarbonyle ou dialkylaminocarbonyle, alcoxy, halogénoalcoxy, halogénoalkylthio, $NHSO_2$-alkyle;

$R^5$ désigne l'hydrogène, un radical hydroxyle, alcoxy ou le radical $-NR^7R^8$;

$R^6$ désigne les radicaux mentionnés en $R^4$;

$R^7$ désigne l'hydrogène ou un radical alkyle;

$R^8$ désigne l'hydrogène, un radical alkyle, halogénoalkyle ou acyle;

à l'exception du composé 6-amino-α-(((1-méthyl-3-phénylpropyl)amino)méthyl)-3-pyridineméthanol,

(Les composés de formule I peuvent se présenter sous la forme de leurs tautomères ainsi que de leurs racémates ou énantiomères) pour la stimulation des performances chez les animaux.

**2.** Mise en oeuvre d'hétéroaryléthylamines de formule I selon la revendication 1 pour la stimulation et l'accélération de la croissance, pour l'amélioration de la valeur nutritive de l'alimentation, de la qualité de la viande et le déplacement du rapport viande-graisse à l'avantage de la viande chez les animaux de rapport, d'élevage, d'ornement et les animaux domestiques.

**3.** Mise en oeuvre selon les revendications 1 et 2 d'hétéroaryléthylamines de formule I selon la revendication 1, dans laquelle

$R^1$ désigne un radical OH, $C_{1-6}$-alcoxy ou acyloxy et le radical acyloxy désigne un radical oxycarbonyl-$C_{1-6}$-alkyle, oxycarbonylphényle éventuellement substitué, oxysulfonyl-$C_{1-6}$-alkyle, oxysulfonylphényle éventuellement substitué;

$R^2$ désigne l'hydrogène ou un radical $C_{1-6}$-alkyle;

$R^3$ désigne l'hydrogène, un radical $C_{1-6}$-alkyle éventuellement substitué, $C_{3-6}$ cycloalkyle, $C_{1-6}$-alkylphényle ou phényle;

$R^2$ et $R^3$ peuvent désigner ensemble avec l'atome d'azote auquel ils sont liés un radical hétérocyclique saturé ou insaturé à 5 ou 6 éléments, éventuellement substitué, qui peut éventuellement contenir encore d'autres hétéroatomes de la série N, O, S,

$R^4$ désigne l'hydrogène, un radical $C_{1-4}$-alkyle, le fluor, le chlore, le brome, un radical $C_{1-4}$-halogénoalkyle, hydroxyle, cyano, $C_{1-4}$-alcoxycarbonyle, mono-$C_{1-4}$-alkylaminocarbonyle ou di-$C_{1-4}$-alkylaminocarbonyle, $C_{1-6}$-alcoxy, $C_{1-6}$-halogénoalcoxy, $C_{1-6}$-halogénoalkylthio, $-NHSO_2$-$C_{1-6}$-alkyle;

$R^5$ désigne l'hydrogène, un radical hydroxyle, $C_{1-6}$-alcoxy ou le radical $-NR^7R^8$;

$R^6$ désigne les radicaux indiqués en $R^4$;

$R^7$ désigne l'hydrogène ou un radical $C_{1-6}$-alkyle

$R^8$ désigne l'hydrogène, un radical $C_{1-6}$-alkyle, $C_{1-4}$-halogénoalkyle, $C_{1-6}$-alkylcarbonyle, benzoyle éventuellement substitué, $C_{1-6}$-alkylsulfonyle, phénylsulfonyle éventuellement substitué,

les substituants des radicaux éventuellement substitués étant de préférence: les radicaux cyano, un halogène comme le fluor ou le chlore, un radical hydroxy, $C_{1-4}$-alkyle, $C_{1-4}$-halogénoalkyle, phényle, $C_{1-4}$-alcoxy, $C_{1-4}$-halogénoalcoxy, $C_{1-4}$-alkylthio, $C_{1-4}$-halogénoalkylthio et, si les substituants se situent dans un radical phényle, un radical méthylènedioxy, éthylènedioxy, méthylènedioxy halogénosubstitué, éthylènedioxy halogénosubstitué, autre phényle, phénoxy qui, à leur tour, peuvent porter un ou plusieurs des substituants susmentionnées,

à l'exception du composé 6-amino-α-(((1-méthyl-3-phénypropyl)amino)méthyl)-3-pyridineméthanol.

**4.** Mise en oeuvre selon les revendications 1 et 2 d'hétéroaryléthylamines de formule I selon la revendication 1,
dans laquelle

- $R^1$ désigne un radical OH, $C_{1-6}$-alcoxy, en particulier méthoxy ou éthoxy;
- $R^2$ désigne l'hydrogène ou un radical $C_{1-4}$-alkyle, en particulier le méthyle ou l'éthyle;
- $R^3$ désigne l'hydrogène, un radical $C_{1-6}$-alkyle éventuellement substitué par 1 à 5 atomes d'un halogène, en particulier, le méthyle, l'éthyle, le propyle, le butyle, le pentyle, l'hexyle, les radicaux alkyle secondaires et tertiaires étant particulièrement recommandés ainsi que le radical $C_{1-6}$-alkylphényle qui peut éventuellement être substitué par un halogène, en particulier le fluor ou le chlore, un radical $C_{1-4}$-alkyle, hydroxy, $C_{1-4}$-alcoxy, un méthylènedioxy ou éthylènedioxy éventuellement halogénosubstitué, un radical phényle qui peut éventuellement être substitué par un halogène, en particulier le fluor ou le chlore, un radical $C_{1-4}$-alkyle, hydroxy, $C_{1-4}$-alcoxy, un méthylènedioxy ou éthylènedioxy éventuellement halogénosubstitué,
- $R^4$ désigne l'hydrogène, un radical $C_{1-4}$-alkyle, un halogène, en particulier le fluor, le chlore, le brome, un radical cyano, hydroxy, $C_{1-4}$-halogénoalkyle avec 1 à 5 atomes d'halogène, un radical $C_{1-4}$-alcoxy, $C_{1-4}$-halogénoalcoxy avec 1 à 5 atomes d'halogène, $NHSO_2$-$C_{1-4}$-alkyle, -COO-$C_{1-4}$-alkyle, -$CONH_2$, -CONH-$C_{1-4}$-alkyle,-CON($C_{1-4}$-alkyle)$_2$,
- $R^5$ désigne l'hydrogène, un radical OH, $C_{1-4}$-alcoxy, -$NR^7R^8$,
- $R^6$ désigne les radicaux mentionnés pour $R^4$,
- $R^7$ désigne l'hydrogène, un radical $C_{1-4}$-alkyle,
- $R^8$ désigne l'hydrogène, un radical $C_{1-4}$-alkyle.

à l'exception du composé 6-amino-α-(((1-méthyl-3-phénylpropyl)amino)méthyl)-3-pyridineméthanol.

**5.** Mise en oeuvre selon les revendications 1 et 2 de l'hétéreoaryléthylamine de formule

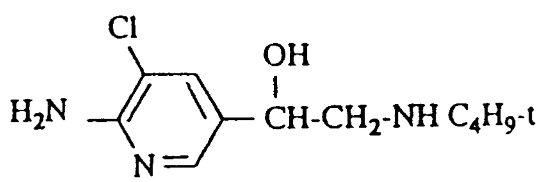

**6.** Mise en oeuvre selon les revendications 1 et 2 de l'hétéroarylamine de formule

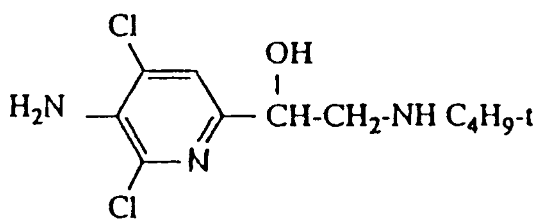

**7.** Aliments pour animaux et prémélanges pour la préparation d'aliments pour animaux contenant des hétéroaryléthylamines de formule générale (I) selon les revendications 1 à 6.

**8.** Agent stimulateur des performances pour animaux contenant des hétéroaryléthylamines de formule générale (I) selon les revendications 1 à 6.

**9.** Procédé de préparation d'agents stimulants des performances pour animaux,caractérisé en ce que les hétéroaryléthylamines de formule générale (I) selon les revendications 1 à 6 sont mélangées avec des charges, diluants et aliments ainsi qu'éventuellement d'autres additifs.

**10.** Mise en oeuvre d'hétéroaryléthylamines de formule (I) selon les revendications 1 à 6 pour la préparation d'agents stimulants des performances.

**11.** Mise en oeuvre d'hétéroaryléthylamines de formule (I) selon les revendications 1 à 6 pour la préparation d'agents de stimulation et d'accélération de la croissance, d'amélioration de la valeur nutritive des aliments, de la qualité de la viande et du déplacement du rapport viandegraisse à l'avantage de la viande chez les animaux de rapport, d'élevage, d'ornement et les animaux domestiques.

**12.** Hétéroaryléthylamines de formule I

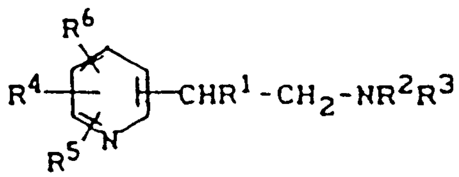

(I)

dans laquelle

$R^1$ désigne un radical OH, $C_{1-6}$-alcoxy, oxycarbonyl-$C_{1-6}$-alkyle, oxycarbonylphényle éventuellement substitué, oxysulfonyl-$C_{1-6}$-alkyle, oxysulfonylphényle éventuellement substitué;

$R^2$ désigne l'hydrogène ou un radical $C_{1-6}$-alkyle;

$R^3$ désigne l'hydrogène, un radical $C_{1-6}$-alkyle éventuellement substitué, $C_{3-6}$-cycloalkyle, $C_{1-6}$-alkylphényle substitué ou phényle;

$R^4$ désigne l'hydrogène, un radical $C_{1-4}$-alkyle, le fluor, le chlore, le brome, un radical $C_{1-4}$-halogénoalkyle, cyano, $C_{1-4}$-alcoxycarbonyle, mono-$C_{1-4}$-alkylaminocarbonyle ou di-$C_{1-4}$-alkylaminocarbonyle, $C_{1-6}$-alcoxy, $C_{1-6}$-halogénoalcoxy, $C_{1-6}$-halogénoalkylthio, -$NHSO_2$-$C_{1-6}$-alkyle;

$R^5$ désigne l'hydrogène, un radical $C_{1-6}$-alcoxy ou le radical -$NR^7R^8$;

$R^6$ désigne l'hydrogène, un radical $C_{1-4}$-alkyle, le fluor, le chlore, le brome, un radical $C_{1-4}$-halogénoalkyle, cyano, $C_{1-4}$-alcoxycarbonyle, $C_{1-6}$-alcoxy, $C_{1-6}$-halogénoalcoxy, $C_{1-6}$-halogénoalkylthio, $NHSO_2$-alkyle;

à condition que $R^4$, $R^5$ et $R^6$ ne désignent pas simultanément l'hydrogène;

$R^7$ désigne l'hydrogène ou un radical $C_{1-6}$-alkyle;

$R^8$ désigne l'hydrogène, un radical $C_{1-6}$-alkyle, $C_{1-6}$-halogénoalkyle ou acyle,

les substituants des radicaux éventuellement substitués étant notamment: un radical cyano, un halogène comme le fluor ou le chlore, un radical hydroxy, $C_{1-4}$-alkyle, $C_{1-4}$-halogénoalkyle, phényle, $C_{1-4}$-alcoxy, $C_{1-4}$-halogénoalcoxy, $C_{1-4}$-alkylthio, $C_{1-4}$-halogénoalkylthio et, si les substituants se trouvent dans un radical phényle, un radical méthylènedioxy, éthylènedioxy, méthylènedioxy halogénosubstitué, éthylènedioxy halogénosubstitué ou encore phényle et phénoxy qui, à leur tour, peuvent porter un ou plusieurs des substituants susmentionnés,

ainsi que leurs sels physiocompatibles et leurs N-oxydes,

à l'exception du 6-chloro-α(isopropylaminométhyl)-3-pyridine-méthanol et du 2-chloro-α-(isopropylaminométhyl)-4-pyridine-méthanol.

**13.** Hétéroaryléthylamines de formule I selon la revendication 12

dans laquelle

$R^1$ désigne un radical OH, $C_{1-6}$-alcoxy;

$R^2$ désigne l'hydrogène ou un radical $C_{1-4}$-alkyle;

$R^3$ désigne l'hydrogène, un radical $C_{1-6}$-alkyle éventuellement substitué par 1 à 5 atomes d'halogène ainsi que $C_{1-6}$-alkylphényle qui est substitué par un halogène, un radical $C_{1-4}$-alkyle, hydroxy, $C_{1-4}$-alcoxy, méthylènedioxy ou éthylènedioxy éventuellement halogénosubstitué;

$R^4$ désigne l'hydrogène, un radical $C_{1-4}$-alkyle, un halogène, un radical cyano, $C_{1-4}$-halogénoalkyle avec 1 à 5 atomes d'halogène, $C_{1-4}$-alcoxy, $C_{1-4}$-halogénoalcoxy avec 1 à 5 atomes

d'halogène, $-NHSO_2-C_{1-4}$-alkyle, $-COO-C_{1-4}$-alkyle, $-CONH_2$, - $CONH-C_{1-4}$-alkyle,-CON-$(C_{1-4}$-alkyle$)_2$;

$R^5$ désigne l'hydrogène, $C_{1-4}$-alcoxy, $-NR^7R^8$,

$R^6$ désigne les radicaux mentionnés pour $R^4$,

$R^7$ désigne l'hydrogène, un radical $C_{1-4}$-alkyle,

$R^8$ désigne l'hydrogène, un radical $C_{1-4}$-alkyle;

ainsi que leurs sels physiocompatibles et leurs N-oxydes,

à l'exception du 6-chloro-α(isopropylaminométhyl)-3-pyridine-méthanol et du 2-chloro-α-(isopropylaminométhyl)-4-pyridine-méthanol.

**14.** Hétéroéthylamine de formule

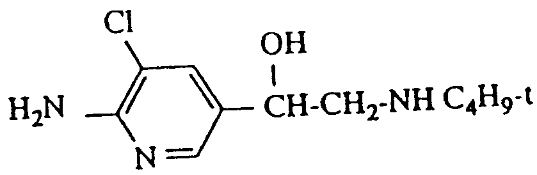

**15.** Hétéroaryléthylamine de formule

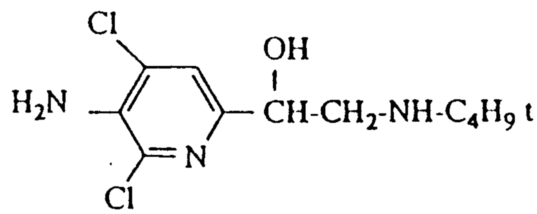

**16.** Procédé de préparation des hétéroaryléthylamines de formule I selon la revendication 12

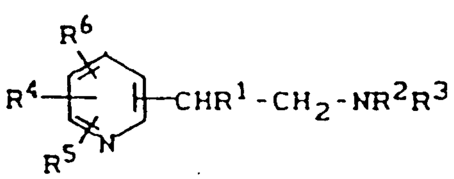

(I)

dans laquelle

$R^1$, $R^2$, $R^3$, $R^4$, $R^5$ et $R^6$ possèdent la signification mentionnée dans la revendication 12

caractérisé en ce que

a) des halogénométhylcétones de formule (II)

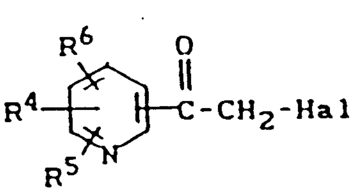

(II)

dans laquelle

$R^4$, $R^5$ et $R^6$ ont la signification susmentionnée dans la revendication 12 et Hal désigne un

halogène,
sont amenées à réagir avec des amines de formule (III)

$HNR^2R^3$ (III)

dans laquelle
$R^2$ et $R^3$ ont la signification susmentionnée à la revendication 12,
et le radical carbonyle est ensuite réduit

ou
b) des époxydes de formule (IV)

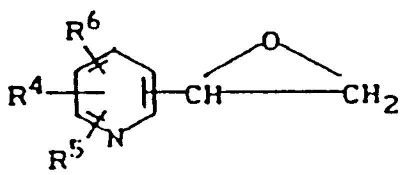

(IV)

dans laquelle
$R^4$, $R^5$ et $R^6$ ont la signification susmentionnée
sont amenés à réagir avec des amines de formule (III)

$HNR^2R^3$ (III)

dans laquelle
$R^2$ et $R^3$ ont la signification susmentionnée
ou
c) des composés bêta-halogénoéthylés de formule (V)

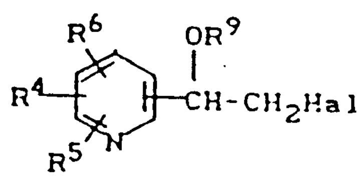

(V)

dans laquelle
$R^4$, $R^5$ et $R^6$ ont la signification susmentionnée,
Hal désigne un halogène,
$R^9$ désigne l'hydrogène,
sont amenés à réagir avec des amines de formule (III)

$HNR^2R^3$ (III)

dans laquelle
$R^2$ et $R^3$ ont la signification susmentionnée
ou
d) si $R^2$ désigne l'hydrogène et $R^3$ désigne un radical alkyle ou aralkyle substitué dans la formule I, des composés de formule (VI)

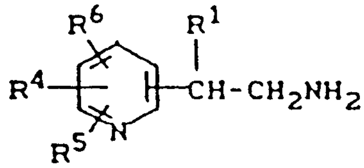

(VI)

dans laquelle

$R^1$, $R^4$, $R^5$ et $R^6$ ont la signification mentionnée à la figure 12

sont amenés à réagir dans des conditions réductrices avec

des composés de formule (VII)

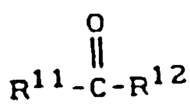

(VII)

dans laquelle

$R^{11}$ désigne un radical alkyle, aryle ou aralkyle substitué,

$R^{12}$ désigne l'hydrogène ou un radical $C_{1-4}$-alkyle ou

e) si $R^2$ désigne l'hydrogène dans la formule I, des composés de formule (VIII)

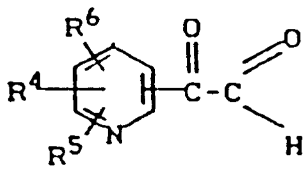

(VIII)

dans laquelle

$R^4$, $R^5$ et $R^6$ ont la signification susmentionnée

sont amenés à réagir dans des conditions réductrices avec

des amines de formule (IX)

$R^3$-$NH_2$ (IX)

dans laquelle

$R^3$ a la signification susmentionnée ou

f) si $R^2$ désigne l'hydrogène dans la formule I, des composés de formule X

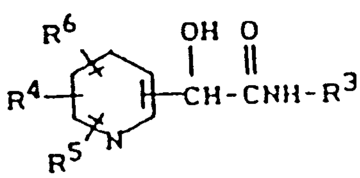

(X)

dans laquelle

$R^3$, $R^4$, $R^5$ et $R^6$ ont la signification susmentionnée

sont réduits.

**17.** Dérivés de pyridine de formule

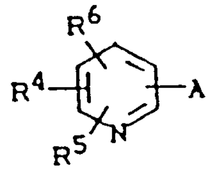

dans laquelle

$R^4$ désigne l'hydrogène, un radical $C_{1-4}$-alkyle, le fluor, le chlore, le brome, un radical $C_{1-4}$-halogénoalkyle, cyano, $C_{1-4}$-alcoxycarbonyle, mono-$C_{1-4}$-alkylaminocarbonyle ou di-$C_{1-4}$-alkylaminocarbonyle, $C_{1-6}$-alcoxy, $C_{1-6}$-halogénoalcoxy, $C_{1-6}$-halogénoalkylthio, -$NHSO_2$-alkyle;

$R^5$ désigne l'hydrogène, un radical $C_{1-6}$-alcoxy ou le radical -$NR^7R^8$;

$R^6$ désigne l'hydrogène, un radical $C_{1-4}$-alkyle, le fluor, le chlore, le brome, un radical $C_{1-4}$-halogénoalkyle, cyano, $C_{1-4}$-alcoxycarbonyle, $C_{1-6}$-alcoxy, $C_{1-6}$-halogénoalcoxy, $C_{1-6}$-halogénoalkylthio,-$NHSO_2$-alkyle;

à condition que $R^4$, $R^5$ et $R^6$ ne désignent pas simultanément l'hydrogène et

A désigne les radicaux -CHO, -COOH, -COO($C_{1-4}$-Alkyl), -COCl,

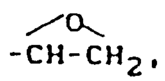

-$COCH_3$, -CO-CHO, -$COCH_2$-Halogéno -CH = CH-$NO_2$, -CO-$CH_2$-COO($C_{1-4}$-Alkyle),

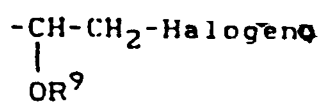 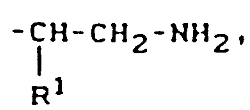 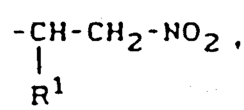

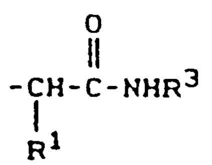

et

$R^9$ désigne l'hydrogène;

$R^1$ désigne un radical hydroxyle ou acyloxy;

$R^3$ désigne l'hydrogène, un radical $C_{1-6}$-alkyle, $C_{1-6}$-alkyle substitué, cycloalkyle, $C_{1-6}$-alkylphényle substitué ou phényle;

$R^7$ désigne l'hydrogène ou un radical $C_{1-6}$-alkyle;

$R^8$ désigne l'hydrogène, un radical $C_{1-6}$-alkyle, $C_{1-6}$-halogénoalkyle ou acyle.

à l'exception de l'α-(bromométhyl)-2-chloro-4-pyridineméthanol, de la bromométhyl-2-chloro-4-pyridinecétone, la 2-chloro-4-pyridylméthylcétone, la bromométhyl-6-chloro-3-pyridylcétone, l'α-(bromométhyl)-2-chloro-4-pyridineméthanol, la 5-acétyl-2-fluoropyridine, la 5-bromoacétyl-2-fluoropyridine, le (2-fluoropyrid-5-yl)oxirane.

**18.** Dérivés de pyridine selon la revendication 17, caractérisés en ce que

$R^5$ désigne le radical -$NR^7R^8$, les radicaux A, $R^4$, $R^6$, $R^7$ et $R^8$ ayant la signification mentionnée à la revendication 17.

**Revendications pour les Etats contractants suivants : ES, GR**

**1.** Mise en oeuvre d'hétéroaryléthylamines de formule (I):

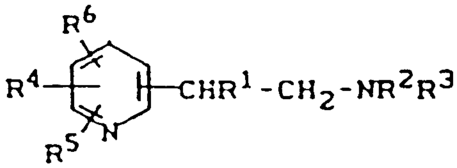

(I)

dans laquelle

| | |
|---|---|
| $R^1$ | désigne un radical OH, acyloxy ou alcoxy; |
| $R^2$ | désigne l'hydrogène ou un radical alkyle; |
| $R^3$ | désigne l'hydrogène, un radical alkyle ou cycloalkyle, un radical alkyle substitué, un radical aralkyle, aryle ou hétérocyclyle éventuellement substitué; |
| $R^2$ et $R^3$ | peuvent désigner ensemble avec l'atome d'azote auquel ils sont liés un radical hétérocyclique éventuellement substitué; |
| $R^4$ | désigne l'hydrogène, un radical alkyle, un halogène, un radical halogénoalkyle, hydroxyle, cyano, alcoxycarbonyle, aminocarbonyle, monoalkylaminocarbonyle ou dialkylaminocarbonyle, alcoxy, halogénoalcoxy, halogénoalkylthio, $NHSO_2$-alkyle; |
| $R^5$ | désigne l'hydrogène, un radical hydroxyle, alcoxy ou le radical $-NR^7R^8$; |
| $R^6$ | désigne les radicaux mentionnés en $R^4$; |
| $R^7$ | désigne l'hydrogène ou un radical alkyle; |
| $R^8$ | désigne l'hydrogène, un radical alkyle, halogénoalkyle ou acyle; |

à l'exception du composé 6-amino-α-(((1-méthyl-3-phénylpropyl)amino)méthyl)-3-pyridineméthanol,

(Les composés de formule I peuvent se présenter sous la forme de leurs tautomères ainsi que de leurs racémates ou énantiomères) pour la stimulation des performances chez les animaux.

**2.** Mise en oeuvre d'hétéroaryléthylamines de formule I selon la revendication 1 pour la stimulation et l'accélération de la croissance, pour l'amélioration de la valeur nutritive des aliments, de la qualité de la viande et le déplacement du rapport viande-graisse à l'avantage de la viande chez les animaux de rapport, d'élevage, d'ornement et les animaux domestiques.

**3.** Mise en oeuvre selon les revendications 1 et 2 d'hétéroaryléthylamines de formule I selon la revendication 1 dans laquelle

| | |
|---|---|
| $R^1$ | désigne un radical OH, $C_{1-6}$-alcoxy ou acyloxy et le radical acyloxy désigne un radical oxycarbonyl-$C_{1-6}$-alkyle, oxycarbonylphényle éventuellement substitué, oxysulfonyl-$C_{1-6}$-alkyle, oxysulfonylphényle éventuellement substitué; |
| $R^2$ | désigne l'hydrogène ou un radical $C_{1-6}$-alkyle; |
| $R^3$ | désigne l'hydrogène, un radical $C_{1-6}$-alkyle éventuellement substitué, $C_{3-6}$ cycloalkyle, $C_{1-6}$-alkylphényle ou phényle; |
| $R^2$ et $R^3$ | peuvent désigner ensemble avec l'atome d'azote auquel ils sont liés un radical hétérocyclique saturé ou insaturé à 5 ou 6 éléments éventuellement substitué, qui peut éventuellement contenir encore d'autres hétéroatomes de la série N, O, S, |
| $R^4$ | désigne l'hydrogène, un radical $C_{1-4}$-alkyle, le fluor, le chlore, le brome, un radical $C_{1-4}$-halogénoalkyle, hydroxyle, cyano, $C_{1-4}$-alcoxycarbonyle, mono-$C_{1-4}$-alkylaminocarbonyle ou di-$C_{1-4}$-alkylaminocarbonyle, $C_{1-6}$-alcoxy, $C_{1-6}$-halogénoalcoxy, $C_{1-6}$-halogénoalkylthio, $-NHSO_2-C_{1-6}$-alkyle; |
| $R^5$ | désigne l'hydrogène, un radical hydroxyle, $C_{1-6}$-alcoxy ou le radical $-NR^7R^8$; |
| $R^6$ | désigne les radicaux indiqués en $R^4$; |
| $R^7$ | désigne l'hydrogène ou un radical $C_{1-6}$-alkyle; |
| $R^8$ | désigne l'hydrogène, un radical $C_{1-6}$-alkyle, $C_{1-4}$-halogénoalkyle, $C_{1-6}$-alkylcarbonyle, benzoyle éventuellement substitué, $C_{1-6}$-alkylsulfonyle, phénylsulfonyle éventuellement substitué, |

les substituants des radicaux éventuellement substitués étant de préférence: les radicaux cyano, un halogène comme le fluor ou le chlore, un radical hydroxy, $C_{1-4}$-alkyle, $C_{1-4}$-halogénoalkyle, phényle, $C_{1-4}$-alcoxy, $C_{1-4}$-halogénoalcoxy, $C_{1-4}$-alkylthio, $C_{1-4}$-halogénoalkylthio et, si les substituants se situent dans un radical phényle, de préférence, un radical méthylènedioxy, éthylènedioxy, méthylènedioxy halogénosubstitué, éthylènedioxy halogénosubstitué, autre phényle, phénoxy qui, à leur tour, peuvent porter un ou plusieurs des substituants susmentionnées,

à l'exception du composé 6-amino-α-(((1-méthyl-3-phénypropyl)amino)méthyl)-3-pyridineméthanol pour la préparation d'agents stimulants des performances chez les animaux.

**4.** Mise en oeuvre selon les revendications 1 et 2 d'hétéroaryléthylamines de formule I selon la revendication 1,

dans laquelle

$R^1$ désigne un radical OH, $C_{1-6}$-alcoxy, en particulier méthoxy ou éthoxy;

$R^2$ désigne l'hydrogène ou un radical $C_{1-4}$-alkyle, en particulier le méthyle ou l'éthyle;

$R^3$ désigne l'hydrogène, un radical $C_{1-6}$-alkyle éventuellement substitué par 1 à 5 atomes d'un halogène, en particulier, le méthyle, l'éthyle, le propyle, le butyle, le pentyle, l'hexyle, les radicaux alkyle secondaires et tertiaires étant particulièrement recommandés ainsi que le radical $C_{1-6}$-alkylphényle qui peut éventuellement être substitué par un halogène, en particulier le fluor ou le chlore, un radical $C_{1-4}$-alkyle, hydroxy, $C_{1-4}$-alcoxy, un méthylènedioxy ou éthylènedioxy éventuellement halogénosubstitué, un radical phényle qui peut éventuellement être substitué par un halogène, en particulier le fluor ou le chlore, un radical $C_{1-4}$-alkyle, hydroxy, $C_{1-4}$-alcoxy, un méthylènedioxy ou éthylènedioxy éventuellement halogénosubstitué,

$R^4$ désigne l'hydrogène, un radical $C_{1-4}$-alkyle, un halogène, en particulier le fluor, le chlore, le brome, un radical cyano, hydroxy, $C_{1-4}$-halogénoalkyle avec 1 à 5 atomes d'halogène, un radical $C_{1-4}$-alcoxy, $C_{1-4}$-halogénoalcoxy avec 1 à 5 atomes d'halogène, $NHSO_2$-$C_{1-4}$-alkyle, -COO-$C_{1-4}$-alkyle, -$CONH_2$, -CONH-$C_{1-4}$-alkyle,-CON($C_{1-4}$-alkyle)$_2$,

$R^5$ désigne l'hydrogène, un radical OH, $C_{1-4}$-alcoxy, -$NR^7R^8$,

$R^6$ désigne les radicaux mentionnés pour $R^4$,

$R^7$ désigne l'hydrogène, un radical $C_{1-4}$-alkyle,

$R^8$ désigne l'hydrogène, un radical $C_{1-4}$-alkyle.

à l'exception du composé 6-amino-α-(((1-méthyl-3-phénylpropyl)amino)méthyl)-3-pyridineméthanol pour la préparation d'agents stimulants des performances chez les animaux.

**5.** Mise en oeuvre selon les revendications 1 et 2 de l'hétéreoaryléthylamine de formule

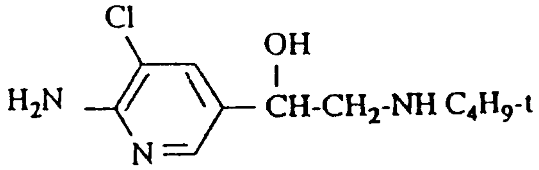

pour la préparation d'agents stimulants des performances chez les animaux.

**6.** Mise en oeuvre selon les revendications 1 et 2 de l'hétéroarylamine de formule

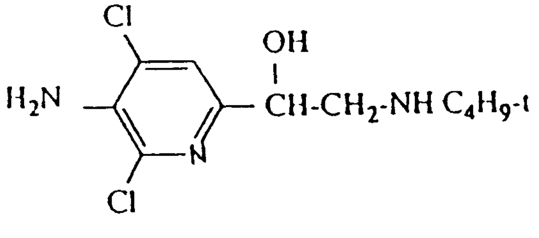

pour la préparation d'agents stimulants des performances chez les animaux.

**7.** Procédé de préparation d'agents stimulants des performances chez les animaux caractérisé en ce que des hétéroaryléthylamines de formule générale (I) selon les revendications 1 à 6 sont mélangées avec des charges, diluants et des aliments ainsi qu'avec éventuellement d'autres additifs.

**8.** Procédé de préparation d'hétéroaryléthylamines de formule I,

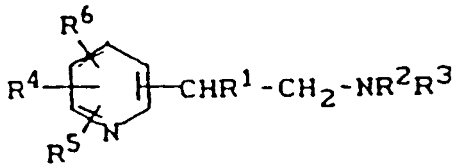

(I)

dans laquelle

$R^1$ désigne un radical OH, $C_{1-6}$-alcoxy, oxycarbonyl-$C_{1-6}$-alkyle, oxycarbonylphényle éventuellement substitué, oxysulfonyl-$C_{1-6}$-alkyle, oxysulfonylphényle éventuellement substitué;

$R^2$ désigne l'hydrogène ou un radical $C_{1-6}$-alkyle;

$R^3$ désigne l'hydrogène, un radical $C_{1-6}$-alkyle éventuellement substitué, $C_{3-6}$-cycloalkyle, $C_{1-6}$-alkylphényle substitué ou phényle;

$R^4$ désigne l'hydrogène, un radical $C_{1-4}$-alkyle, le fluor, le chlore, le brome, un radical $C_{1-4}$-halogénoalkyle, cyano, $C_{1-4}$ -alcoxycarbonyle, mono-$C_{1-4}$-alkylaminocarbonyle ou di-$C_{1-4}$-alkylaminocarbonyle, $C_{1-6}$-alcoxy, $C_{1-6}$-halogénoalcoxy, $C_{1-6}$-halogénoalkylthio, -$NHSO_2$-$C_{1-6}$-alkyle;

$R^5$ désigne l'hydrogène, un radical $C_{1-6}$-alcoxy ou le radical -$NR^7R^8$;

$R^6$ désigne l'hydrogène, un radical $C_{1-4}$-alkyle, le fluor, le chlore, le brome un radical $C_{1-4}$-halogénoalkyle, cyano, $C_{1-4}$-alcoxycarbonyle, $C_{1-6}$-alcoxy, $C_{1-6}$-halogénoalcoxy, $C_{1-6}$-halogénoalkylthio, $NHSO_2$-alkyle;

à condition que $R^4$, $R^5$ et $R^6$ ne désignent pas simultanément l'hydrogène;

$R^7$ désigne l'hydrogène ou un radical $C_{1-6}$-alkyle;

$R^8$ désigne l'hydrogène, un radical $C_{1-6}$-alkyle, $C_{1-6}$-halogénoalkyle ou acyle,

les substituants des radicaux éventuellement substitués étant notamment: un radical cyano, un halogène comme le fluor ou le chlore, un radical hydroxy, $C_{1-4}$-alkyle, $C_{1-4}$-halogénoalkyle, phényle, $C_{1-4}$-alcoxy, $C_{1-4}$-halogénoalcoxy, $C_{1-4}$-alkylthio, $C_{1-4}$-halogénoalkylthio et, si les substituants se trouvent dans un radical phényle, un radical méthylènedioxy, éthylènedioxy, méthylènedioxy halogénosubstitué, éthylènedioxy halogénosubstitué ou encore phényle et phénoxy qui, à leur tour, peuvent porter un ou plusieurs des substituants susmentionnés,

ainsi que leurs sels physiocompatibles et leurs N-oxydes,

à l'exception du 6-chloro-α-(isopropylaminométhyl)-3 -pyridine-méthanol et du 2-chloro-α-(isopropylaminométhyl)-4-pyridine-méthanol,

caractérisé en ce que

a) des halogénométhylcétones de formule (II)

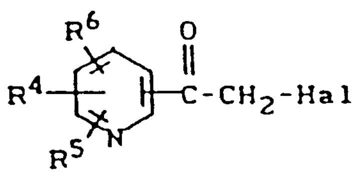

(II)

dans laquelle

$R^4$, $R^5$ et $R^6$ ont la signification susmentionnée et Hal désigne un halogène,

sont amenées à réagir avec des amines de formule (III)

$HNR^2R^3$ (III)

dans laquelle

$R^2$ et $R^3$ ont la signification susmentionnée,

et le radical carbonyle est ensuite réduit

ou

b) des époxydes de formule (IV)

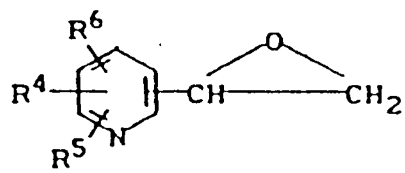

(IV)

dans laquelle

$R^4$, $R^5$ et $R^6$ ont la signification susmentionnée

sont amenés à réagir avec des amines de formule (III)

$HNR^2R^3$ (III)

dans laquelle

$R^2$ et $R^3$ ont la signification susmentionnée

ou

c) des composés bêta-halogénoéthylés de formule (V)

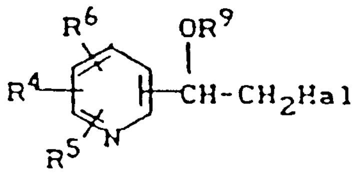

(V)

dans laquelle

$R^4$, $R^5$ et $R^6$ ont la signification susmentionnée

Hal désigne un halogène,

$R^9$ désigne l'hydrogène,

sont amenés à réagir avec des amines de formule (III)

$HNR^2R^3$ (III)

dans laquelle

$R^2$ et $R^3$ ont la signification susmentionnée

ou

d) si $R^2$ désigne l'hydrogène et $R^3$ désigne un radical alkyle ou aralkyle substitué dans la formule Ia, des composés de formule (VI)

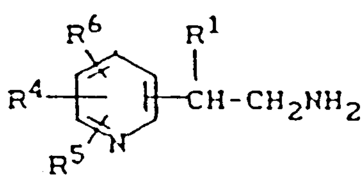

(VI)

dans laquelle

$R^1$, $R^4$, $R^5$ et $R^6$ ont la signification susmentionnée

sont amenés à réagir dans des conditions réductrices avec des composés de formule (VII)

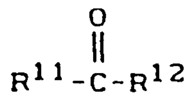

(VII)

dans laquelle

$R^{11}$ désigne un radical alkyle, aryle ou aralkyle substitué,

$R^{12}$ désigne l'hydrogène ou un radical $C_{1-4}$-alkyle ou

e) si $R^2$ désigne l'hydrogène dans la formule I, des composés de formule (VIII)

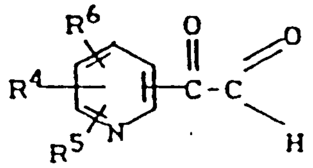

(VIII)

dans laquelle

$R^4$, $R^5$ et $R^6$ ont la signification susmentionnée

sont amenés à réagir dans des conditions réductrices avec des amines de formule (IX) $R^3$-$NH_2$ - (IX)

dans laquelle

$R^3$ a la signification susmentionnée ou

f) si $R^2$ désigne l'hydrogène dans la formule I, des composés de formule X

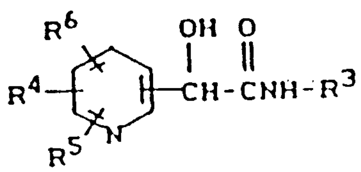

(X)

dans laquelle

$R^3$, $R^4$, $R^5$ et $R^6$ ont la signification susmentionnée sont réduits.

**9.** Procédé selon la revendication 8 pour la préparation d'hétéroaryléthylamines de formule I

dans laquelle

$R^1$ désigne un radical OH, $C_{1-6}$-alcoxy,

$R^2$ désigne l'hydrogène ou un radical $C_{1-4}$-alkyle,

$R^3$ désigne l'hydrogène, un radical $C_{1-6}$-alkyle éventuellement substitué par 1 à 5 atomes d'halogène ainsi que $C_{1-6}$-alkylphényle qui est substitué par un halogène, un radical $C_{1-4}$-alkyle, hydroxy, $C_{1-4}$-alcoxy, méthylènedioxy ou éthylènedioxy éventuellement halogéno-substitué;

$R^4$ désigne l'hydrogène, un radical $C_{1-4}$-alkyle, un halogène, un radical cyano, $C_{1-4}$-halogénoalkyle avec 1 à 5 atomes d'halogène, un radical $C_{1-4}$-alcoxy, $C_{1-4}$-halogénoalcoxy avec 1 à 5 atomes d'halogène, $NHSO_2$-$C_{1-4}$-alkyle, -COO-$C_{1-4}$-alkyle, -$CONH_2$, -CONH-$C_{1-4}$-alkyle, -CON($C_{1-4}$-alkyle)$_2$,

$R^5$ désigne l'hydrogène, un radical $C_{1-4}$-alcoxy, -$NR^7R^8$,

$R^6$ désigne les radicaux mentionnés pour $R^4$,

$R^7$ désigne l'hydrogène, un radical $C_{1-4}$-alkyle,

$R^8$ désigne l'hydrogène, un radical $C_{1-4}$-alkyle.

ainsi que leurs sels physiocompatibles et leurs N-oxydes,

à l'exception du 6-chloro-α-(isopropylaminométhyl)-3-pyridine-méthanol et du 2-chloro-α-(isopropylaminométhyl)-4-pyridine-méthanol,

**10.** Procédé selon la revendication 8 pour la préparation de l'hétéroaryléthylamine de formule

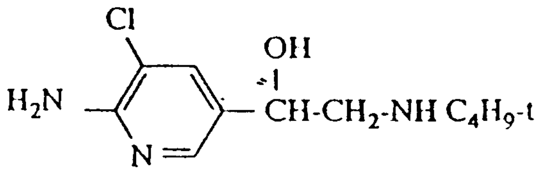

**11.** Procédé selon la revendication 8 pour la préparation de l'hétéroaryléthylamine de formule